(19) Europäisches Patentamt
European Patent Office
Office européen des brevets

(11) EP 2 066 683 B1

# (12) FASCICULE DE BREVET EUROPEEN

(45) Date de publication et mention de la délivrance du brevet:
**03.11.2010 Bulletin 2010/44**

(21) Numéro de dépôt: **07823850.8**

(22) Date de dépôt: **26.09.2007**

(51) Int Cl.:
***C07H 17/02*** *(2006.01)* ***A61K 31/706*** *(2006.01)*
***A61P 7/02*** *(2006.01)* ***A61P 9/04*** *(2006.01)*

(86) Numéro de dépôt international:
**PCT/FR2007/052005**

(87) Numéro de publication internationale:
**WO 2008/037922 (03.04.2008 Gazette 2008/14)**

## (54) DERIVES DE 5 - THIOXYLOPYRANOSE ET LEUR UTILISATION EN THERAPEUTIQUE

DERIVATE VON 5-THIOXYLOPYRANOSE UND DEREN VERWENDUNG FÜR EINE BEHANDLUNG

DERIVATIVES OF 5-THIOXYLOPYRANOSE AND USE OF SAME FOR TREATMENT

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **27.09.2006 FR 0653961**

(43) Date de publication de la demande:
**10.06.2009 Bulletin 2009/24**

(73) Titulaire: **LABORATOIRES FOURNIER SA**
**21000 Dijon (FR)**

(72) Inventeurs:
- **BARBEROUSSE, Véronique**
  **21121 Hauteville-les-Dijon (FR)**
- **THOMAS, Didier**
  **21850 Saint Apollinaire (FR)**
- **BONDOUX, Michel**
  **21121 Fontaine Les Dijon (FR)**

(74) Mandataire: **Hubert, Philippe et al**
**Cabinet Beau de Loménie**
**158, rue de l'Université**
**75340 Paris Cédex 07 (FR)**

(56) Documents cités:
**EP-A- 1 609 799** **EP-A1- 0 290 321**
**EP-A2- 0 365 397** **WO-A-2005/030785**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 066 683 B1

## Description

La présente invention concerne de nouveaux composés du 5-thioxylose, préférentiellement des dérivés de type 5-thioxylopyranose, ainsi que leur procédé de préparation et leur utilisation en tant que principe actif de médicaments, notamment destinés au traitement ou à la prévention des thromboses.

### *Art antérieur*

On connaît déjà des dérivés du D-xylose, par exemple dans EP 051 023 B1, US 4 877 808, EP 421 829 B1 ou dans la publication J. Med. Chem. Vol. 36 n° 7, p 898-903. Les composés décrits dans ces documents sont utiles pour réduire les risques de thrombose veineuse chez l'homme. Le mécanisme d'action de ces composés semble être un effet sur les glycosaminoglycanes (J. Biol. Chem., Vol 270 n° 6 p 2662-68, Thromb. Haemost. 1999, 81 p 945-950). Le document WO 2005/030785 décrit des pyridinyl 5-thio-β-D-xylopyranosides présentant une activité pour traiter les thromboses veineuses. Le document EP 0 365 397 décrit des β-D-phényl-thioxylosides et leur utilisation en thérapeutique, en tant qu'agents antithrombotiques.

On sait par ailleurs que les effets bénéfiques d'une angioplastie transluminale coronarienne peuvent être compromis en raison d'une resténose du vaisseau, provoquant ainsi une nouvelle obstruction de la lumière artérielle. Des composés permettant d'éviter cette resténose sont donc du plus grand intérêt pour maintenir un bon diagnostic suite à l'intervention chirurgicale vis à vis de l'arthérosclérose.

On a maintenant découvert, et c'est l'objet de la présente invention, de nouveaux composés qui présentent une bonne efficacité lorsqu'ils sont administrés par voie orale avec un excellent résultat pharmacologique - généralement proche de 100%- contre l'apparition d'une thrombose artérielle ou veineuse.

### *Description*

Les nouveaux composés selon l'invention sont **caractérisés en ce qu**'ils sont choisis parmi :

a) les composés de formule :

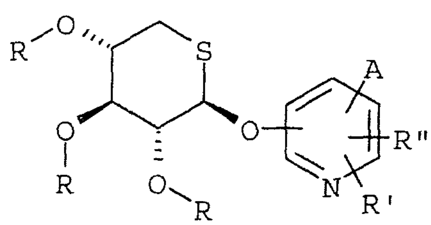

I

dans laquelle :

- le groupe pentapyranosyle représente un groupe 5-thio-β-D-xylopyranosyle,
- R représente un atome d'hydrogène ou un groupe acyle en $C_2$-$C_6$,
- R' et R" représentent chacun indépendamment, un atome d'hydrogène, un atome d'halogène, un groupe alkyle en $C_1$-$C_4$ ou un groupe 6-fluoro-3-pyridinyle,
- A représente un cycle aromatique à 5 ou 6 chaînons de formule :

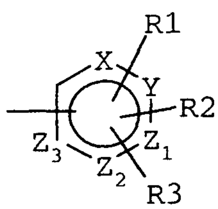

dans laquelle :

- X représente un atome d'azote, d'oxygène ou de soufre,

- Y représente un atome de carbone ou une liaison simple,
- $Z_1$, $Z_2$ et $Z_3$ représentent chacun indépendamment, un atome de carbone ou d'azote,
- $R_1$, $R_2$ et $R_3$ représentent chacun indépendamment, un atome d'hydrogène ou un atome d'halogène, un groupe cyano, un groupe alkyle en $C_1$-$C_4$, un groupe alcoxy en $C_1$-$C_4$, un groupe trifluorométhyle ; ou
- $R_1$ et $R_2$ forment ensemble avec les atomes de l'hétérocycle auxquels ils sont rattachés un cycle aromatique comprenant 6 atomes de carbone, A représentant ainsi un groupe bicyclique fusionné, en particulier un groupe benzofuranyle ou benzothiényle,

b) leurs sels d'addition.

L'invention concerne également les composés de formule I pour leur utilisation en tant que substance pharmacologiquement active.

En particulier, l'invention concerne l'utilisation d'au moins une substance choisie parmi les composés de formule I et leurs sels non toxiques pour la préparation d'un médicament, utile en thérapeutique humaine ou animale, destiné à la prévention ou au traitement des thromboses, notamment les thromboses veineuses. Les composés selon l'invention trouvent également leur utilité comme principes actifs de médicaments destinés à la prévention d'une resténose après angioplastie transluminale coronarienne. Les composés selon l'invention étant actifs selon un mode d'action faisant intervenir les glycosaminoglycanes, ils pourront encore être utiles en tant que principe actif d'un médicament destiné au traitement ou à la prévention de toute autre maladie dans laquelle les glycosaminoglycanes sont impliqués.

***Description détaillée***

Dans la formule I, on entend par groupe alkyle en $C_1$-$C_4$ une chaîne hydrocarbonée saturée linéaire ou ramifiée ayant de 1 à 4 atomes de carbone ou partiellement ou totalement cyclisée, la portion cyclisée ayant 3 ou 4 atomes de carbone. Des exemples de groupes alkyle en $C_1$-$C_4$ sont notamment les groupes méthyle, éthyle, propyle, butyle, 1-méthyléthyle, 1,1-diméthyléthyle, 1-méthylpropyle, 2-méthylpropyle, cyclopropyle ou cyclopropylméthyle.

Par halogène, il faut comprendre un atome de fluor, de chlore, de brome ou d'iode, et préférentiellement un atome de fluor ou de chlore.

Par groupe acyle en $C_2$-$C_6$, on entend un groupe R-CO-, dans lequel R représente un groupe alkyle tel que défini précédemment ayant de 1 à 5 atomes de carbone. Des exemples de groupes acyle en $C_2$-$C_6$ sont notamment les groupes acétyle, propanoyle, butanoyle, pentanoyle, hexanoyle, ainsi que leurs homologues dans lesquels la chaîne peut être ramifiée.

Par groupe alcoxy en $C_1$-$C_4$ on entend un groupe RO-, dans lequel R présente un groupe alkyle ayant de 1 à 4 atomes de carbone tel que défini précédemment. A titre d'exemples de groupes alcoxy en $C_1$-$C_4$ on peut citer les groupes méthoxy, éthoxy, propoxy, butoxy, 1-méthyléthoxy, 1,1-diméthyléthoxy, 1-méthylpropoxy, 2-méthylpropoxy ou cyclopropylméthoxy.

Par sels d'addition, on entend les sels d'addition obtenus par réaction d'un composé de formule I avec un acide minéral ou organique. De préférence, il s'agit des sels d'addition pharmaceutiquement acceptables. Les hydrates ou solvates des composés de formule I ou des sels des composés de formule I font également partie intégrante de l'invention.

Parmi les acides minéraux convenant pour salifier un composé basique de formule I, on préfère les acides chlorhydrique, bromhydrique, phosphorique et sulfurique. Parmi les acides organiques convenant pour salifier un composé basique de formule I, on préfère les acides méthanesulfonique, benzènesulfonique, toluènesulfonique, maléïque, fumarique, oxalique, citrique, tartrique, lactique et trifluoroacétique.

A titre d'exemples particuliers de groupes bicycliques fusionnés représentés par A dans le cas où $R_1$ et $R_2$ forment ensemble un cycle aromatique contenant 6 atomes de carbone, on peut citer les groupes benzofuranyle, benzothiényle, benzisoxazolyle, benzoxazolyle, benzimidazolyle, quinolinyle, quinoxalinyle, quinazolinyle, indolyle, benzothiazolyle, indazolyle.

Parmi les composés selon la présente invention, on préfère tout particulièrement ceux dans lesquels le groupe thioxyloside est en position 3 du cycle pyridine.

Parmi les composés selon la présente invention, on préfère également les composés dans lesquels R est l'atome d'hydrogène ou le groupe $-COCH_3$.

D'autres composés préférés dans le cadre de la présente invention sont les composés de formule I précitée dans laquelle l'une ou moins des conditions suivantes est respectée :

- A représente un groupe pyridinyle non substitué ou substitué par l'un des groupes $R_1$, $R_2$ et $R_3$ tels que définis précédemment ;
- R' et R" représentent chacun un atome d'hydrogène, un atome d'halogène ou un groupe méthyle.

Les composés de formule I selon l'invention peuvent être préparés en mettant en oeuvre les méthodes de glycosylation connues de l'homme de métier, notamment :

a) la méthode de HELFERICH décrite dans l'ouvrage « The Carbohydrate, Chemistry and Biochemistry », 2ème édition, Academic Press, New-York-Londres 1972, Tome IA pages 292-294, par condensation d'un sucre peracétylé avec un hydroxyhétérocycle aromatique en présence d'un acide de Lewis ;
b) la méthode de KOENIGS-KNORR (idem, pages 295-299) par condensation d'un acylose halogéné avec un groupe hydroxy à caractère phénolique en présence d'un accepteur de protons, tel que le cyanure mercurique, l'imidazolate d'argent ou le trifluorométhylsulfonate d'argent.
c) La méthode de SCHMIDT par condensation d'un trichloracétimidate d'osyle avec un hydroxyhétérocycle aromatique, en présence d'un acide de Lewis, tel que par exemple le trifluorométhanesulfonate de triméthylsilyle ou l'éthérate de trifluorure de bore.

Les composés de formule I sont préparés de préférence selon des méthodes dérivées des procédés mentionnés ci-dessus.
Selon un premier procédé général, on effectue les étapes consistant à :

a) faire réagir un pyridinol de formule :

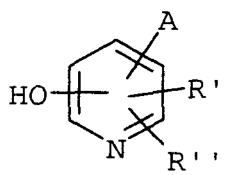

II

dans laquelle :

- R' et R" représentent chacun indépendamment, un atome d'hydrogène, un atome d'halogène, un groupe alkyle en $C_1$-$C_4$ ou un groupe 6-fluoro-3-pyridinyle,
- A représente un cycle aromatique à 5 ou 6 chaînons de formule :

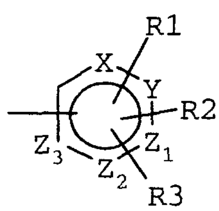

dans laquelle :
- X représente un atome d'azote, d'oxygène ou de soufre,
- Y représente un atome de carbone ou une liaison simple,
- $Z_1$, $Z_2$ et $Z_3$ représentent chacun indépendamment, un atome de carbone ou d'azote,
- $R_1$, $R_2$ et $R_3$ représentent chacun indépendamment, un atome d'hydrogène ou un atome d'halogène, un groupe cyano, un groupe alkyle en $C_1$-$C_4$ un groupe alcoxy en $C_1$-$C_4$, un groupe trifluorométhyle ; ou $R_1$ et $R_2$ forment ensemble avec les atomes de l'hétérocycle auxquels ils sont rattachés un cycle aromatique comprenant 6 atomes de carbone, A représentant ainsi un groupe bicyclique fusionné, en particulier un groupe benzofuranyle ou benzothiényle,

avec un dérivé du 5-thioxylopyranose de formule :

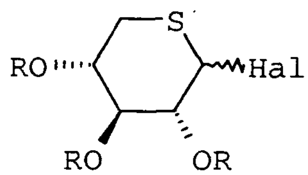

dans laquelle Hal représente un halogène, préférentiellement le brome et R représente un groupe acyle en $C_2$-$C_6$, préférentiellement le groupe acétyle, dans un solvant aprotique tel que l'acétonitrile ou le toluène, en présence d'un sel d'argent, notamment l'oxyde ou l'imidazolate d'argent ou d'un sel de zinc (notamment l'oxyde ou le chlorure) en milieu anhydre, à une température comprise entre 25 et 110°C et pendant 1 à 10 heures, pour obtenir le composé de formule:

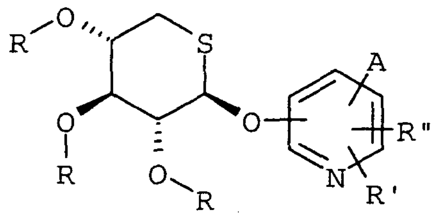

I

dans laquelle A, R, R' et R" conservent la même signification que dans les composés de départ ;

b) si nécessaire, faire réagir le composé de formule I obtenu ci-dessus avec une solution d'ammoniac dans du méthanol pour réaliser la désacylation et ainsi remplacer le groupe acyle par des atomes d'hydrogène et obtenir le composé de formule :

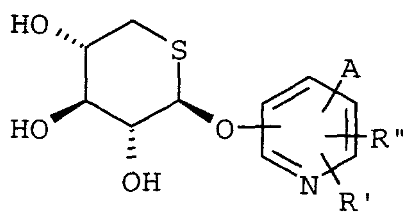

Ia

dans laquelle $R_1$ et $R_2$ conservent la même signification que ci-dessus ;

c) si nécessaire, faire réagir l'un des composés I ou Ia obtenus ci-dessus avec un acide selon des méthodes connues de l'homme de métier pour obtenir le sel d'addition correspondant.

En variante de l'étape b) décrite ci-dessus, le remplacement du groupe acyle par un atome d'hydrogène peut être réalisé par action d'un alcoolate métallique, préférentiellement le méthylate de sodium en quantité catalytique, dans le méthanol, à une température comprise entre 0 et 30°C et pendant 0,5 à 2 heures pour obtenir le composé de formule Ia à partir du composé de formule I dans laquelle R représente un groupe acyle en $C_2$-$C_6$.

Selon un second procédé, les composés de formule I peuvent être obtenus par action du tétra-O-acétyl-5-thioxylopyranose de formule :

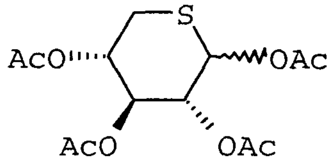

dans laquelle Ac représente le groupe acétyle avec un composé de formule :

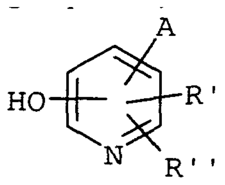

II

dans laquelle :

- R' et R" représentent chacun indépendamment, un atome d'hydrogène, un atome d'halogène, un groupe alkyle en $C_1$-$C_4$ ou un groupe 6-fluoro-3-pyridinyle,
- A représente un cycle aromatique à 5 ou 6 chaînons de formule :

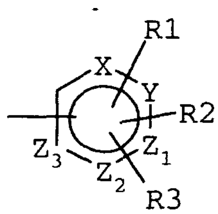

dans laquelle :

- X représente un atome d'azote, d'oxygène ou de soufre,
- Y représente un atome de carbone ou une liaison simple,
- $Z_1$, $Z_2$ et $Z_3$ représentent chacun indépendamment, un atome de carbone ou d'azote,
- $R_1$, $R_2$ et $R_3$ représentent chacun indépendamment, un atome d'hydrogène ou un atome d'halogène, un groupe cyano, un groupe alkyle en $C_1$-$C_4$, un groupe alcoxy en $C_1$-$C_4$, un groupe trifluorométhyle ; ou
- $R_1$ et $R_2$ forment ensemble avec les atomes de l'hétérocycle auxquels ils sont rattachés un cycle aromatique comprenant 6 atomes de carbone, A représentant ainsi un groupe bicyclique fusionné, en particulier un groupe benzofuranyle ou benzothiényle,

dans un solvant aprotique, tel que par exemple le dichlorométhane, en présence d'un catalyseur de type acide de Lewis, par exemple le tétrachlorure d'étain, à une température comprise entre 20 et 60 °C et pendant 1 à 2 heures, pour obtenir le composé de formule :

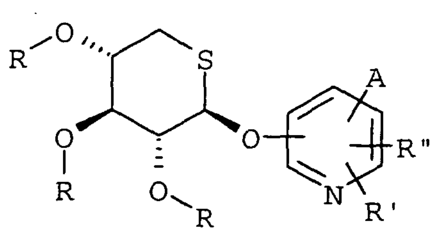

Ib

dans laquelle A, R, R' et R" conservent la même signification que dans les composés de départ.

Le composé de formule Ib peut ensuite être mis en réaction selon le protocole décrit dans le procédé précédent pour obtenir le composé pyranosyle non substitué et/ou un sel avec un acide.

Selon un troisième procédé, les composés de formule I peuvent être obtenus en faisant réagir un dérivé du thioxylose de formule :

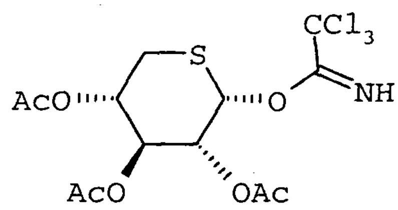

dans laquelle Ac représente le groupe acétyle, avec un composé de formule :

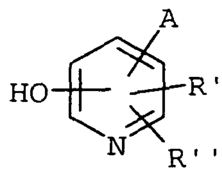

II

dans laquelle :

- R' et R" représentent chacun indépendamment, un atome d'hydrogène, un atome d'halogène, un groupe alkyle en $C_1$-$C_4$ ou un groupe 6-fluoro-3-pyridinyle,
- A représente un cycle aromatique à 5 ou 6 chaînons de formule :

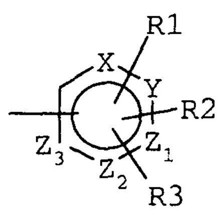

dans laquelle :

- X représente un atome d'azote, d'oxygène ou de soufre,
- Y représente un atome de carbone ou une liaison simple,
- $Z_1$, $Z_2$ et $Z_3$ représentent chacun indépendamment, un atome de carbone ou d'azote,
- $R_1$, $R_2$ et $R_3$ représentent chacun indépendamment, un atome d'hydrogène ou un atome d'halogène, un groupe cyano, un groupe alkyle en $C_1$-$C_4$, un groupe alcoxy en $C_1$-$C_4$, un groupe trifluorométhyle ; ou
- $R_1$ et $R_2$ forment ensemble avec les atomes de l'hétérocycle auxquels ils sont rattachés un cycle aromatique comprenant 6 atomes de carbone, A représentant ainsi un groupe bicyclique fusionné, en particulier un groupe benzofuranyle ou benzothiényle,

dans un solvant aprotique, tel que le dichlorométhane, en présence d'un catalyseur tel que le trifluorométhanesulfonate de triméthylsilyle, à une température comprise entre -25°C et la température ambiante et pendant 1 à 5 heures pour obtenir le thioxylopyranoside de formule :

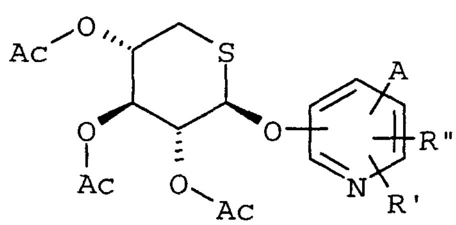

Ib

dans laquelle A, R' et R" conservent la même signification que dans les composés de départ.

Le composé de formule Ib ainsi obtenu peut être ensuite mis en réaction comme précédemment pour obtenir

les composés pyranosyle non substitué et/ou les sels d'acide.

Les composés de formules I selon l'invention peuvent également être préparés à partir de dérivés halogénés d'une pyridine glycosylée, par une réaction de couplage selon Suzuki entre deux cycles aromatiques.

Selon un procédé général, on effectue les étapes consistant à :

a) faire réagir un composé de formule :

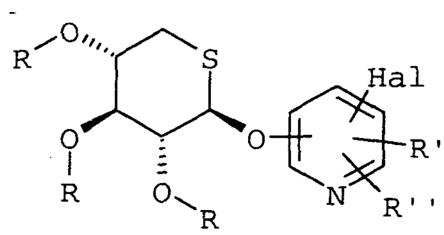

dans laquelle Hal est un atome d'halogène, préférentiellement le brome ou l'iode, R' et R" représentent chacun indépendamment un atome d'hydrogène, un atome d'halogène (autre que le brome ou l'iode), ou un groupe alkyle en $C_1$-$C_4$, et R représente un atome d'hydrogène ou un groupe acyle en $C_2$-$C_6$ ;
avec un acide hétéroarylboronique ou un hétéroarylboronate d'alkyle de formule :

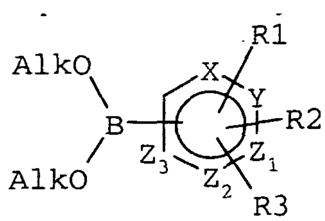

dans laquelle :

- X représente un atome d'azote, d'oxygène ou de soufre,
- Y représente un atome de carbone ou une liaison simple,
- $Z_1$, $Z_2$ et $Z_3$ représentent chacun indépendamment, un atome de carbone ou d'azote,
- $R_1$, $R_2$ et $R_3$ représentent chacun indépendamment, un atome d'hydrogène ou un atome d'halogène, de préférence un atome de fluor, un groupe cyano, un groupe alkyle en $C_1$-$C_4$, un groupe alcoxy en $C_1$-$C_4$, un groupe trifluorométhyle ; ou
- $R_1$ et $R_2$ forment ensemble avec les atomes de l'hétérocycle auxquels ils sont rattachés un cycle aromatique comprenant 6 atomes de carbone,
- Alk représente un atome d'hydrogène ou un groupe alkyle en $C_1$-$C_4$.

en présence d'un catalyseur au palladium, tel que le [1,1-bisdiphényl-phosphinoferrocène]dichloropalladium dichlorométhane, un catalyseur au palladium immobilisé sur résine ou le catalyseur d'Herrmann, d'un solvant polaire tel que le méthanol ou un éther de glycol, et du fluorure de césium ou du carbonate de sodium ou d'autres bases minérales éventuellement additionné de chlorure de lithium, à une température comprise entre 70°C et 150°C pendant 5 minutes à 72 heures à l'aide de micro-ondes ou d'un mode de chauffage classique, pour obtenir le composé de formule :

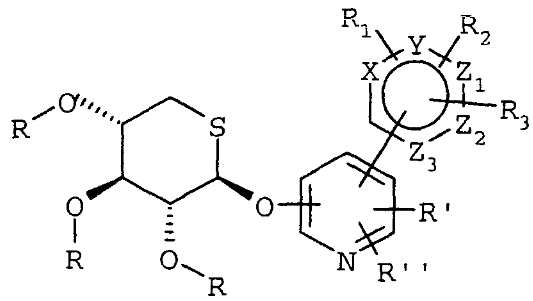

dans laquelle :

R, $R_1$, $R_2$, $R_3$, R', R", X, Y, $Z_1$, $Z_2$ et $Z_3$ conservent la même signification que dans les produits de départ.

Pour ce type de composés, un autre procédé proche consiste à faire réagir un acide pyridineboronique glycosylé ou un pyridinylboronate glycosylé de formule:

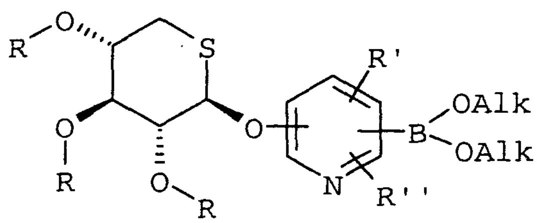

dans laquelle R représente un atome d'hydrogène ou un groupe acyle en $C_2$-$C_6$, R' et R" représentent chacun indépendamment un atome d'hydrogène, un atome d'halogène (autre que le brome ou l'iode), ou un groupe alkyle en $C_1$-$C_4$ et Alk représente un atome d'hydrogène ou un groupe alkyle en $C_1$-$C_{4.}$ avec un halogénure d'hétéroaryle de formule:

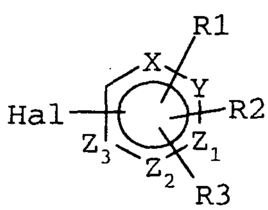

dans laquelle Hal représente un halogène, préférentiellement le brome ou l'iode, et $R_1$, $R_2$ et $R_3$ représentent chacun indépendamment, un atome d'hydrogène ou un atome d'halogène, de préférence un atome de fluor, un groupe cyano, un groupe alkyle en $C_1$-$C_4$, un groupe alcoxy en $C_1$-$C_4$, un groupe trifluorométhyle ; ou $R_1$ et $R_2$ forment ensemble avec les atomes de l'hétérocycle auxquels ils sont rattachés un cycle aromatique comprenant 6 atomes de carbone, dans les mêmes conditions que précédemment, pour obtenir le composé de formule:

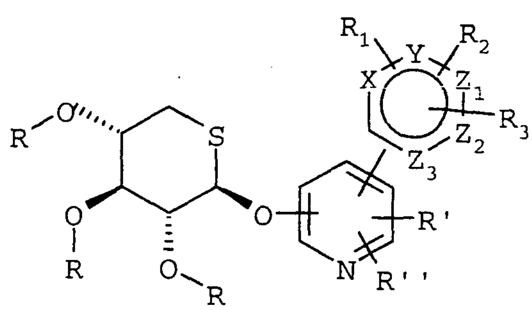

dans laquelle :

R, $R_1$, $R_2$, $R_3$, R', R", X, Y, $Z_1$, $Z_2$ et $Z_3$ conservent la même signification que dans les produits de départ.

D'une façon générale on préfère utiliser le bromure de 2,3,4-tri-O-acétyl-5-thio-α-D-xylopyranosyle ou le tétra-O-acétyl-5-thio-α-D-xylopyranose quand il s'agit d'obtenir un dérivé du β-D-5-thio-xylopyranose.

Les réactions de glycosylation décrites précédemment conduisent le plus souvent à un mélange des isomères de configuration α et β et il est généralement nécessaire d'optimiser les conditions opératoires afin d'obtenir des proportions favorables à l'isomère de configuration β. Pour cette même raison, il peut être aussi nécessaire d'effectuer des purifications soit par recristallisation, soit par chromatographie, pour obtenir l'isomère β pur.

Les exemples suivants ont pour but d'illustrer l'invention. Les points de fusion ont été mesurés au banc Kofler ou capillaire et les valeurs spectrales de Résonance Magnétique Nucléaire sont caractérisées par le déplacement chimique calculé par rapport au TMS, par le nombre de protons associés au signal et par la forme du signal (s pour singulet, d pour doublet, t pour triplet, q pour quadruplet, m pour multiplet). La fréquence de travail et le solvant utilisé sont indiqués pour chaque composé.

Les abréviations suivantes ont été utilisées :

mM signifie millimole ($10^{-3}$ mole)
DMSO désigne le diméthylsulfoxyde
THF désigne le tétrahydrofurane
$CHCl_3$ désigne le chloroforme
DME désigne le diméthoxyéthane

Le groupe "pinacolborane" signifie:

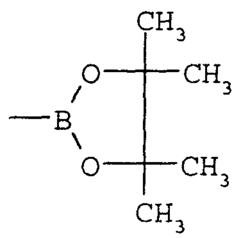

**Préparation I:**

**5,6-dichloro-3-pyridinyl 2,3,4-tri-O-acétyl-5-thio-β-D-xylopyranoside**

On fait fondre, sous pression réduite, 5,1 g (37,5 mM) de chlorure de zinc, on refroidit sous atmosphère inerte, puis on ajoute 12 ml de toluène, 12 ml d'acétonitrile, 3 g de tamis moléculaire 4Å et 2,45 g (15 mM) de 5,6-dichloro-3-pyridinol. La température du mélange est portée à 90°C et on ajoute 3,78 g (37,5 mM) de triéthylamine et 5,86 g (16,5 mM) de bromure de 2,3,4-tri-O-acétyl-5-thio-α-D-xylopyranosyle. On agite à 90°C pendant 20 minutes puis le milieu réactionnel est refroidi, filtré pour éliminer les sels minéraux que l'on lave à l'acétate d'éthyle. Les phases organiques réunies sont lavées avec une solution de soude 0,5 N puis le pH est amené à une valeur de 3 à l'aide d'une solution d'acide chlorhydrique 0,1N. La phase organique est ensuite lavée avec une solution de chlorure de sodium saturée, séchée sur sulfate de magnésium et concentrée sous pression réduite. On cristallise le produit dans l'éther éthylique et on obtient le produit désiré sous forme d'un solide marron clair avec un rendement de 50 %.
F = 128°C.

$[\alpha]_{D}^{27} = -92°$ c = 0,23 ; $CHCl_3$).

**Préparation II:**

**4-bromo-3-pyridinyl 2,3,4-tri-O-acétyl-5-thio-β-D-xylopyranoside**

En opérant de façon analogue à la préparation I, au départ de 4-bromo-3-pyridinol, on obtient le produit désiré

sous forme d'une poudre jaune (rendement = 38 %).
F = 153°C.

$[\alpha]_D^{30} = -69°$ (c = 0,31 ; DMSO).

**Préparation III:**

**2-bromo-3-pyridinyl 2,3,4-tri-O-acétyl-5-thio-β-D-xylopyranoside**

En opérant de façon analogue à la préparation I, au départ de 2-bromo-3-pyridinol, on obtient le produit désiré sous forme d'une poudre blanche (rendement = 41 %).
F = 156°C.

$[\alpha]_D^{24} = -78°$ (c = 0,40 ; $CH_3OH$).

**Préparation IV:**

**6-bromo-3-pyridinyl 2,3,4-tri-O-acétyl-5-thio-β-D-xylopyranoside**

En opérant de façon analogue à la préparation I, au départ de 6-bromo-3-pyridinol, on obtient le produit désiré sous forme d'une poudre beige (rendement = 43 %).
F = 145°C.

$[\alpha]_D^{29} = -20°$ (c = 0,52 ; DMSO).

**Préparation V:**

**2-iodo-6-méthyl-3-pyridinyl 2,3,4-tri-O-acétyl-5-thio-β-D-xylopyranoside**

En opérant de façon analogue à la préparation I, au départ de 2-iodo-6-méthyl-3-pyridinol, on obtient le produit désiré sous forme d'une poudre blanche (rendement = 81 %).
F = 187°C.

$[\alpha]_D^{30} = -88°$ (c = 0,28 ; DMSO).

**Préparation VI:**

**2-chloro-4-méthyl-3-pyridinyl 2,3,4-tri-O-acétyl-5-thio-β-D-xylopyranoside**

En opérant de façon analogue à la préparation I, au départ de 2-chloro-4-méthyl-3-pyridinol, on obtient le produit désiré sous forme d'une poudre blanche (rendement = 30 %).
F = 144°C.

$[\alpha]_D^{30} = +45°$ (c = 0,37 ; DMSO).

**Préparation VII:**

**2-bromo-4-pyridinyl 2,3,4-tri-O-acétyl-5-thio-β-D-xylopyranoside**

En opérant de façon analogue à la préparation I, au départ de 2-bromo-4-pyridinol, on obtient le produit désiré sous forme d'un solide blanc (rendement = 37 %).
F = 162°C (recristallisé dans l'éther).

$[\alpha]_D^{29} = -11°$ (c = 0,48 ; DMSO).

**Préparation VIII:**

**5-bromo-2-fluoro-3-pyridinyl 2,3,4-tri-O-acétyl-5-thio-β-D-xylopyranoside**

En opérant de façon analogue à la préparation I, au départ de 5-bromo-2-fluoro-3-pyridinol, on obtient le produit désiré sous forme de cristaux blancs (rendement = 39 %).
F = 120-122°C. (recristallisé dans l'isopropanol)

**Préparation IX:**

**5-bromo-3-pyridinyl 2,3,4-tri-O-acétyl-5-thio-β-D-xylopyranoside**

En opérant de façon analogue à la préparation I, au départ de 5-bromo-3-pyridinol, on obtient le produit désiré sous forme d'une poudre marron clair (rendement = 55 %).
F = 174°C.
$[\alpha]_D^{20} = -20°$ (c = 0,23 ; DMSO).

**Préparation X:**

**2-chloro-6-iodo-3-pyridinyl 2,3,4-tri-O-acétyl-5-thio-β-D-xylopyranoside**

En opérant de façon analogue à la préparation I, au départ de 2-chloro-6-iodo-3-pyridinol, on obtient le produit désiré sous forme d'une poudre blanche (rendement = 53 %).
F = 188°C (recristallisé dans l'éther éthylique).
$[\alpha]_D^{30} = -34°$ (c = 0,39 ; DMSO).

**Préparation XI:**

**6-chloro-5-fluoro-3-pyridinyl 2,3,4-tri-O-acétyl-5-thio-β-D-xylopyranoside**

En opérant de façon analogue à la préparation I, au départ de 6-chloro-5-fluoro-3-pyridinol, on obtient le produit désiré sous forme de paillettes blanches (rendement = 43 %).
F = 158-162°C (recristallisé dans l'éthanol).
$[\alpha]_D^{27} = -21°$ (c = 0,33 ; DMSO).

**Préparation XII:**

**2-fluoro-6-iodo-3-pyridinyl 2,3,4-tri-O-acétyl-5-thio-β-D-xylopyranoside**

En opérant de façon analogue à la préparation I, au départ de 2-fluoro-6-iodo-3-pyridinol, on obtient le produit désiré sous forme d'une poudre blanche (rendement = 41 %).
F = 234°C (recristallisé dans l'éther éthylique).
$[\alpha]_D^{30} = -10°$ (c = 0,49 ; DMSO).

**Préparation XIII:**

**6-chloro-2-pyridinyl 2,3,4-tri-O-acétyl-5-thio-β-D-xylopyranoside**

En opérant de façon analogue à la préparation I, au départ de 6-chloro-2-pyridinol, on obtient le produit désiré sous forme d'une poudre blanche (rendement = 20 %).
F = 127°C.
$[\alpha]_D^{29} = -72°$ (c = 0,33 ; $CHCl_3$).

**Préparation XIV:**

**5-bromo-2-chloro-3-pyridinyl 2,3,4-tri-O-acétyl-5-thio-β-D-xylopyranoside**

En opérant de façon analogue à la préparation 1, au départ de 5-bromo-2-chloro-3-pyridinol, on obtient le produit désiré sous forme de cristaux beige (rendement = 38 %).
F = 143-147°C. (cristallisé dans l'éther éthylique)

$[\alpha]_D^{24} = -35°$ (c = 0,15 ; DMSO).

**Préparation XV**

**2-cyano-3-pyridinyl 2,3,4-tri-O-acétyl-5-thio-β-D-xylopyranoside**

On prépare une solution de 1,7 g (14,17 mM) de 2-cyano-3-pyridinol dans 80 ml d'acétonitrile et on ajoute, à l'abri de la lumière, 4,3 g (18,3 mM) d'oxyde d'argent et 3 g de tamis moléculaire 13X. Le mélange est agité pendant 10 mn à 50°C puis on ajoute 6,5 g (18,3 mM) de bromure de 2,3,4-tri-O-acétyl-D-xylopyranoside et on maintient le mélange réactionnel pendant 18 heures sous agitation à 50°C. Le mélange est ensuite refroidi à température ambiante et filtré sur adjuvant de filtration. Le filtrat est dilué avec de l'acétate d'éthyle et lavé avec de l'eau, une solution N de soude puis à l'eau jusqu'à pH neutre, et enfin séché sur sulfate de magnésium et concentré sous pression réduite. L'huile résiduelle est cristallisée par addition d'éther éthylique. On obtient 0,89 g du produit attendu sous forme de cristaux beiges (rendement = 16 %).
RMN $^1H$ (300 MHz ; $CDCl_3$) δ : 8,43 (m, 1H) ; 7,53 (m, 2H) ; 5,49 (t, 1H) ; 5,30 (d, 1H) ; 5,19 (m, 2H) ; 3,18 (m, 1H) ; 2,76 (m, 1H) ; 2,10 (m, 9H).
Le produit contient une faible proportion de dérivés α, dont le proton anomère donne des signaux à δ = 5,76 et δ = 5,63).

**Exemple 1:**

**6-(2-méthoxy-3-pyridinyl)-3-pyridinyl 2,3-tri-O-acétyl-5-thio-β-D-xylopyranoside**

A une solution de 1 g (2,23 mM) de 6-bromo-3-pyridinyl 2,3,4-tri-O-acétyl-5-thio-β-D-xylopyranoside obtenu selon la preparation IV dans 10 ml de DME, on ajoute une solution de 0,354 g (3,34 mM) de carbonate de sodium dans 3 ml d'eau, 0,18 g (0,223 mM) de [1,1'-bis(diphénylphosphino)ferrocène]dichloropalladium(II)-dichlorométhane et 0,68 g (4,46 mM) d'acide 2-méthoxy-3-pyridineboronique. Le mélange réactionnel est chauffé à l'aide de micro-ondes à 120°C pendant 20 minutes, refroidi, additionné d'eau et extrait à l'acétate d'éthyle. La phase organique est lavée par une solution de carbonate de sodium 1 M, puis à l'eau jusqu'à pH neutre, séchée sur sulfate de magnésium et concentrée sous pression réduite. Le résidu d'évaporation est purifié par chromatographie sur colonne de silice (éluant: toluène/acétone 90/10 ; v/v) pour obtenir le produit attendu sous forme d'un solide blanc avec un rendement de 70%.
F = 176°C.

$[\alpha]_D^{29} = +5°$ (c = 0,30; DMSO).

**Exemple 2:**

**6-(2-méthoxy-3-pyridinyl)-3-pyridinyl 5-thio-β-D-xylopyranoside**

On agite à température ambiante pendant 4 heures une solution de 0,3 g (0,63 mM) de 6-(2-méthoxy-3-pyridinyl)-3-pyridinyl 2,3,4-tri-O-acétyl-5-thio-β-D-xylopyranoside obtenu selon l'exemple 1, dans 4 ml d'une solution d'ammoniac 7 M dans le méthanol. Le mélange réactionnel est ensuite concentré sous pression réduite et le résidu d'évaporation est cristallisé dans de l'éther. On obtient le produit désiré sous la forme d'un solide blanc avec un rendement de 81%.
F = 127°C.

$[\alpha]_D^{29} = -45°$ (c = 0,26 ; DMSO).

**Exemple 3:**

**4-méthyl-2-(3-pyridinyl)-3-pyridinyl 2,3,4-tri-O-acétyl-5-thio-β-D-xylopyranoside**

En opérant de façon analogue à l'exemple 1, au départ de 2-chloro-4-méthyl-3-pyridinyl 2,3,4-tri-O-acétyl-5-thio-β-D-xylopyranoside obtenu selon la préparation VI et d'acide 3-pyridineboronique, on obtient le produit désiré sous forme d'une mousse blanche (rendement = 27 %).
F = 143°C.
$[\alpha]_D^{33} = +29°$ (c = 0,43 ; DMSO).

**Exemple 4:**

**4-méthyl-2-(3-pyridinyl)-3-pyridinyl 5-thio-β-D-xylopyranoside**

En opérant de façon analogue à l'exemple 2, au départ du produit préparé selon l'exemple 3, on obtient le produit désiré sous forme d'un coton blanc (rendement = 40 %).
F = 98°C.
$[\alpha]_D^{29} = +66°$ (c = 0,20 ; DMSO).

**Exemple 5:**

**2-(3-furanyl)-6-méthyl-3-pyridinyl 2,3,4-tri-O-acétyl-5-thio-β-D-xylopyranoside**

En opérant de façon analogue à l'exemple 1, au départ de 2-iodo-6-méthyl-3-pyridinyl 2,3,4-tri-O-acétyl-5-thio-β-D-xylopyranoside obtenu selon la préparation V et d'acide 3-furaneboronique, on obtient le produit désiré sous forme d'un solide blanc (rendement = 42 %).
F = 117°C.
$[\alpha]_D^{25} = -73°$ (c = 0,10 ; DMSO).

**Exemple 6:**

**2-(3-furanyl)-6-méthyl-3-pyridinyl 5-thio-β-D-xylopyranoside**

En opérant de façon analogue à l'exemple 2, au départ du produit préparé selon l'exemple 5, on obtient le produit désiré sous forme d'un solide blanc crème (rendement = 81 %).
F = 162°C.
$[\alpha]_D^{29} = -117°$ (c = 0,10 ; DMSO).

**Exemple 7:**

**2-(5-méthyl-2-furanyl)-3-pyridinyl 2,3,4-tri-O-acétyl-5-thio-β-D-xylopyranoside**

En opérant de façon analogue à l'exemple 1, au départ de 2-bromo-3-pyridinyl 2,3,4-tri-O-acétyl-5-thio-β-D-xylopyranoside obtenu selon la préparation III et d'acide 5-méthyl-2-furaneboronique, on obtient le produit désiré sous forme d'un solide blanc (rendement = 45 %).
F = 128°C.
$[\alpha]_D^{32} = -68°$ (c = 0,27 ; DMSO).

**Exemple 8:**

**2-(5-méthyl-2-furanyl)-3-pyridinyl 5-thio-β-D-xylopyranoside**

En opérant de façon analogue à l'exemple 2, au départ du produit préparé selon l'exemple 7, on obtient le

produit désiré sous forme d'un solide blanc (rendement = 83 %).
F = 191°C.

$[\alpha]_D^{30} = -103°$ (c = 0,28 ; DMSO).

**Exemple 9:**

**2-chloro-6-(3-pyridinyl)-3-pyridinyl 2,3,4-tri-O-acétyl-5-thio-β-D-xylopyranoside**

En opérant de façon analogue à l'exemple 3, au départ de 2-chloro-6-iodo-3-pyridinyl 2,3,4-tri-O-acétyl-5-thio-β-D-xylopyranoside obtenu selon la préparation X, on obtient le produit désiré sous forme d'une poudre beige (rendement = 34 %).
F = 155°C.

$[\alpha]_D^{36} = -38°$ (c = 0,13 ; DMSO).

**Exemple 10:**

**2-chloro-6-(3-pyridinyl)-3-pyridinyl 5-thio-β-D-xylopyranoside**

En opérant de façon analogue à l'exemple 2, au départ du produit préparé selon l'exemple 9, on obtient le produit désiré sous forme d'une poudre beige (rendement = 35 %).
F = 180°C.

$[\alpha]_D^{35} = -51°$ (c = 0,11 ; DMSO).

**Exemple 11:**

**2-fluoro-5-(4-pyridinyl)-3-pyridinyl 2,3,4-tri-O-acétyl-5-thio-β-D-xylopyranoside**

En opérant de façon analogue à l'exemple 1, au départ de 5-bromo-2-fluoro-3-pyridinyl 2,3,4-tri-O-acétyl-5-thio-β-D-xylopyranoside obtenu selon la préparation VIII et d'acide 4-pyridineboronique, on obtient le produit désiré sous forme d'une mousse beige (rendement = 53 %).
F = 143-144°C.

$[\alpha]_D^{30} = +8°$ (c = 0,26 ; DMSO).

**Exemple 12:**

**2-fluoro-5-(4-pyridinyl)-3-pyridinyl 5-thio-β-D-xylopyranoside**

En opérant de façon analogue à l'exemple 2, au départ du produit préparé selon l'exemple 11, on obtient le produit désiré sous forme d'une mousse rose (rendement = 59 %).
F = 98-102°C.

$[\alpha]_D^{30} = -61°$ (c = 0,28 ; DMSO).

**Exemple 13:**

**2-chloro-6-(2-méthoxy-3-pyridinyl)-3-pyridinyl 2,3,4-tri-O-acétyl-5-thio-β-D-xylopyranoside**

En opérant de façon analogue à l'exemple 1, au départ de 2-chloro-6-iodo-3-pyridinyl 2,3.4-tri-O-acétyl-5-thio-β-D-xylopyranoside obtenu selon la préparation X et d'acide 2-méthoxy-3-pyridineboronique, on obtient le produit désiré sous forme d'un solide rose (rendement = 40 %).
F = 221°C.

$[\alpha]_D^{30} = -44°$ (c = 0,20 ; DMSO).

**Exemple 14:**

**2-chloro-6-(2-méthoxy-3-pyridinyl)-3-pyridinyl 5-thio-β-D-xylopyranoside**

En opérant de façon analogue à l'exemple 2, au départ du produit préparé selon l'exemple 13, on obtient le produit désiré sous forme d'un coton blanc (rendement = 60 %).
F = 102°C (recristallisé dans un mélange méthanol/eau).

$[\alpha]_D^{30} = -53°$ (c = 0,16 ; DMSO).

**Exemple 15:**

**2-chloro-6-(2-fluoro-3-pyridinyl)-3-pyridinyl 2,3,4-tri-O-acétyl-5-thio-β-D-xylopyranoside**

En opérant de façon analogue à l'exemple 1, au départ de 2-chloro-6-iodo-3-pyridinyl 2,3,4-tri-O-acétyl-5-thio-β-D-xylopyranoside obtenu selon la préparation X et d'acide 2-fluoro-3-pyridineboronique, on obtient le produit désiré sous forme d'une poudre blanche (rendement = 27 %).
F = 227°C (recristallisé dans l'éthanol).

$[\alpha]_D^{30} = -38°$ (c = 0,61 ; DMSO).

**Exemple 16:**

**2-chloro-6-(2-fluoro-3-pyridinyl)-3-pyridinyl 5-thio-β-D-xylopyranoside**

En opérant de façon analogue à l'exemple 2, au départ du produit préparé selon l'exemple 15, on obtient le produit désiré sous forme d'un coton blanc (rendement = 80 %).
F = 153°C (recristallisé dans un mélange éthanol/eau).

$[\alpha]_D^{30} = -28°$ (c = 0,48 ; DMSO).

**Exemple 17:**

**2-chloro-6-(2-fluoro-4-pyridinyl)-3-pyridinyl 2,3,4-tri-O-acétyl-5-thio-β-D-xylopyranoside**

En opérant de façon analogue à l'exemple 1, au départ de 2-chloro-6-iodo-3-pyridinyl 2.3,4-tri-O-acétyl-5-thio-β-D-xylopyranoside obtenu selon la préparation X et d'acide 2-fluoro-4-pyridineboronique, on obtient le produit désiré sous forme d'une poudre blanche (rendement = 37 %).
F = 226°C (recristallisé dans un mélange eau/acétonitrile).

$[\alpha]_D^{32} = -34°$ (c = 0,31 ; DMSO).

**Exemple 18:**

**2-chloro-6-(2-fluoro-4-pyridinyl)-3-pyridinyl 5-thio-β-D-xylopyranoside**

En opérant de façon analogue à l'exemple 2, au départ du produit préparé selon l'exemple 17, on obtient le produit désiré sous forme d'une poudre blanche (rendement = 27 %).
F = 195°C (recristallisé dans un mélange isopropanol/eau).

$[\alpha]_D^{32} = -41°$ (c = 0,18 ; DMSO).

**Exemple 19:**

**2-chloro-6-(6-fluoro-3-pyridinyl)-3-pyridinyl 2,3,4-tri-O-acétyl-5-thio-β-D-xylopyranoside**

En opérant de façon analogue à l'exemple 1, au départ de 2-chloro-6-iodo-3-pyridinyl 2,3,4-tri-O-acétyl-5-thio-β-D-xylopyranoside obtenu selon la préparation X et d'acide 6-fluoro-3-pyridineboronique, on obtient le produit désiré sous forme d'une poudre blanche (rendement = 43 %).
F = 226˚C (recristallisé dans un mélange eau/acétonitrile).

$[\alpha]_D^{32} = -23°$ (c = 0,26 ; DMSO).

**Exemple 20:**

**2-chloro-6-(6-fluoro-3-pyridinyl)-3-pyridinyl 5-thio-β-D-xylopyranoside**

En opérant de façon analogue à l'exemple 2, au départ du produit préparé selon l'exemple 19, on obtient le produit désiré sous forme d'aiguilles blanches (rendement = 27 %).
F = 199˚C (recristallisé dans un mélange éthanol/eau).

$[\alpha]_D^{32} = -33°$ (c = 0,33 ; DMSO).

**Exemple 21:**

**2-fluoro-6-(4-pyridinyl)-3-pyridinyl 2,3,4-tri-O-acétyl-5-thio-β-D-xylopyranoside**

En opérant de façon analogue à l'exemple 11, au départ de 2-fluoro-6-iodo-3-pyridinyl 2,3,4-tri-O-acétyl-5-thio-β-D-xylopyranoside obtenu selon la préparation XII, on obtient le produit désiré sous forme d'un coton blanc (rendement = 47 %).
F = 164˚C (recristallisé dans un mélange eau/acétonitrile).

$[\alpha]_D^{30} = -2°$ (c = 0,14 ; DMSO).

**Exemple 22:**

**2-fluoro-6-(4-pyridinyl)-3-pyridinyl 5-thio-β-D-xylopyranoside**

En opérant de façon analogue à l'exemple 2, au départ du produit préparé selon l'exemple 21, on obtient le produit désiré sous forme d'une poudre blanche (rendement = 34 %).
F = 196˚C (recristallisé dans un mélange méthanol/eau).

$[\alpha]_D^{30} = -40°$ (c = 0,38 ; DMSO).

**Exemple 23:**

**2-chloro-6-(4-pyridinyl)-3-pyridinyl 2,3,4-tri-O-acétyl-5-thio-β-D-xylopyranoside**

En opérant de façon analogue à l'exemple 11, au départ de 2-chloro-6-iodo-3-pyridinyl 2,3,4-tri-O-acétyl-5-thio-β-D-xylopyranoside obtenu selon la préparation X, on obtient le produit désiré sous forme d'un solide blanc (rendement = 13 %).
F = 179˚C (recristallisé dans l'éther éthylique).

$[\alpha]_D^{30} = -31°$ (c = 0,30 ; DMSO).

**Exemple 24:**

**2-chloro-6-(4-pyridinyl)-3-pyridinyl 5-thio-β-D-xylopyranoside**

En opérant de façon analogue à l'exemple 2, au départ du produit préparé selon l'exemple 23, on obtient le produit désiré sous forme d'une poudre jaune (rendement = 47 %).
F = 186˚C (recristallisé dans un mélange méthanol/eau).

$[\alpha]_D^{30} = -36°$ (c = 0,17 ; DMSO).

**Exemple 25:**

**5-(2-chloro-3-pyridinyl)-3-pyridinyl 2,3,4-tri-O-acétyl-5-thio-β-D-xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et d'acide 2-chloro-3-pyridineboronique, on obtient le produit désiré sous forme d'un solide blanc (rendement = 21 %).
F = 162°C.

$[\alpha]_D^{29} = -16°$ (c = 0,36 ; DMSO).

**Exemple 26:**

**5-(2-chloro-3-pyridinyl)-3-pyridinyl 5-thio-β-D-xylopyranoside**

En opérant de façon analogue à l'exemple 2, au départ du produit préparé selon l'exemple 25, on obtient le produit désiré sous forme d'un solide blanc (rendement = 79 %).
F = 215°C.

$[\alpha]_D^{29} = -47°$ (c = 0,35 ; DMSO).

**Exemple 27:**

**5-(6-méthoxy-3-pyridinyl)-3-pyridinyl 2,3,4-tri-O-acétyl-5-thio-β-D-xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et d'acide 6-méthoxy-3-pyridineboronique, on obtient le produit désiré sous forme d'un solide blanc (rendement = 72 %).
F = 172°C.

$[\alpha]_D^{29} = -9°$ (c = 0,24 ; DMSO).

**Exemple 28:**

**5-(6-méthoxy-3-pyridinyl)-3-pyridinyl 5-thio-β-D-xylopyranoside**

En opérant de façon analogue à l'exemple 2, au départ du produit préparé selon l'exemple 27, on obtient le produit désiré sous forme d'un solide blanc (rendement = 57 %).
F = 189°C.

$[\alpha]_D^{30} = -47°$ (c = 0,34 ; DMSO).

**Exemple 29:**

**5-(6-chloro-3-pyridinyl)-3-pyridinyl 2,3,4-tri-O-acétyl-5-thio-β-D-xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et d'acide 6-chloro-3-pyridineboronique, on obtient le produit désiré sous forme d'un solide blanc (rendement = 33 %).
F = 194°C.

$[\alpha]_D^{29} = -17°$ (c = 0,30 ; DMSO).

**Exemple 30:**

**5-(6-chloro-3-pyridinyl)-3-pyridinyl 5-thio-β-D-xylopyranoside**

En opérant de façon analogue à l'exemple 2, au départ du produit préparé selon l'exemple 29, on obtient le produit désiré sous forme d'un solide blanc (rendement = 72 %).
F = 211°C.

$[\alpha]_D^{30} = -46°$ (c = 0,45 ; DMSO).

**Exemple 31:**

**5-(2-méthoxy-3-pyridinyl)-3-pyridinyl 2,3,4-tri-O-acétyl-5-thio-β-D-xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et d'acide 2-méthoxy-3-pyridineboronique, on obtient le produit désiré sous forme d'un solide écru (rendement = 63 %).
F = 167°C.

$[\alpha]_D^{29} = -13°$ (c = 0,27 ; DMSO).

**Exemple 32:**

**5-(2-méthoxy-3-pyridinyl)-3-pyridiny 5-thio-β-D-xylopyranoside**

En opérant de façon analogue à l'exemple 2, au départ du produit préparé selon l'exemple 31, on obtient le produit désiré sous forme d'un solide blanc (rendement = 77 %).
F = 209°C.

$[\alpha]_D^{29} = -90°$ (c = 0,22 ; DMSO).

**Exemple 33:**

**6-(5-méthyl-2-furanyl)-3-pyridinyl 2,3,4-tri-O-acétyl-5-thio-β-D-xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et d'acide 5-méthyl-2-furaneboronique, on obtient le produit désiré sous forme d'un solide écru (rendement = 74 %).
F = 134°C.

$[\alpha]_D^{29} = +22°$ (c = 0,23 ; DMSO).

**Exemple 34:**

**6-(5-méthyl-2-furanyl)-3-pyridinyl 5-thio-β-D-xylopyranoside**

En opérant de façon analogue à l'exemple 2, au départ du produit préparé selon l'exemple 33, on obtient le produit désiré sous forme d'un solide fin écru (rendement = 71 %).
F = 168°C.

$[\alpha]_D^{30} = -38°$ (c = 0,30 ; DMSO).

**Exemple 35:**

**6-(6-méthyl-3-pyridinyl)-3-pyridinyl 2,3,4-tri-O-acétyl-5-thio-β-D-xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et d'acide 6-méthyl-3-pyridineboronique, on obtient le produit désiré sous

forme d'un solide blanc (rendement = 62 %).
F = 181°C.

$[\alpha]_D^{29} = +15°$ (c = 0,21 ; DMSO).

**Exemple 36:**

**6-(6-méthyl-3-pyridinyl)-3-pyridinyl 5-thio-β-D-xylopyranoside**

En opérant de façon analogue à l'exempte 2, au départ du produit préparé selon l'exemple 35, on obtient le produit désiré sous forme d'une poudre blanche (rendement = 48 %).
F = 228°C.

$[\alpha]_D^{29} = -46°$ (c = 0,31 ; DMSO).

**Exemple 37:**

**6-(6-chloro-3-pyridinyl)-3-pyridinyl 2,3,4-tri-O-acétyl-5-thio-β-D-xylopyranoside**

En opérant de façon analogue à l'exemple 1, au départ de 6-bromo-3-pyridinyl 2.3,4-tri-O-acétyl-5-thio-β-D-xylopyranoside obtenu selon la préparation IV et d'acide 6-chloro-3-pyridineboronique, on obtient le produit désiré sous forme d'un solide blanc (rendement = 41 %).
F = 204°C.

$[\alpha]_D^{29} = +5°$ (c = 0,22 ; DMSO).

**Exemple 38:**

**6-(6-chloro-3-pyridinyl)-3-pyridinyl 5-thio-β-D-xylopyranoside**

En opérant de façon analogue à l'exemple 2, au départ du produit préparé selon l'exemple 37, on obtient le produit désiré sous forme d'un solide blanc (rendement = 62 %).
F = 170°C (recristallisé dans l'eau).

$[\alpha]_D^{30} = -32°$ (c = 0,34 ; DMSO).

**Exemple 39:**

**6-(2-chloro-3-pyridinyl)-3-pyridinyl 2,3,4-tri-O-acétyl-5-thio-β-D-xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et d'acide 2-chloro-3-pyridineboronique, on obtient le produit désiré sous forme d'un solide écru (rendement = 43 %).
F = 180°C.

$[\alpha]_D^{29} = -8°$ (c = 0,42 ; DMSO).

**Exemple 40:**

**6-(2-chloro-3-pyridinyl)-3-pyridinyl 5-thio-β-D-xylopyranoside**

On mélange 0,394 g (0,82 mM) de produit obtenu selon l'exemple 39, 25 ml de méthanol et 0,025 ml d'une solution de méthylate de sodium à 8% dans le méthanol. On agite le mélange 40 minutes à température ambiante, on filtre et on sèche le précipité obtenu sous pression réduite à 40°C. On obtient le produit attendu sous forme d'une poudre blanchâtre avec un rendement de 93 %.
F = 203°C

$[\alpha]_D^{28} = -51°$ (c = 0,30 ; DMSO).

**Exemple 41:**

**5-chloro-6-(4-pyridinyl)-3-pyridinyl 2,3,4-tri-O-acétyl-5-thio-β-D-xylopyranoside**

On mélange 0,633 g (1,45 mM) de 5,6-dichloro-3-pyridinyl 2,3,4-tri-O-acétyl-5-thio-β-D-xylopyranoside obtenu selon la préparation I, 0,214 g (1,74 mM) d'acide 4-pyridineboronique, 0,595 g (3,9 mM) de fluorure de césium et 0,118 g (0,145 mM) de [1,1'-bis(diphénylphosphino)ferrocène]dichloropalladium(II)-dichlorométhane dans 6 ml de DME. Le mélange est chauffé à 125°C pendant 90 minutes à l'aide de micro-ondes. Le mélange refroidi est filtré, rincé au méthanol et les phases organiques réunies sont concentrées sous pression réduite. Le résidu d'évaporation est purifié par chromatographie sur colonne de silice en éluant à l'aide d'une mélange dichlorométhane/acétate d'éthyle (80/20 puis 50/50 ; v/v). On obtient le produit attendu sous forme d'un solide marron clair avec un rendement de 20%.
F = 153°C.

$[\alpha]_D^{27} = -52°$ (c = 0,10; $CHCl_3$).

**Exemple 42:**

**5-chloro-6-(4-pyridinyl)-3-pyridinyl 5-thio-β-D-xylopyranoside**

On mélange 0,315 g (0,656 mM) de produit obtenu selon l'exemple 41, 20 ml de méthanol et 0,02 ml d'une solution de méthylate de sodium à 8% dans le méthanol. On agite le mélange 40 minutes à température ambiante, on filtre et on sèche le précipité obtenu sous pression réduite à 50°C. On obtient le produit attendu sous forme d'une poudre beige avec un rendement de 82%.
F = 226°C.

$[\alpha]_D^{28} = -49°$ (c = 0,15 ; DMSO).

**Exemple 43:**

**5-fluoro-6-(4-pyridinyl)-3-pyridinyl 2,3,4-tri-O-acétyl-5-thio-β-D-xylopyranoside**

En opérant de façon analogue à l'exemple 41, au départ de 6-chloro-5-fluoro-3-pyridinyl 2,3,4-tri-O-acétyl-5-thio-β-D-xylopyranoside obtenu selon la préparation XI, on obtient le produit désiré sous forme d'une poudre blanche (rendement = 38 %).
F = 165°C.

$[\alpha]_D^{29} = -28°$ (c = 0,23 ; DMSO).

**Exemple 44:**

**5-fluoro-6-(4-pyridinyl)-3-pyridinyl 5-thio-β-D-xylopyranoside**

En opérant de façon analogue à l'exemple 42, au départ du produit préparé selon l'exemple 43, on obtient le produit désiré sous forme d'une poudre grise (rendement = 55 %).
F = 192°C.

$[\alpha]_D^{29} = -54°$ (c = 0,12 ; DMSO).

**Exemple 45:**

**6-(4-pyridinyl)-2-pyridinyl 2,3,4-tri-O-acétyl-5-thio-β-D-xylopyranoside**

En opérant de façon analogue à l'exemple 41, au départ de 6-chloro-2-pyridinyl 2,3,4-tri-O-acétyl-5-thio-β-D-xylopyranoside obtenu selon la préparation XIII, on obtient le produit désiré sous forme d'une poudre crème (rendement = 65 %).
F = 137°C.

$[\alpha]_D^{28} = -41°$ (c = 0,32 ; $CHCl_3$).

**Exemple 46:**

**6-(4-pyridinyl)-2-pyridinyl 5-thio-β-D-xylopyranoside**

En opérant de façon analogue à l'exemple 42, au départ du produit préparé selon l'exemple 45, on obtient le produit désiré sous forme d'une poudre verdâtre (rendement = 98 %).
F = 178°C.

$[\alpha]_D^{29} = -23°$ (c = 0,28 ; DMSO).

**Exemple 47:**

**6-(3-pyridinyl)-2-pyridinyl 2,3,4-tri-O-acétyl-5-thio-β-D-xylopyranoside**

En opérant de façon analogue à l'exemple 41, au départ de 6-chloro-2-pyridinyl 2.3,4-tri-O-acétyl-5-thio-β-D-xylopyranoside obtenu selon la préparation XIII et d'acide 3-pyridineboronique, on obtient le produit désiré sous forme d'une poudre crème (rendement = 38 %).
F = 141 °C.

$[\alpha]_D^{29} = -31°$ (c = 0,26 ; $CHCl_3$).

**Exemple 48:**

**6-(3-pyridinyl)-2-pyridinyl 5-thio-β-D-xylopyranoside**

En opérant de façon analogue à l'exemple 42, au départ du produit préparé selon l'exemple 47, on obtient le produit désiré sous forme d'une poudre crème (rendement = 99 %).
F = 179°C.

$[\alpha]_D^{29} = -44°$ (c = 0,22 ; DMSO).

**Exemple 49:**

**5-(5-fluoro-2-pyridinyl)-3-pyridinyl 2,3,4-tri-O-acétyl-5-thio-β-D-xylopyranoside**

Sous atmosphère inerte, on chauffe 30 minutes à 110°C à l'aide de micro-ondes un mélange composé de 2,5 g (5,59 mM) de 5-bromo-3-pyridinyl 2,3,4-tri-O-acétyl-5-thio-β-D-xylopyranoside obtenu selon la préparation IX, 9 ml de DME, 0,136 g (0,166 mM) de [1,1'-bis(diphénylphosphino)ferrocène]dichloropalladium(II)-dichlorométhane, 2,12 g (8,34 mM) de bis(pinacolato)diborane et 1,64 g (16,7 mM) d'acétate de potassium. Après refroidissement, on filtre le milieu réactionnel et on additionne au filtrat 0,52 g (2,97 mM) de 2-bromo-5-tluoropyridine, 0,24 g (0,29 mM) de [1,1'-bis(diphénylphosphino)ferrocène]-dichloropalladium(II)-dichlorométhane et 4,45 ml d'une solution aqueuse de carbonate de potassium 1M. Le mélange est à nouveau chauffé 20 minutes à 120°C à l'aide de micro-ondes. Le milieu est refroidi, dilué par de l'eau et extrait à l'acétate d'éthyle. La phase organique est lavée par une solution aqueuse de bicarbonate de sodium puis par de l'eau, séchée sur sulfate de magnésium et concentrée sous pression réduite. Le résidu d'évaporation est purifié par chromatographie sur colonne de silice en éluant à l'aide d'une mélange toluène/acétone (80/20 ; v/v). Le produit obtenu est trituré avec de l'éther et filtré. On obtient le produit désiré sous forme de cristaux blancs avec un rendement de 71%.
F = 164-166°C.

$[\alpha]_D^{22} = -16°$ (c = 0,23 ; DMSO).

**Exemple 50:**

**5-(5-fluoro-2-pyridinyl)-3-pyridinyl 5-thio-β-D-xylopyranoside**

En opérant de façon analogue à l'exemple 42, au départ du produit préparé selon l'exemple 49, on obtient le produit désiré sous forme de cristaux blancs (rendement = 69 %).
F = 166-190°C (recristallisé dans l'eau).

$[\alpha]_D^{22} = -85°$ (c = 0,59 ; DMSO).

**Exemple 51:**

**2-fluoro-5-(3-pyridinyl)-3-pyridinyl 2,3,4-tri-O-acétyl-5-thio-β-D-xylopyranoside**

En opérant de façon analogue à l'exemple 49, au départ de 5-bromo-2-fluoro-3-pyridinyl 2,3,4-tri-O-acétyl-5-thio-β-D-xylopyranoside obtenu selon la préparation VIII et de 3-bromopyridine, on obtient le produit désiré sous forme d'un solide gris (rendement = 26 %).
F = 67-68°C.

$[\alpha]_D^{33} = -8°$ (c = 1,90 ; DMSO).

**Exemple 52:**

**2-fluoro-5-(3-pyridinyl)-3-pyridinyl 5-thio-β-D-xylopyranoside**

En opérant de façon analogue à l'exemple 2, au départ du produit préparé selon l'exemple 51, on obtient le produit désiré sous forme d'un solide blanc (rendement = 86 %).
F = 215-216°C.

$[\alpha]_D^{33} = -70°$ (c = 0,62 ; DMSO).

**Exemple 53:**

**5-(3-méthyl-4-pyridinyl)-3-pyridinyl 2,3,4-tri-O-acétyl-5-thio-β-D-xylopyranoside**

En opérant de façon analogue à l'exemple 49, au départ de 5-bromo-3-pyridinyl 2,3,4-tri-O-acétyl-5-thio-β-D-xylopyranoside obtenu selon la préparation IX et de 4-chloro-3-méthylpyridine, on obtient le produit désiré sous forme de cristaux beiges (rendement = 15 %).
F = 153-174°C (recristallisé dans l'éther).

$[\alpha]_D^{23} = -21°$ (c = 0,17 ; DMSO).

**Exemple 54:**

**5-(3-méthyl-4-pyridinyl)-3-pyridinyl 5-thio-β-D- xylopyranoside**

En opérant de façon analogue à l'exemple 42, au départ du produit préparé selon l'exemple 53, on obtient le produit désiré sous forme d'un coton beige (rendement = 50 %).
F = 194-214°C.

$[\alpha]_D^{23} = -58°$ (c = 0,20 ; DMSO).

**Exemple 55:**

**5-(3-méthyl-2-pyridinyl)-3-pyridinyl 2,3,4-tri-O-acétyl-5-thio-β-D- xylopyranoside**

En opérant de façon analogue à l'exemple 53, au départ de 2-bromo-3-méthylpyridine, on obtient le produit désiré sous forme d'un solide blanc (rendement = 17 %).
F = 148-150°C (recristallisé dans l'éther).

$[\alpha]_D^{32} = -22°$ (c = 0,25 ; DMSO).

**Exemple 56:**

**5-(3-méthyl-2-pyridinyl)-3-pyridinyl 5-thio-β-D- xylopyranoside**

En opérant de façon analogue à l'exemple 42, au départ du produit préparé selon l'exemple 55, on obtient le produit désiré sous forme d'un solide beige (rendement = 67 %).
F = 162-175°C.

$[\alpha]_D^{32} = -44°$ (c = 0,32 ; DMSO).

**Exemple 57:**

**5-(2,4-diméthyl-5-thiazolyl)-3-pyridinyl 2,3,4-tri-O-acétyl-5-thio-β-D- xylopyranoside**

En opérant de façon analogue à l'exemple 53, au départ de 5-bromo-2,4-diméthylthiazole, on obtient le produit désiré qui est directement engagé dans l'étape de désacétylation.

**Exemple 58:**

**5-(2,4-diméthyl-5-thiazolyl)-3-pyridinyl 5-thio-β-D- xylopyranoside**

En opérant de façon analogue à l'exemple 42, au départ du produit préparé selon l'exemple 57, on obtient le produit désiré sous forme d'un solide blanc (rendement = 32 %).
F = 150°C.

$[\alpha]_D^{30} = -61°$ (c = 0,10 ; DMSO).

**Exemple 59:**

**5-[1-méthyl-3-(trifluorométhyl)-1*H*-pyrazol-4-yl]-3-pyridinyl 2,3,4-tri-O-acétyl-5-thio-β-D- xylopyranoside**

En opérant de façon analogue à l'exemple 53, au départ de 4-bromo-1-méthyl-3-(trifluorométhyl)-1*H*-pyrazole, on obtient le produit désiré qui est directement engagé dans l'étape de désacétylation.

**Exemple 60:**

**5-[1-méthyl-3-(trifluorométhyl)-1*H*-pyrazol-4-yl]-3-pyridinyl 5-thio-β-D- xylopyranoside**

En opérant de façon analogue à l'exemple 2, au départ du produit préparé selon l'exemple 59, on obtient le produit désiré sous forme d'un solide blanc (rendement = 26 %).
F = 210°C.

$[\alpha]_D^{29} = -60°$ (c = 0,24 ; DMSO).

**Exemple 61:**

**5-[5-méthyl-3-(trifluorométhyl)-1*H*-pyrazol-4-yl]-3-pyridinyl 2,3,4-tri-O-acétyl-5-thio-β-D- xylopyranoside**

En opérant de façon analogue à l'exemple 53, au départ de 4-bromo-5-méthyl-3-(trifluorométhyl)-1*H*-pyrazole, on obtient le produit désiré qui est directement engagé dans l'étape de désacétylation.

**Exemple 62:**

**5-[5-méthyl-3-(trifluorométhyl)-1*H*-pyrazol-4-yl)]-3-pyridinyl 5-thio-β-D- xylopyranoside**

En opérant de façon analogue à l'exemple 2, au départ du produit préparé selon l'exemple 61, on obtient le produit désiré sous forme d'un solide beige (rendement = 6 %).
F = 234°C.

$[\alpha]_D^{29} = -76°$ (c = 0,21 ; DMSO).

**Exemple 63:**

**5-(5-méthyl-4-isoxazolyl)-3-pyridinyl 2,3,4-tri-O-acétyl-5-thio-β-D- xylopyranoside**

En opérant de façon analogue à l'exemple 53, au départ de 4-iodo-5-méthylisoxazole, on obtient le produit désiré sous forme d'un solide blanc (rendement = 28 %).
F = 127˚C.

$[\alpha]_D^{28} = -28°$ (c = 0,22 ; DMSO).

**Exemple 64:**

**5-(5-méthyl-4-isoxazolyl)-3-pyridinyl 5-thio-β-D- xylopyranoside**

En opérant de façon analogue à l'exemple 2, au départ du produit préparé selon l'exemple 63, on obtient le produit désiré sous forme d'un solide blanc (rendement = 79 %).
F = 209˚C.

$[\alpha]_D^{28} = -87°$ (c = 0,19 ; DMSO).

**Exemple 65:**

**5-pyrazinyl-3-pyridinyl 2,3,4-tri-O-acétyl-5-thic-β-D-xylopyranoside**

En opérant de façon analogue à l'exemple 53, au départ de iodopyrazine, on obtient le produit désiré sous forme d'une poudre blanche (rendement = 13 %). F = 145˚C.

$[\alpha]_D^{29} = -11°$ (c = 0,22 ; DMSO).

**Exemple 66:**

**5-pyrazinyl-3-pyridinyl 5-thio-β-D-xylopyranoside**

En opérant de façon analogue à l'exemple 2, au départ du produit préparé selon l'exemple 65, on obtient le produit désiré sous forme d'un solide blanc (rendement = 70 %).
F = 197˚C.

$[\alpha]_D^{29} = -68°$ (c = 0,20 ; DMSO).

**Exemple 67:**

**5-(2-pyrimidinyl)-3-pyridinyl 2,3,4-tri-O-acétyl-5-thio-β-D- xylopyranoside**

En opérant de façon analogue à l'exemple 53, au départ de 2-bromopyrimidine, on obtient le produit désiré qui est directement engagé dans l'étape de désacétylation.

**Exemple 68:**

**5-(2-pyrimidinyl)-3-pyridinyl 5-thio-β-D- xylopyranoside**

En opérant de façon analogue à l'exemple 2, au départ du produit préparé selon l'exemple 67, on obtient le produit désiré sous forme d'une poudre blanche (rendement = 31 %).
F = 201 ˚C.

$[\alpha]_D^{29} = -69°$ (c = 0,23 ; DMSO).

**Exemple 69:**

**5-(2-thiazolyl)-3-pyridinyl 2,3,4-tri-O-acétyl-5-thio-β-D- xylopyranoside**

En opérant de façon analogue à l'exemple 53, au départ de 2-bromothiazole, on obtient le produit désiré qui est directement engagé dans l'étape de désacétylation.

**Exemple 70:**

**5-(2-thiazolyl)-3-pyridinyl 5-thio-β-D- xylopyranoside**

En opérant de façon analogue à l'exemple 2, au départ du produit préparé selon l'exemple 69, on obtient le produit désiré sous forme d'une poudre blanche (rendement = 26 %).
F = 204°C.

$[\alpha]_D^{29} = -93°$ (c = 0,20 ; DMSO).

**Exemple 71:**

**5-[1-méthyl-*1H*-imidazol-5-yl]-3-pyridinyl 2,3,4-tri-O-acétyl-5-thio-β-D- xylopyranoside**

En opérant de façon analogue à l'exemple 53, au départ de 5-bromo-1-méthyl-*1H*-imidazole, on obtient le produit désiré qui est directement engagé dans l'étape de désacétylation.

**Exemple 72:**

**5-[1-méthyl-*1H*-imidazol-5-yl]-3-pyridinyl 5-thio-β-D- xylopyranoside**

En opérant de façon analogue à l'exemple 2, au départ du produit préparé selon l'exemple 71, on obtient le produit désiré sous forme d'une poudre beige (rendement = 25 %).
F = 213°C.

$[\alpha]_D^{29} = -83°$ (c = 0,32 ; DMSO).

**Exemple 73:**

**5-(2-chloro-4-pyridinyl)-3-pyridinyl 2,3,4-tri-O-acétyl-5-thio-β-D- xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et d'acide 2-chloro-4-pyridineboronique, on obtient le produit désiré sous forme d'un solide écru (rendement = 55 %).
F = 105-108°C.

$[\alpha]_D^{30} = -21°$ (c = 0,30 ; DMSO).

**Exemple 74:**

**5-(2-chloro-4-pyridinyl)-3-pyridinyl 5-thio-β-D- xylopyranoside**

En opérant de façon analogue à l'exemple 42, au départ du produit préparé selon l'exemple 73, on obtient le produit désiré sous forme d'un solide blanc (rendement = 68 %).
F = 204°C.

$[\alpha]_D^{30} = -76°$ (c = 0,34 ; DMSO).

**Exemple 75:**

**5-(3-chloro-4-pyridinyl)-3-pyridinyl 2,3,4-tri-O-acétyl-5-thio-β-D- xylopyranoside**

En opérant de façon analogue à l'exemple 73, au départ de l'acide 3-chloro-4-pyridineboronique, on obtient le produit désiré sous forme d'un solide blanc (rendement = 39 %).
F = 175°C.

$[\alpha]_D^{25} = -22°$ (c = 0,25 ; DMSO).

**Exemple 76:**

**5-(3-chloro-4-pyridinyl)-3-pyridinyl 5-thio-β-D- xylopyranoside**

En opérant de façon analogue à l'exemple 42, au départ du produit préparé selon l'exemple 75, on obtient le produit désiré sous forme d'un solide blanc (rendement = 88 %).
F = 208°C.

$[\alpha]_D^{25} = -72°$ (c = 0,24 ; DMSO).

**Exemple 77:**

**6-(6-méthoxy-3-pyridinyl)-3-pyridinyl 2,3,4-tri-O-acétyl-5-thio-β-D- xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et d'acide 6-méthoxy-3-pyridineboronique, on obtient le produit désiré sous forme d'un solide blanc (rendement = 65 %).
F = 74°C.

$[\alpha]_D^{29} = +10°$ (c = 0,44 ; DMSO).

**Exemple 78:**

**6-(6-méthoxy-3-pyridinyl)-3-pyridinyl 5-thio-β-D- xylopyranoside**

En opérant de façon analogue à l'exemple 42, au départ du produit préparé selon l'exemple 77, on obtient le produit désiré sous forme d'un solide blanc (rendement = 30 %).
F = 167°C.

$[\alpha]_D^{28} = -27°$ (c = 0,23 ; DMSO).

**Exemple 79:**

**2-(3-furanyl)-4-méthyl-3-pyridinyl 2,3,4-tri-O-acétyl-5-thio-β-D- xylopyranoside**

En opérant de façon analogue à l'exemple 1, au départ de 2-chloro-4-méthyl-3-pyridinyl 2,3,4-tlri-O-acétyl-5-thio-β-D-xylopyranoside obtenu selon la préparation VI et d'acide 3-furaneboronique, on obtient le produit désiré sous forme d'un solide écru (rendement = 77 %).
F = 164°C.

$[\alpha]_D^{33} = +61°$ (c = 0,23 ; DMSO).

**Exemple 80:**

**2-(3-furanyl)-4-méthyl-3-pyridinyl 5-thio-β-D- xylopyranoside**

En opérant de façon analogue à l'exemple 42, au départ du produit préparé selon l'exemple 79, on obtient le produit désiré sous forme d'un coton blanc (rendement = 59 %).

F = 95°C.

$[\alpha]_D^{29} = +83°$ (c = 0,18 ; DMSO).

**Exemple 81:**

**2-(3,5-diméthyl-4-isoxazolyl)-4-méthyl-3-pyridinyl 2,3,4-tri-O-acétyl-5-thio-β-D-xylopyranoside**

En opérant de façon analogue à l'exemple 79, au départ d'acide 3,5-diméthyl-4-isoxazoleboronique, on obtient le produit désiré qui est directement engagé dans l'étape de désacétylation.

**Exemple 82:**

**2-(3,5-diméthyl-4-isoxazolyl)-4-méthyl-3-pyridinyl 5-thio-β-D- xyiopyranoside**

En opérant de façon analogue à l'exemple 42, vau départ du produit préparé selon l'exemple 81, on obtient le produit désiré sous forme d'une poudre blanche (rendement = 20 %).
F = 70-100°C.

$[\alpha]_D^{25} = +115°$ (c = 0,10 ; DMSO).

**Exemple 83:**

**4-(3-pyridinyl)-3-pyridinyl 2,3,4-tri-O-acétyl-5-thio-β-D- xylopyranoside**

En opérant de façon analogue à l'exemple 1, au départ de 4-bromo-3-pyridinyl 2,3,4-tà-O-acétyl-5-thio-β-D-xylopyranoside obtenu selon la préparation II et d'acide 3-pyridineboronique, on obtient le produit désiré sous forme d'un solide écru (rendement = 57 %).
F = 159°C.

$[\alpha]_D^{32} = -72°$ (c = 0,25 ; DMSO).

**Exemple 84:**

**4-(3-pyridinyl)-3-pyridinyl 5-thio-β-D- xylopyranoside**

En opérant de façon analogue à l'exemple 42, au départ du produit préparé selon l'exemple 83, on obtient le produit désiré sous forme d'un solide blanc (rendement = 86 %).
F = 212°C.

$[\alpha]_D^{30} = -53°$ (c = 0,35 ; DMSO).

**Exemple 85:**

**2-(2-benzofuranyl)-3-pyridinyl 2,3,4-tri-O-acétyl-5-thio-β-D- xylopyranoside**

En opérant de façon analogue à l'exemple 1, au départ de 2-bromo-3-pyridinyl 2,3,4-tri-O-acétyl-5-thio-β-D-xylopyranoside obtenu selon la préparation III et d'acide 2-benzofuraneboronique, on obtient le produit désiré qui est directement engagé dans l'étape de désacétylation.

**Exemple 86:**

**2-(2-benzofuranyl)-3-pyridinyl 5-thio-β-D-xylopyranoside**

En opérant de façon analogue à l'exemple 42, au départ du produit préparé selon l'exemple 85, on obtient le produit désiré sous forme d'un solide blanc (rendement = 15 %).
F = 215°C.

$[\alpha]_D^{32} = -68°$ (c = 0,26 ; DMSO).

**Exemple 87:**

**4-(3-furanyl)-3-pyridinyl 2,3,4-tri-O-acétyl-5-thio-β-D- xylopyranoside**

En opérant de façon analogue à l'exemple 83, au départ d'acide 3-furaneboronique, on obtient le produit désiré sous forme d'un solide écru (rendement = 36 %).
F = 167°C.

$[\alpha]_D^{31} = -77°$ (c = 0,37 ; DMSO).

**Exemple 88:**

**4-(3-furanyl)-3-pyridinyl 5-thio-β-D- xylopyranoside**

En opérant de façon analogue à l'exemple 42, au départ du produit préparé selon l'exemple 87, on obtient le produit désiré sous forme d'un solide blanc (rendement = 64 %).
F = 231°C.

$[\alpha]_D^{32} = -112°$ (c = 0,28 ; DMSO).

**Exemple 89:**

**4-(4-pyridinyl)-3-pyridinyl 2,3,4-tri-O-acétyl-5-thio-β-D- xylopyranoside**

En opérant de façon analogue à l'exemple 83, au départ d'acide 4-pyridineboronique, on obtient le produit désiré sous forme d'un solide écru (rendement = 55 %).
F = 185°C.

$[\alpha]_D^{31} = -114°$ (c = 0,47 ; DMSO).

**Exemple 90:**

**4-(4-pyridinyl)-3-pyridinyl 5-thio-β-D- xylopyranoside**

En opérant de façon analogue à l'exemple 42, au départ du produit préparé selon l'exemple 89, on obtient le produit désiré sous forme d'un solide blanc (rendement = 84 %).
F = 212°C.

$[\alpha]_D^{32} = -54°$ (c = 0,26 ; DMSO).

**Exemple 91:**

**5-(2-benzothiényl)-3-pyridinyl 2,3,4-tri-O-acétyl-5-thio-β-D- xylopyranoside**

En opérant de façon analogue à l'exemple 73, au départ d'acide 2-benzothiophèneboronique, on obtient le produit désiré sous forme d'un solide beige (rendement = 72 %).
F = 168°C.

$[\alpha]_D^{30} = +7°$ (c = 0,36 ; DMSO).

**Exemple 92:**

**5-(2-benzothiényl)-3-pyridinyl 5-thio-β-D- xylopyranoside**

En opérant de façon analogue à l'exemple 42, au départ du produit préparé selon l'exemple 91, on obtient le

produit désiré sous forme d'un solide écru (rendement = 91 %).
F = 235°C.

$[\alpha]_D^{32} = -50°$ (c = 0,32 ; DMSO).

**Exemple 93:**

**2-(2-thiényl)-3-pyridinyl 2,3,4-tri-O-acétyl-5-thio-β-D- xylopyranoside**

En opérant de façon analogue à l'exemple 85, au départ d'acide 2-thiophèneboronique, on obtient le produit désiré sous forme d'un solide écru (rendement = 51 %).
F = 166°C.

$[\alpha]_D^{32} = -112°$ (c = 0,20 ; DMSO).

**Exemple 94:**

**2-(2-thiényl)-3-pyridinyl 5-thio-β-D- xylopyranoside**

En opérant de façon analogue à l'exemple 42, au départ du produit préparé selon l'exemple 93, on obtient le produit désiré sous forme d'un solide écru (rendement = 80 %).
F = 130°C.

$[\alpha]_D^{30} = -90°$ (c = 0,46 ; DMSO).

**Exemple 95:**

**5-(5-méthyl-2-furanyl)-3-pyridinyl 2,3,4-tri-O-acétyl-5-thio-β-D- xylopyranoside**

En opérant de façon analogue à l'exemple 73, au départ d'acide 5-méthyl-2-furaneboronique, on obtient le produit désiré sous forme d'un solide beige (rendement = 56 %).
F = 124°C.

$[\alpha]_D^{30} = +1°$ (c = 0,40 ; DMSO).

**Exemple 96:**

**5-(5-méthyl-2-furanyl)-3-pyridinyl 5-thio-β-D- xylopyranoside**

En opérant de façon analogue à l'exemple 42, au départ du produit préparé selon l'exemple 95, on obtient le produit désiré sous forme d'un solide blanc (rendement = 55 %).
F = 188°C.

$[\alpha]_D^{30} = -75°$ (c = 0,40 ; DMSO).

**Exemple 97:**

**6-(2-benzofuranyl)-3-pyridinyl 2,3,4-tri-O-acétyl-5-thio-β-D- xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et d'acide 2-benzofuraneboronique, on obtient le produit désiré sous forme d'un solide écru (rendement = 65 %).
F = 179°C.

$[\alpha]_D^{30} = +13°$ (c = 0,31 ; DMSO).

**Exemple 98:**

**6-(2-benzofuranyl)-3-pyridinyl 5-thio-β-D- xylopyranoside**

En opérant de façon analogue à l'exemple 42, au départ du produit préparé selon l'exemple 97, on obtient le produit désiré sous forme d'un solide blanc (rendement = 50 %).
F = 195°C.

$[\alpha]_D^{30} = -24°$ (c = 0,28 ; DMSO).

**Exemple 99:**

**6-(3-thiényl)-3-pyridinyl 2,3,4-tri-O-acétyl-5-thio-β-D- xylopyranoside**

En opérant de façon analogue à l'exemple 97, au départ d'acide 3-thiophèneboronique, on obtient le produit désiré sous forme d'une mousse blanche (rendement = 66 %).
F = 158°C.

$[\alpha]_D^{35} = -9°$ (c = 0,30 ; DMSO).

**Exemple 100:**

**6-(3-thiényl)-3-pyridinyl 5-thio-β-D- xylopyranoside**

En opérant de façon analogue à l'exemple 42, au départ du produit préparé selon l'exemple 99, on obtient le produit désiré sous forme d'un solide blanc cassé (rendement = 98 %).
F = 154°C.

$[\alpha]_D^{30} = -50°$ (c = 0,28 ; DMSO).

**Exemple 101:**

**2-(2-furanyl)-3-pyridinyl 2,3,4-tri-O-acétyl-5-thio-β-D- xylopyranoside**

En opérant de façon analogue à l'exemple 85, au départ d'acide 2-furaneboronique, on obtient le produit désiré sous forme d'un solide écru (rendement = 46 %).
F = 160°C.

$[\alpha]_D^{28} = -60°$ (c = 0,51 ; DMSO).

**Exemple 102:**

**2-(2-furanyl)-3-pyridinyl 5-thio-β-D- xylopyranoside**

En opérant de façon analogue à l'exemple 42, au départ du produit préparé selon l'exemple 101, on obtient le produit désiré sous forme d'un solide écru (rendement = 91 %).
F = 184°C.

$[\alpha]_D^{34} = -108°$ (c = 0,30 ; DMSO).

**Exemple 103:**

**6-(2-furanyl)-3-pyridinyl 2,3,4-tri-O-acétyl-5-thio-β-D- xylopyranoside**

En opérant de façon analogue à l'exemple 97, au départ d'acide 2-furaneboronique, on obtient le produit désiré sous forme d'un solide blanc cassé (rendement = 64 %).
F = 133°C.

$[\alpha]_D^{30} = +16°$ (c = 0,30 ; DMSO).

**Exemple 104:**

**6-(2-furanyl)-3-pyridinyl 5-thio-β-D- xylopyranoside**

En opérant de façon analogue à l'exemple 42, au départ du produit préparé selon l'exemple 103, on obtient le produit désiré sous forme d'un solide rose pale (rendement = 92 %).
F = 146°C.

$[\alpha]_D^{30} = -53°$ (c = 0,30 ; DMSO).

**Exemple 105:**

**2-(4-pyridinyl)-3-pyridinyl 2,3,4-tri-O-acétyl-5-thio-β-D- xylopyranoside**

En opérant de façon analogue à l'exemple 85, au départ d'acide 4-pyridineboronique, on obtient le produit désiré sous forme d'un solide écru (rendement = 28 %).
F = 161°C.

$[\alpha]_D^{28} = -82°$ (c = 0,30 ; DMSO).

**Exemple 106:**

**2-(4-pyridinyl)-3-pyridinyl 5-thio-β-D- xylopyranoside**

En opérant de façon analogue à l'exemple 42, au départ du produit préparé selon l'exemple 105, on obtient le produit désiré sous forme d'un solide beige (rendement = 97 %).
F = 129°C.

$[\alpha]_D^{30} = -56°$ (c = 0,40 ; DMSO).

**Exemple 107:**

**6-(2-thiényl)-3-pyridinyl 2,3,4-tri-O-acétyl-5-thio-β-D- xylopyranoside**

En opérant de façon analogue à l'exemple 97, au départ d'acide 2-thiophèneboronique, on obtient le produit désiré sous forme d'un solide écru (rendement = 68 %).
F = 172°C.

$[\alpha]_D^{33} = -6°$ (c = 0,40 ; DMSO).

**Exemple 108:**

**6-(2-thiényl)-3-pyridinyl 5-thio-β-D- xylopyranoside**

En opérant de façon analogue à l'exemple 42, au départ du produit préparé selon l'exemple 107, on obtient le produit désiré sous forme d'un solide beige (rendement = 80 %).
F = 134°C.

$[\alpha]_D^{30} = -40°$ (c = 0,38 ; DMSO).

**Exemple 109:**

**5-(2-furanyl)-3-pyridinyl 2,3,4-tri-O-acétyl-5-thio-β-D- xylopyranoside**

En opérant de façon analogue à l'exemple 73, au départ d'acide 2-furaneboronique, on obtient le produit désiré sous forme d'une mousse beige (rendement = 61 %).
F = 184°C.

$[\alpha]_D^{25} = -10°$ (c = 0,28 ; DMSO).

**Exemple 110:**

**5-(2-furanyl)-3-pyridinyl 5-thio-β-D- xylopyranoside**

En opérant de façon analogue à l'exemple 42, au départ du produit préparé selon l'exemple 109, on obtient le produit désiré sous forme d'un solide beige (rendement = 66 %).
F = 215°C.

$[\alpha]_D^{30} = -68°$ (c = 0,25 ; DMSO).

**Exemple 111:**

**2-(3-pyridinyl)-3-pyridinyl 2,3,4-tri-O-acétyl-5-thio-β-D- xylopyranoside**

En opérant de façon analogue à l'exemple 85, au départ d'acide 3-pyridineboronique, on obtient le produit désiré sous forme d'un solide ocre (rendement = 28 %).
F = 70°C.

$[\alpha]_D^{24} = -70°$ (c = 0,35 ; DMSO).

**Exemple 112:**

**2-(3-pyridinyl)-3-pyridinyl 5-thio-β-D- xylopyranoside**

En opérant de façon analogue à l'exemple 42, au départ du produit préparé selon l'exemple 111, on obtient le produit désiré sous forme d'un solide ocre (rendement = 89 %).
F = 80°C.

$[\alpha]_D^{30} = -40°$ (c = 0,44 ; DMSO).

**Exemple 113:**

**2-(3-furanyl)-3-pyridinyl 2,3,4-tri-O-acétyl-5-thio-β-D- xylopyranoside**

En opérant de façon analogue à l'exemple 85, au départ d'acide 3-furaneboronique, on obtient le produit désiré sous forme d'un solide blanc (rendement = 20 %).
F = 185°C.

$[\alpha]_D^{25} = -99°$ (c = 0,28 ; DMSO).

**Exemple 114:**

**2-(3-furanyl)-3-pyridinyl 5-thio-β-D- xylopyranoside**

En opérant de façon analogue à l'exemple 42, au départ du produit préparé selon l'exemple 113, on obtient le produit désiré sous forme d'un solide blanc (rendement = 91 %).
F = 128°C.

$[\alpha]_D^{24} = -74°$ (c = 0,30 ; DMSO).

**Exemple 115:**

**5-(2-benzofuranyl)-3-pyridinyl 2,3,4-tri-O-acétyl-5-thio-β-D- xylopyranoside**

En opérant de façon analogue à l'exemple 73, au départ d'acide 2-benzofuraneboronique, on obtient le produit désiré sous forme d'un solide écru (rendement = 70 %).

F = 125°C.

$[\alpha]_D^{29} = +8°$ (c = 0,40 ; DMSO).

**Exemple 116:**

**5-(2-benzofuranyl)-3-pyridinyl 5-thio-β-D- xylopyranoside**

En opérant de façon analogue à l'exemple 42, au départ du produit préparé selon l'exemple 115, on obtient le produit désiré sous forme d'un solide blanc (rendement = 86 %).
F = 210°C.

$[\alpha]_D^{24} = -44°$ (c = 0,30 ; DMSO).

**Exemple 117:**

**6-(3-furanyl)-3-pyridinyl 2,3,4-tri-O-acétyl-5-thio-β-D- xylopyranoside**

En opérant de façon analogue à l'exemple 97, au départ d'acide 3-furaneboronique, on obtient le produit désiré sous forme d'un solide écru (rendement = 66 %).
F = 187°C.

$[\alpha]_D^{29} = +3°$ (c = 0,25 ; DMSO).

**Exemple 118:**

**6-(3-furanyl)-3-pyridinyl 5-thio-β-D- xylopyranoside**

En opérant de façon analogue à l'exemple 42, au départ du produit préparé selon l'exemple 117, on obtient le produit désiré sous forme d'un solide écru (rendement = 85 %).
F = 132°C.

$[\alpha]_D^{29} = -29°$ (c = 0,27 ; DMSO).

**Exemple 119:**

**6-(3-pyridinyl)-3-pyridinyl 2,3,4-tri-O-acétyl-5-thio-β-D- xylopyranoside**

En opérant de façon analogue à l'exemple 97, au départ d'acide 3-pyridineboronique, on obtient le produit désiré sous forme d'un solide écru (rendement = 50 %).
F = 196°C.

$[\alpha]_D^{26} = +4°$ (c = 0,27 ; DMSO).

**Exemple 120:**

**6-(3-pyridinyl)-3-pyridinyl 5-thio-β-D- xylopyranoside**

En opérant de façon analogue à l'exemple 42, au départ du produit préparé selon l'exemple 119, on obtient le produit désiré sous forme d'un solide rose pâle (rendement = 99 %).
F = 152°C.

$[\alpha]_D^{28} = -40°$ (c = 0,30 ; DMSO).

**Exemple 121:**

**6-(4-pyridinyl)-3-pyridinyl 2,3,4-tri-O-acétyl-5-thio-β-D- xylopyranoside**

En opérant de façon analogue à l'exemple 97, au départ d'acide 4-pyridineboronique, on obtient le produit

désiré sous forme d'un solide blanc cassé (rendement = 31 %).
F = 189°C.

$[\alpha]_D^{29} = +4°$ (c = 0,40 ; DMSO).

**Exemple 122:**

**6-(4-pyridinyl)-3-pyridinyl 5-thio-β-D- xylopyranoside**

En opérant de façon analogue à l'exemple 42, au départ du produit préparé selon l'exemple 121, on obtint le produit désiré sous forme d'un solide écru (rendement = 82 %).
F = 248°C.

$[\alpha]_D^{26} = -35°$ (c = 0,34 ; DMSO).

**Exemple 123:**

**5-(4-pyridinyl)-3-pyridinyl 2,3,4-tri-O-acétyl-5-thio-β-D- xylopyranoside**

En opérant de façon analogue à l'exemple 73, au départ d'acide 4-pyridineboronique, on obtient le produit désiré sous forme d'un solide blanc cassé (rendement = 40 %).
F = 138°C.

$[\alpha]_D^{27} = -14°$ (c = 0,35 ; DMSO).

**Exemple 124:**

**5-(4-pyridinyl)-3-pyridinyl 5-thio-β-D- xylopyranoside**

On additionne 0,41 g (0,92 mM) de produit obtenu selon l'exemple 123 à un mélange de 6 ml de THF et 6 ml d'eau. Le milieu est refroidi à l'aide d'un bain de glace, on additionne 0,385 g (9,18 mM) d'hydroxyde de lithium (monohydrate) et on agite pendant 90 minutes. Le milieu réactionnel est concentré partiellement sous pression réduite et la phase aqueuse résultante est amenée à pH 5-6 à l'aide d'une solution d'acide chlorhydrique 1N. Le produit précipité est filtré et séché. On obtient ainsi le produit désiré sous la forme d'un solide blanc avec un rendement de 88%.
F = 213°C.

$[\alpha]_D^{27} = -48°$ (c = 0,40 ; DMSO).

**Exemple 125:**

**5-(3-furanyl)-3-pyridinyl 2,3,4-tri-O-acétyl-5-thio-β-D- xylopyranoside**

En opérant de façon analogue à l'exemple 73, au départ d'acide 3-furaneboronique, on obtient le produit désiré sous forme d'un solide beige (rendement = 32 %).
F = 127°C.

$[\alpha]_D^{27} = -20°$ (c = 0,29 ; DMSO).

**Exemple 126:**

**5-(3-furanyl)-3-pyridinyl 5-thio-β-D- xylopyranoside**

En opérant de façon analogue à l'exemple 42, au départ du produit préparé selon l'exemple 125, on obtient le produit désiré sous forme d'un solide écru (rendement = 93 %).
F = 170°C.

$[\alpha]_D^{27} = -72°$ (c = 0,31 ; DMSO).

**Exemple 127:**

**5-(3-pyridinyl)-3-pyridinyl 2,3,4-tri-O-acétyl-5-thio-β-D- xylopyranoside**

En opérant de façon analogue à l'exemple 73, au départ d'acide 3-pyridineboronique, on obtient le produit désiré sous forme d'un solide écru (rendement = 63 %).
F = 149°C.

$[\alpha]_D^{23} = -23°$ (c = 0,25 ; DMSO).

**Exemple 128:**

**5-(3-pyridinyl)-3-pyridinyl 5-thio-β-D- xylopyranoside**

En opérant de façon analogue à l'exemple 42, au départ du produit préparé selon l'exemple 127, on obtient le produit désiré sous forme d'un solide écru (rendement = 50 %).
F = 203°C.

$[\alpha]_D^{23} = -80°$ (c = 0,43 ; DMSO).

**Exemple 129:**

**2-(4-pyridinyl)-4-pyridinyl 2,3,4-tri-O-acétyl-5-thio-β-D- xylopyranoside**

En opérant de façon analogue à l'exemple 1, au départ de 2-bromo-4-pyridinyl 2,3,4-tri-O-acétyl-5-thio-β-D-xylopyranoside obtenu selon la préparation VII et d'acide 4-pyridineboronique, on obtient le produit désiré sous forme d'un solide blanc (rendement = 53 %).
F = 185°C.

$[\alpha]_D^{29} = -27°$ (c = 0,13 ; DMSO).

**Exemple 130:**

**2-(4-pyridinyl)-4-pyridinyl 5-thio-β-D- xylopyranoside**

En opérant de façon analogue à l'exemple 2, au départ du produit préparé selon l'exemple 129, on obtient le produit désiré sous forme d'un solide blanc (rendement = 42 %).
F = 170°C.

$[\alpha]_D^{29} = -49°$ (c = 0,10 ; DMSO).

**Exemple 131:**

**2-(6-fluoro-3-pyridinyl)-4-pyridinyl 2,3,4-tri-O-acétyl-5-thio-β-D- xylopyranoside**

En opérant de façon analogue à l'exemple 129, au départ d'acide 6-fluoro-3-pyridineboronique, on obtient le produit désiré sous forme d'un solide blanc (rendement = 51 %).
F = 151°C.

$[\alpha]_D^{29} = -34°$ (c = 0,15 ; DMSO).

**Exemple 132:**

**2-(6-fluoro-3-pyridinyl)-4-pyridinyl 5-thio-β-D- xylopyranoside**

En opérant de façon analogue à l'exemple 2, au départ du produit préparé selon l'exemple 131, on obtient le produit désiré sous forme d'un solide blanc (rendement = 94 %).
F = 171°C.

$[\alpha]_D^{29} = -66°$ (c = 0,26 ; DMSO).

**Exemple 133:**

**2-(3-pyridinyl)-4-pyridinyl 2,3,4-tri-O-acétyl-5-thio-β-D- xylopyranoside**

En opérant de façon analogue à l'exemple 129, au départ d'acide 3-pyridineboronique, on obtient le produit désiré sous forme de cristaux beiges (rendement = 22 %).
F = 185°C (recristallisé dans l'isopropanol).

$[\alpha]_D^{31} = -21°$ (c = 0,24 ; DMSO).

**Exemple 134:**

**2-(3-pyridinyl)-4-pyridinyl 5-thio-β-D- xylopyranoside**

En opérant de façon analogue à l'exemple 2, an départ du produit préparé selon l'exemple 133, on obtient le produit désiré sous forme de cristaux blancs (rendement = 66 %).
F = 197°C (recristallisé dans l'eau).

$[\alpha]_D^{29} = -83°$ (c = 0,10 ; DMSO).

**Exemple 135:**

**5-(2-thiényl)-3-pyridinyl 2,3,4-tri-O-acétyl-5-thio-β-D- xylopyranoside**

En opérant de façon analogue à l'exemple 73, au départ d'acide 2-thiophèneneboronique, on obtient le produit désiré sous forme d'un solide écru (rendement = 57 %).
F = 166°C.

$[\alpha]_D^{27} = +6°$ (c = 0,25 ; DMSO).

**Exemple 136:**

**5-(2-thiényl)-3-pyridinyl 5-thio-β-D- xylopyranoside**

En opérant de façon analogue à l'exemple 42, au départ du produit préparé selon l'exemple 135, on obtient le produit désiré sous forme d'un solide écru (rendement = 78 %).
F = 205°C.

$[\alpha]_D^{30} = -68°$ (c = 0,40 ; DMSO).

**Exemple 137:**

**5-(5-méthoxy-3-pyridinyl)-3-pyridinyl 2,3,4-tri-O-acétyl-5-thio-β-D- xylopyranoside**

Sous atmosphère d'argon, on chauffe 30 minutes à 110°C à l'aide de micro-ondes un mélange constitué de 1 g (5,32 mM) de 3-bromo-5-méthoxypyridine, 10 ml de DME, 2,02 g (7,98 mM) de bis(pinacolato)diborane, 0,13 g (0,16 mM) de [1,1'-bis(diphénylphosphino)ferrocène]dichloropalladium(II)-dichlorométhane, et 1,56 g (15,96 mM) d'acétate de potassium. Le mélange est refroidi, filtré et on ajoute au filtrat 1,59 g (3,55 mM) de 5-bromo-3-pyridinyl 2,3,4-tri-O-acétyl-5-thio-β-D-xylopyranoside obtenu selon la préparation IX, 0,29 g (0,355 mM) de [1,1'-bis(diphénylphosphino) ferrocène]dichloropalladium(II)-dichlorométhane et 0,564 g (5,32 mM) de carbonate de sodium dissout dans 5 ml d'eau. On chauffe le mélange réactionnel 20 minutes à 120°C à l'aide de micro-ondes. Le milieu est ensuite refroidi, additionné d'eau, et extrait à l'acétate d'éthyle. La phase organique est lavée par une solution aqueuse de bicarbonate de sodium, par de l'eau, puis séchée sur sulfate de sodium et concentrée sous pression réduite. Le résidu d'évaporation est purifié par chromatographie sur colonne de silice (éluant: toluène/isopropanol 90/10 ; v/v), le produit obtenu est trituré en présence d'éther et filtré. On obtient le produit attendu sous forme d'un solide blanc avec un rendement de 28 %.

F = 181°C.

$[\alpha]_D^{28} = -7°$ (c = 0,26 ; DMSO).

**Exemple 138:**

**5-(5-méthoxy-3-pyridinyl)-3-pyridinyl 5-thio-β-D- xylopyranoside**

En opérant de façon analogue à l'exemple 42, au départ du produit préparé selon l'exemple 137, on obtient le produit désiré sous forme d'un solide blanc (rendement = 33 %).
F = 193°C.

$[\alpha]_D^{27} = -71°$ (c = 0,40 ; DMSO).

**Exemple 139:**

**6-(5-méthyl-4-isoxazolyl)-3-pyridinyl 2,3,4-tri-O-acétyl-5-thio-β-D- xylopyranoside**

Sous atmosphère d'argon, on prépare une solution de 0,208 g (1 mM) de 4-iodo-5-méthyl-isoxazole dans 5 ml de THF et on ajoute 0,019 g (0,1 mM) d'iodure cuivreux et 0,060 g (1,5 mM) d'hydrure de sodium à 60% dans l'huile. Le mélange réactionnel est agité à température ambiante pendant 5 minutes et on ajoute 0,192 g (1,5 mM) de bis (pinacolato)diborane. Le mélange réactionnel est ensuite agité pendant 1 heure à température ambiante puis additionné de 4 ml d'une solution saturée de bicarbonate de sodium et extrait à l'acétate d'éthyle. La phase organique est lavée à l'eau, séchée sur sulfate de sodium et concentrée sous pression réduite. On obtient l'ester pinacolique de l'acide 5-méthyl-4-isoxazoleboronique [$^1$H RMN (250 MHz ; $DMSOd_6$) $\delta$ = 8,44 (s;1H), 2,55 (s;3H), 12,27 (s;12H)] sous forme d'un solide blanc avec un rendement de 81%. Ce composé est remis en réaction avec le 6-bromo-3-pyridinyl 2.3,4-tri-O-acétyl-5-thio-βD-xylopyranoside obtenu selon la préparation IV, de façon analogue au procédé mis en oeuvre pour l'exemple 1 et on obtient ainsi le composé attendu sous forme d'un solide blanc (rendement = 43 %)
F = 165°C.

$[\alpha]_D^{30} = -4°$ (c = 0,31 ; DMSO).

**Exemple 140:**

**6-(5-méthyl-4-isoxazolyl)-3-pyridinyl 5-thio-β-D- xylopyranoside**

En opérant de façon analogue à l'exemple 2, au départ du produit préparé selon l'exemple 139, on obtient le produit désiré sous forme d'un solide blanc (rendement = 39 %).
F = 184°C.

$[\alpha]_D^{30} = -69°$ (c = 0,25 ; DMSO).

**Exemple 141:**

**6-(5-méthyl-3-pyridinyl)-3-pyridinyl 2,3,4-tri-O-acétyl-5-thio-β-D- xylopyranoside**

En opérant de façon analogue à l'exemple 137, au départ de 3-bromo-5-méthylpyridine et de 6-bromo-3-pyridinyl 2,3,4-tri-O-acétyl-5-thio-β-D-xylopyranoside obtenu selon la préparation IV, on obtient le produit désiré sous forme d'un solide blanc (rendement = 38 %).
F = 189-190°C.

$[\alpha]_D^{25} = +9°$ (c = 0,20 ; DMSO).

**Exemple 142:**

**6-(5-méthyl-3-pyridinyl)-3-pyridinyl 5-thio-β-D- xylopyranoside**

En opérant de façon analogue à l'exemple 42, au départ du produit préparé selon l'exemple 141, on obtient

le produit désiré sous forme d'un solide blanc (rendement = 49 %).
F = 165-166°C.

$[\alpha]_D^{30} = -44°$ (c = 0,25 ; DMSO).

**Exemple 143:**

**5-(5-méthyl-3-pyridinyl)-3-pyridinyl 2,3,4-tri-O-acétyl-5-thio-β-D- xylopyranoside**

En opérant de façon analogue à l'exemple 137, au départ de 3-bromo-5-méthylpyridine, on obtient le produit désiré sous forme d'un solide beige (rendement = 45 %).
F = 180-182°C.

$[\alpha]_D^{25} = -17°$ (c = 0,24; DMSO).

**Exemple 144:**

**5-(5-méthyl-3-pyridinyl)-3-pyridinyl 5-thio-β-D- xylopyranoside**

En opérant de façon analogue à l'exemple 42, au départ du produit préparé selon l'exemple 143, on obtient le produit désiré sous forme d'un solide blanc (rendement = 83 %).
F = 119-120°C.

$[\alpha]_D^{25} = -73°$ (c = 0,27 ; DMSO).

**Exemple 145:**

**5-(5-chloro-3-pyridinyl)-3-pyridinyl 2,3,4-tri-O-acétyl-5-thio-β-D- xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et de 3-chloro-5-(pinacolborane)pyridine, on obtient le produit désiré sous forme d'un solide blanc (rendement = 31 %).
F = 199°C.

$[\alpha]_D^{29} = -23°$ (c = 0,29 ; DMSO).

**Exemple 146:**

**5-(5-chloro-3-pyridinyl)-3-pyridinyl 5-thio-β-D- xylopyranoside**

En opérant de façon analogue à l'exemple 42, au départ du produit préparé selon l'exemple 145, on obtient le produit désiré sous forme d'un solide blanc (rendement = 73 %).
F = 185-187°C.

$[\alpha]_D^{29} = -23°$ (c = 0,27 ; DMSO).

**Exemple 147:**

**5-(6-cyano-3-pyridinyl)-3-pyridinyl 2,3,4-tri-O-acétyl-5-thio-β-D- xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et de 6-cyano-3-(pinacolborane)pyridine, on obtient le produit désiré sous forme d'un solide mauve (rendement = 22 %).
F = 142-151°C.

$[\alpha]_D^{29} = -14°$ (c = 0,26 ; DMSO).

**Exemple 148:**

**5-(6-cyano-3-pyridinyl)-3-pyridinyl 5-thio-β-D- xylopyranoside**

En opérant de façon analogue à l'exemple 2, au départ du produit préparé selon l'exemple 147, on obtient le produit désiré sous forme d'un solide blanc (rendement = 30 %).
F = 197-203°C.

$[\alpha]_D^{29} = -68°$ (c = 0,34 ; DMSO).

**Exemple 149:**

**6-(5-fluoro-3-pyridinyl)-3-pyridinyl 2,3,4-tri-O-acétyl-5-thio-β-D- xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et de 3-fluoro-5-(pinacolborane)pyridine, on obtient le produit désiré sous forme d'un solide blanc (rendement = 22 %).
F = 197-198°C.

$[\alpha]_D^{29} = +1°$ (c = 0,22 ; DMSO).

**Exemple 150:**

**6-(5-fluoro-3-pyridinyl)-3-pyridinyl 5-thio-β-D- xylopyranoside**

En opérant de façon analogue à l'exemple 2, au départ du produit préparé selon l'exemple 149, on obtient le produit désiré sous forme d'un solide blanc (rendement = 66 %).
F = 207-217°C.

$[\alpha]_D^{29} = -56°$ (c = 0,21 ; DMSO).

**Exemple 151:**

**5-(2-méthyl-3-pyridinyl)-3-pyridinyl 2,3,4-tri-O-acétyl-5-thio-β-xylopyranoside**

En opérant de façon analogue à l'exemple 137, au départ de 5-bromo-3-pyridinyl 2,3,4-tri-O-acétyl-5-thio-β-D-xylopyranoside obtenu selon la préparation IX et de 2-méthyl-3-(pinacolborane)pyridine (obtenu au départ de trifluorométhane-sulfonate de 2-méthyl-3-pyridinyle), on obtient le produit désiré sous forme d'un solide beige (rendement = 30 %).
F = 152-153°C.

$[\alpha]_D^{29} = -21°$ (c = 0,24 ; DMSO).

**Exemple 152:**

**5-(2-méthyl-3-pyridinyl)-3-pyridinyl 5-thio-β-D- xylopyranoside**

En opérant de façon analogue à l'exemple 42, au départ du produit préparé selon l'exemple 151, on obtient le produit désiré sous forme d'un solide blanc (rendement = 85 %).
F = 204-207°C.

$[\alpha]_D^{29} = -81°$ (c = 0,21 ; DMSO).

**Exemple 153:**

**5-(5-fluoro-3-pyridinyl)-3-pyridinyl 2,3,4-tri-O-acétyl-5-thio-β-D- xylopyranoside**

En opérant de façon analogue à l'exemple 1, au départ de 5-bromo-3-pyridinyl 2,3,4-tri-O-acétyl-5-thio-β-D-xylopyranoside et de 3-fluoro-5-(pinacolborane)-pyridine, on obtient le produit désiré sous forme d'un solide blanc (rendement = 26 %).
F = 175-176°C.

$[\alpha]_D^{29} = -21°$ (c = 0,30 ; DMSO).

**Exemple 154:**

**5-(5-fluoro-3-pyridinyl)-3-pyridinyl 5-thio-β-D- xylopyranoside**

En opérant de façon analogue à l'exemple 42, au départ du produit préparé selon l'exemple 153, on obtient le produit désiré sous forme d'un solide blanc (rendement = 77 %).
F = 160-171°C.

$[\alpha]_D^{29} = -72°$ (c = 0,25 ; DMSO).

**Exemple 155:**

**6-(5-méthoxy-3-pyridinyl)-3-pyridinyl 2,3,4-tri-O-acétyl-5-thio-β-D-xylopyranoside**

En opérant de façon analogue à l'exemple 137, au départ de 3-bromo-5-méthoxypyridine et de 6-bromo-3-pyridinyl 2,3,4-tri-O-acétyl-5-thio-β-D-xylopyranoside obtenu selon la préparation IV, on obtient le produit désiré sous forme d'un solide blanc (rendement = 48 %).
F = 155°C.

$[\alpha]_D^{29} = +1°$ (c = 0,47 ; DMSO).

**Exemple 156:**

**6-(5-méthoxy-3-pyridinyl)-3-pyridinyl 5-thio-β-D- xylopyranoside**

En opérant de façon analogue à l'exemple 2, au départ du produit préparé selon l'exemple 155, on obtient le produit désiré sous forme d'un solide blanc (rendement = 93 %).
F = 193°C.

$[\alpha]_D^{28} = -31°$ (c = 0,42 ; DMSO).

**Exemple 157:**

**5-(1,3,5-triméthyl-1*H*-pyrazol-4-yl)-3-pyridinyl 2,3,4-tri-O-acétyl-5-thio-β-D- xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et de 1,3,5-triméthyl-4-(pinacolborane)pyrazole on obtient le produit désiré sous forme d'un solide blanc (rendement = 60 %).
F = 166°C.

$[\alpha]_D^{29} = -26°$ (c = 0,19 ; DMSO).

**Exemple 158:**

**5-(1,3,5-triméthyl-1*H*-pyrazol-4-yl)-3-pyridinyl 5-thio-β-D- xylopyranoside**

En opérant de façon analogue à l'exemple 2, au départ du produit préparé selon l'exemple 157, on obtient le produit désiré sous forme d'une poudre blanche (rendement = 48 %).
F = 174°C.

$[\alpha]_D^{29} = -82°$ (c = 0,27 ; DMSO).

**Exemple 159:**

**5-(3,5-diméthyl-1*H*-pyrazol-4-yl)-3-pyridinyl 2,3,4-tri-O-acétyl-5-thio-β-D- xylopyranoside**

En opérant de façon analogue à l'exemple 1 au départ de 5-bromo-3-pyridinyl 2,3,4-tri-O-acétyl-5-thio-β-D-xylopyranoside obtenu selon la préparation IX et 3,5-diméthyl-4-(pinacolborane)pyrazole, on obtient le produit désiré sous forme d'un solide beige (rendement = 45 %).
F = 95°C (cristallisé dans l'éther).
$[\alpha]_D^{29} = -22°$ (c = 0,24 ; DMSO).

**Exemple 160:**

**5-(3,5-diméthyl-1*H*-pyrazol-4-yl)-3-pyridinyl 5-thio-β-D- xylopyranoside**

En opérant de façon analogue à l'exemple 2, au départ du produit préparé selon l'exemple 159, on obtient le produit désiré sous forme d'une poudre blanche (rendement = 41 %).
F = 227°C.
$[\alpha]_D^{28} = -86°$ (c = 0,28 ; DMSO).

**Exemple 161:**

**6-(6-cyano-3-pyridinyl)-3-pyridinyl 2,3,4-tri-O-acétyl-5-thio-β-D-xylopyranoside**

En opérant de façon analogue à l'exemple 1 au départ de 6-bromo-3-pyridinyl 2,3,4-tri-O-acétyl-5-thio-β-D-xylopyranoside obtenu selon la préparation IV et de 6-cyano-3-(pinacolborane)pyridine, on obtient le produit désiré sous forme d'un solide blanc (rendement = 41 %).
F = 247°C.
$[\alpha]_D^{29} = +15°$ (c = 0,20 ; DMSO).

**Exemple 162:**

**6-(6-cyano-3-pyridinyl)-3-pyridinyl 5-thio-β-D- xylopyranoside**

En opérant de façon analogue à l'exemple 2, au départ du produit préparé selon l'exemple 161, on obtient le produit désiré sous forme d'un solide blanc (rendement = 49 %).
F = 167°C.
$[\alpha]_D^{27} = -43°$ (c = 0,30 ; DMSO).

**Exemple 163:**

**6-(1,3,5-triméthyl-1*H*-pyrazol-4-yl)-3-pyridinyl 2,3,4-tri-O-acétyl-5-thio-β-D- xylopyranoside**

En opérant de façon analogue à l'exemple 1 au départ de 6-bromo-3-pyridinyl 2,3,4-tri-O-acétyl-5-thio-β-D-xylopyranoside obtenu selon la préparation IV et de 1,3,5-triméthyl-4-(pinacolborane)-1*H*-pyrazole on obtient le produit désiré sous forme d'un solide blanc (rendement = 54 %).
F = 156°C.
$[\alpha]_D^{28} = 0°$ (c = 0,24 ; DMSO).

**Exemple 164:**

**6-(1,3,5-triméthyl-1*H*-pyrazol-4-yl)-3-pyridinyl 5-thio-β-D- xylopyranoside**

En opérant de façon analogue à l'exemple 2, au départ du produit préparé selon l'exemple 163, on obtient le

produit désiré sous forme d'une poudre blanche (rendement = 57 %).
F = 132°C.

$[\alpha]_D^{28} = -41°$ (c = 0,29 ; DMSO).

**Exemple 165:**

**6-(1-méthyl-1*H*-pyrazol-4-yl)-3-pyridinyl 2,3,4-tri-O-acétyl-5-thio-β-D- xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et de 1-méthyl-4-(pinacolborane)-1*H*-pyrazole, on obtient le produit désiré sous forme d'un solide blanc (rendement = 46 %).
F = 183°C.

$[\alpha]_D^{28} = +7°$ (c = 0,18 ; DMSO).

**Exemple 166:**

**6-(1-méthyl-1*H*-pyrazol-4-yl)-3-pyridinyl 5-thio-β-D- xylopyranoside**

En opérant de façon analogue à l'exemple 2, au départ du produit préparé selon l'exemple 165, on obtient le produit désiré sous forme d'un solide blanc (rendement = 65 %).

$[\alpha]_D^{28} = -50°$ (c = 0,20 ; DMSO).

**Exemple 167:**

**6-(3,5-diméthyl-1*H*-pyrazol-4-yl)-3-pyridinyl 2,3,4-tri-O-acétyl-5-thio-β-D- xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et 3,5-diméthyl-4-(pinacolborane)-1*H*-pyrazole, on obtient le produit désiré sous forme d'un solide blanc (rendement = 21 %).
F= 154°C.

$[\alpha]_D^{28} = -1°$ (c = 0,26 ; DMSO).

**Exemple 168:**

**6-(3,5-diméthyl-1*H*-pyrazol-4-yl)-3-pridinyl 5-thio-β-D- xylopyranoside**

En opérant de façon analogue à l'exemple 2, au départ du produit préparé selon l'exemple 167, on obtient le produit désiré sous forme d'un solide blanc (rendement = 72 %).
F = 210°C.

$[\alpha]_D^{28} = -47°$ (c = 0,24 ; DMSO).

**Exemple 169:**

**5-(6-fluoro-3-pyridinyl)-3-pyridinyl 2,3,4-tri-O-acétyl-5-thio-β-D- xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et d'acide 6-fluoro-3-pyridineboronique, on obtient le produit désiré sous forme d'un solide crème (rendement = 56 %).
F = 167-169°C.

$[\alpha]_D^{27} = -18°$ (c = 0,23 ; DMSO).

**Exemple 170:**

**5-(6-fluoro-3-pyridinyl)-3-pyridinyl 5-thio-β-D- xylopyranoside**

En opérant de façon analogue à l'exemple 124, au départ du produit préparé selon l'exemple 169, on obtient le produit désiré sous forme d'un solide blanc (rendement = 47 %).
F = 210-212°C.

$[\alpha]_D^{24} = -47°$ (c = 0,14 ; DMSO).

**Exemple 171:**

**5-(1-méthyl-1*H*-pyrazol-4-yl)-3-pyridinyl 2,3,4-tri-O-acétyl-5-thio-β-D- xylopyranoside**

En opérant de façon analogue à l'exemple 1, au départ de 5-bromo-3-pyridinyl 2,3,4-tri-O-acétyl-5-thio-β-D-xylopyranoside obtenu selon la préparation IX et de 1-méthyl-4-(pinacolborane)-1*H*-pyrazole, on obtient le produit désiré sous forme d'un solide beige (rendement = 58 %).
F = 68°C.

$[\alpha]_D^{29} = -19°$ (c = 0,20 ; DMSO).

**Exemple 172:**

**5-(1-méthyl-1*H*-pyrazol-4-yl)-3-pyridinyl 5-thio-β-D- xylopyranoside**

En opérant de façon analogue à l'exemple 2, au départ du produit préparé selon l'exemple 171, on obtient le produit désiré sous forme d'un solide blanc (rendement = 72 %).
F=216°C.

$[\alpha]_D^{29} = -74°$ (c = 0,25 ; DMSO).

**Exemple 173:**

**6-(2,4-diméthyl-5-thiazolyl)-3-pyridinyl 2,3,4-tri-O-acétyl-5-thio-β-D- xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et de 2,4-diméthyl-5-(pinacolborane)thiazole, on obtient le produit désiré sous forme de cristaux blancs (rendement = 21 %).
F = 174-177°C (cristallisé dans l'éther éthylique).

$[\alpha]_D^{32} = +9°$ (c = 0,43 ; DMSO).

**Exemple 174:**

**6-(2,4-diméthyl-5-thiazolyl)-3-pyridinyl 5-thio-β-D- xylopyranoside**

En opérant de façon analogue à l'exemple 42, au départ du produit préparé selon l'exemple 173, on obtient le produit désiré sous forme de cristaux crème (rendement = 92 %).
F = 175°C.

$[\alpha]_D^{32} = -53°$ (c = 0,41 ; DMSO).

**Exemple 175:**

**6-(2-méthyl-3-pyridinyl)-3-pyridinyl 2,3,4-tri-O-acétyl-5-thio-β-D- xylopyranoside**

En opérant de façon analogue à l'exemple 137, au départ de 6-bromo-3-pyridinyl 2,3,4-tri-O-acétyl-5-thio-β-D-xylopyranoside obtenu selon la préparation IV et de 2-méthyl-3-(pinacolborane)pyridine (obtenu au départ de trifluorométhanesulfonate de 2-méthyl-3-pyridinyle), on obtient le produit désiré sous forme d'un solide blanc (rendement = 11 %).
F = 198-200°C.

$[\alpha]_D^{29} = -3°$ (c = 0,21 ; DMSO).

**Exemple 176:**

**6-(2-méthyl-3-pyridinyl)-3-pyridinyl 5-thio-β-D- xylopyranoside**

En opérant de façon analogue à l'exemple 2, au départ du produit préparé selon l'exemple 175, on obtient le produit désiré sous forme d'un solide blanc (rendement = 39 %).
F = 167-168°C.

$[\alpha]_D^{32} = -26°$ (c = 0,32 ; DMSO).

**Exemple 177:**

**5-(5-pyrimidinyl)-3-pyridinyl 2,3,4-tri-O-acétyl-5-thio-β-D- xylopyranoside**

On mélange 0,2 g (0,45 mM) de 5-bromo-3-pyridinyl 2,3,4-tri-O-acétyl-5-thio-β-D-xylopyranoside obtenu selon la préparation IX, 66 mg (0,53 mM) d'acide 5-pyrimidineboronique, 0,281 g (0,90 mM) de résine greffée benzyl-triéthylammonium carbonate et 36 mg (0,044 mM) de [1,1'-bis(diphénylphosphino)-ferrocène]dichloropalladium(II)-dichlorométhane dans 3 ml de DME et 2 ml de méthanol. Le mélange réactionnel est porté à 120°C pendant 30 minutes par chauffage sous micro-ondes. Après filtration et rinçage du résidu solide au méthanol, la solution résultante est concentrée sous pression réduite. Le résidu d'évaporation est purifié par chromatographie sur colonne de silice (éluant: dichlorométhane/méthanol 70/30 ; v/v), le produit est ensuite recristallisé dans l'isopropanol pour obtenir le produit attendu sous forme de cristaux roses nacrés avec un rendement de 50 %.
F = 213-217°C.

$[\alpha]_D^{30} = -4°$ (c = 0,10 ; DMSO).

**Exemple 178:**

**5-(5-pyrimidinyl)-3-pyridinyl 5-thio-β-D- xylopyranoside**

En opérant de façon analogue à l'exemple 42, au départ du produit préparé selon l'exemple 177, on obtient le produit désiré sous forme d'une poudre rose (rendement = 67 %).
F = 196°C.

$[\alpha]_D^{30} = -96°$ (c = 0,17 ; DMSO).

**Exemple 179:**

**5-(3,5-diméthyl-4-isoxazolyl)-3-pyridinyl 2,3,4-tri-O-acétyl-5-thio-β-D- xylopyranoside**

En opérant de façon analogue à l'exemple 177, au départ d'acide 3,5-diméthyl-4-isoxazoleboronique, on obtient le produit désiré sous forme de cristaux blancs (rendement = 33 %).
F = 129-131°C.

$[\alpha]_D^{30} = -36°$ (c = 0,10 ; DMSO).

**Exemple 180:**

**5-(3,5-diméthyl-4-isoxazolyl)-3-pyridinyl 5-thio-β-D- xylopyranoside**

En opérant de façon analogue à l'exemple 42, au départ du produit préparé selon l'exemple 179, on obtient le produit désiré sous forme de cristaux blancs (rendement = 70 %).

F = 222-223°C.

$[\alpha]_D^{24} = -51°$ (c = 0,10 ; DMSO).

**Exemple 181:**

**2-(3,5-diméthyl-4-isoxazolyl)-3-pyridinyl 2,3,4-tri-O-acétyl-5-thio-β-D- xylopyranoside**

En opérant de façon analogue à l'exemple 179, au départ de 2-bromo-3-pyridinyl 5-thio-β-D-xylopyranoside obtenu selon la préparation III, on obtient le produit désiré sous forme d'une mousse rose (rendement = 49 %).
F = 68-72°C.

$[\alpha]_D^{27} = -92°$ (c = 0,24 ; DMSO).

**Exemple 182:**

**2-(3,5-diméthyl-4-isoxazolyl)-3-pyridinyl 5-thio-β-D- xylopyranoside**

En opérant de façon analogue à l'exemple 42, au départ du produit préparé selon l'exemple 181, on obtient le produit désiré sous forme d'une poudre blanc cassé (rendement = 65 %).
F = 112°C.

$[\alpha]_D^{27} = -53°$ (c = 0,24 ; DMSO).

**Exemple 183:**

**5-(2-fluoro-3-pyridinyl)-3-pyridinyl 2,3,4-tri-O-acétyl-5-thio-β-D- xylopyranoside**

En opérant de façon analogue à l'exemple 177, au départ d'acide 2-fluoro-3-pyridineboronique, on obtient le produit désiré sous forme de cristaux beiges (rendement = 52 %).
F = 169-170°C (recristallisé dans l'isopropanol).

$[\alpha]_D^{24} = -37°$ (c = 0,17 ; DMSO).

**Exemple 184:**

**5-(2-fluoro-3-pyridinyl)-3-pyridinyl 5-thio-β-D- xylopyranoside**

En opérant de façon analogue à l'exemple 2, au départ du produit préparé selon l'exemple 183, on obtient le produit désiré sous forme d'un solide blanc (rendement = 46 %).
F = 193-196°C.

$[\alpha]_D^{31} = -81°$ (c = 0,11 ; DMSO).

**Exemple 185:**

**5-(2-fluoro-4-pyridinyl)-3-pyridinyl 2,3,4-tri-O-acétyl-5-thio-β-D- xylopyranoside**

En opérant de façon analogue à l'exemple 177, au départ d'acide 2-fluoro-4-pyridineboronique, on obtient le produit désiré sous forme d'un solide beige (rendement = 70 %).
F = 122-125°C.

$[\alpha]_D^{31} = -29°$ (c = 0,11 ; DMSO).

**Exemple 186:**

**5-(2-fluoro-4-pyridinyl)-3-pyridinyl 5-thio-β-D- xylopyranoside**

En opérant de façon analogue à l'exemple 2, au départ du produit préparé selon l'exemple 185, on obtient le

produit désiré sous forme d'un solide blanc (rendement = 76 %).
F = 208°C (recristallisé dans le méthanol).

$[\alpha]_D^{28} = -92°$ (c = 0,32 ; DMSO).

**Exemple 187:**

**6-(2-fluoro-3-pyridinyl)-3-pyridinyl 2,3,4-tri-O-acétyl-5-thio-β-D- xylopyranoside**

En opérant de façon analogue à l'exemple 183, au départ de 6-bromo-3-pyridinyl 5-thio-β-O-xylopyranoside obtenu selon la préparation IV, on obtient le produit désiré sous forme d'une poudre crème (rendement = 38 %).
F = 153°C.

$[\alpha]_D^{26} = -7°$ (c = 0,19 ; DMSO).

**Exemple 188:**

**6-(2-fluoro-3-pyridinyl)-3-pyridinyl 5-thio-β-D- xylopyranoside**

En opérant de façon analogue à l'exemple 2, au départ du produit préparé selon l'exemple 187, on obtient le produit désiré sous forme d'une poudre blanche (rendement = 88 %).
F = 181 °C (recristallisé dans l'eau).

$[\alpha]_D^{27} = -48°$ (c = 0,17 ; DMSO).

**Exemple 189:**

**6-(5-pyrimidinyl)-3-pyridinyl 2,3,4-tri-O-acétyl-5-thio-β-D- xylopyranoside**

En opérant de façon analogue à l'exemple 187, au départ d'acide 5-pyrimidineboronique, on obtient le produit désiré sous forme d'un solide rose (rendement = 39 %).
F = 215°C (cristallisé dans l'éther éthylique).

$[\alpha]_D^{28} = -2°$ (c = 0,18 ; DMSO).

**Exemple 190:**

**6-(5-pyrimidinyl)-3-pyridinyl 5-thio-β-D- xylopyranoside**

En opérant de façon analogue à l'exemple 42, au départ du produit préparé selon l'exemple 189, on obtient le produit désiré sous forme d'une poudre rosée (rendement = 77 %).
F = 188°C.

$[\alpha]_D^{27} = -45°$ (c = 0,19 ; DMSO).

**Exemple 191:**

**6-(3,5-diméthyl-4-isoxazolyl)-3-pyridinyl 2,3,4-tri-O-acétyl-5-thio-β-D- xylopyranoside**

En opérant de façon analogue à l'exemple 187, au départ d'acide 3,5-diméthyl-4-isoxazoleboronique, on obtient le produit désiré sous forme d'un solide blanc (rendement = 21 %).
F = 172°C (recristallisé dans l'éther éthylique).

$[\alpha]_D^{28} = -13°$ (c = 0,14; DMSO)

**Exemple 192:**

**6-(3,5-diméthyl-4-isoxazolyl)-3-pyridinyl 5-thio-β-D- xylopyranoside**

En opérant de façon analogue à l'exemple 42, au départ du produit préparé selon l'exemple 191, on obtient

le produit désiré sous forme d'un solide floconneux blanc (rendement = 52 %).
F = 138°C.

$[\alpha]_D^{27} = -43°$ (c = 0,10 ; DMSO).

**Exemple 193:**

**6-(6-fluoro-3-pyridinyl)-3-pyridinyl 2,3,4-tri-O-acétyl-5-thio-β-D- xylopyranoside**

En opérant de façon analogue à l'exemple 187, au départ d'acide 6-fluoro-3-pyridineboronique, on obtient le produit désiré sous forme de paillettes blanches (rendement = 30 %).
F = 185°C (recristallisé dans un mélange éthanol/eau).

$[\alpha]_D^{28} = +3°$ (c = 0,43 ; DMSO).

**Exemple 194:**

**6-(6-fluoro-3-pyridinyl)-3-pyridinyl 5-thio-β-D- xylopyranoside**

En opérant de façon analogue à l'exemple 2, au départ du produit préparé selon l'exemple 193, on obtient le produit désiré sous forme d'une poudre blanche (rendement = 98 %).
F = 143-156°C.

$[\alpha]_D^{28} = -56°$ (c = 0,26 ; DMSO).

**Exemple 195:**

**2-fluoro-5-(2-fluoro-4-pyridinyl)-3-pyridinyl 2,3,4-tri-O-acétyl-5-thio-β-D- xylopyranoside**

En opérant de façon analogue à l'exemple 177, au départ au départ de 5-bromo-2-fluoro-3-pyridinyl 5-thio-β-D-xylopyranoside obtenu selon la préparation VIII et d'acide 2-fluoro-4-pyridineboronique, on obtient le produit désiré sous forme d'un solide rose (rendement = 65 %).
F = 142-144°C.

$[\alpha]_D^{30} = -9°$ (c = 0,25 ; DMSO).

**Exemple 196:**

**2-fluoro-5-(2-fluoro-4-pyridinyl)-3-pyridinyl 5-thio-β-D- xylopyranoside**

En opérant de façon analogue à l'exemple 2, au départ du produit préparé selon l'exemple 195, on obtient le produit désiré sous forme d'un solide gris (rendement = 65 %).
F = 145-146°C.

$[\alpha]_D^{30} = +8°$ (c = 0,24 ; DMSO).

**Exemple 197:**

**2-fluoro-5-(6-fluoro-3-pyridinyl)-3-pyridinyl 2,3,4-tri-O-acétyl-5-thio-β-D- xylopyranoside**

En opérant de façon analogue à l'exemple 195, au départ d'acide 6-fluoro-3-pyridineboronique, on obtient le produit désiré sous forme d'un solide rose (rendement = 35 %).
F = 135-136°C.

$[\alpha]_D^{30} = -22°$ (c = 0,28 ; DMSO).

**Exemple 198:**

**2-fluoro-5-(6-fluoro-3-pyridinyl)-3-pyridinyl 5-thio-β-D- xylopyranoside**

En opérant de façon analogue à l'exemple 2, au départ du produit préparé selon l'exemple 197, on obtient le produit désiré sous forme d'un solide gris (rendement = 71 %).
F = 158-159°C.

$[\alpha]_D^{30} = -72°$ (c = 0,30 ; DMSO).

**Exemple 199:**

**5-(3-thiényl)-3-pyridinyl 2,3,4-tri-O-acétyl-5-thio-β-D- xylopyranoside**

En opérant de façon analogue à l'exemple 177, au départ d'acide 3-thiénylboronique, on obtient le produit désiré sous forme d'un solide beige (rendement = 25 %).
F = 175°C.

$[\alpha]_D^{27} = +6°$ (c = 0,25 ; DMSO).

**Exemple 200:**

**5-(3-thiényl)-3-pyridinyl 5-thio-β-D- xylopyranoside**

En opérant de façon analogue à l'exemple 42, au départ du produit préparé selon l'exemple 199, on obtient le produit désiré sous forme d'un solide blanc (rendement = 32 %).
F = 212°C.

$[\alpha]_D^{27} = -113°$ (c = 0,10 ; DMSO).

**Exemple 201:**

**6-(2-chloro-4-pyridinyl)-3-pyridinyl 2,3,4-tri-O-acétyl-5-thio-β-D- xylopyranoside**

En opérant de façon analogue à l'exemple 187, au départ d'acide 2-chloro-4-pyridineboronique, on obtient le produit désiré sous forme d'une poudre rose (rendement = 21 %).
F = 226°C.

$[\alpha]_D^{29} = +12°$ (c = 0,20 ; DMSO).

**Exemple 202:**

**6-(2-chloro-4-pyridinyl) -3-pyridinyl 5-thio-β-D- xylopyranoside**

En opérant de façon analogue à l'exemple 2, au départ du produit préparé selon l'exemple 201, on obtient le produit désiré sous forme d'une poudre jaune (rendement = 62 %).
F = 179°C (recristallisé dans l'eau).

$[\alpha]_D^{29} = -36°$ (c = 0,13 ; DMSO).

**Exemple 203:**

**4-(3,5-diméthyl-4-isoxazolyl)-3-pyridinyl 2,3,4-tri-O-acétyl-5-thio-β-D- xylopyranoside**

En opérant de façon analogue à l'exemple 177, au départ de 4-bromo-3-pyridinyl 5-thio-β-D-xylopyranoside obtenu selon la préparation II et d'acide 3,5-diméthyl-4-isoxazoleboronique, on obtient le produit désiré sous forme d'une poudre rose (rendement = 30 %).
F = 150-155°C.

$[\alpha]_D^{25} = -38°$ (c = 0,10 ; DMSO).

**Exemple 204:**

**4-(3,5-diméthyl-4-isoxazolyl)-3-pyridinyl 5-thio-β-D- xylopyranoside**

En opérant de façon analogue à l'exemple 2, au départ du produit préparé selon l'exemple 203, on obtient le produit désiré sous forme d'une poudre beige (rendement = 62 %).
F = 175-179°C.

$[\alpha]_D^{31} = -159°$ (c = 0,10 ; DMSO).

**Exemple 205:**

**6-méthyl-2-(3-pyridinyl)-3-pyridinyl 2,3,4-tri-O-acétyl-5-thio-β-D- xylopyranoside**

En opérant de façon analogue à l'exemple 177, au départ de 2-iodo-6-méthyl-3-pyridinyl 5-thio-β-D-xylopyranoside obtenu selon la préparation V et d'acide 3-pyridineboronique, on obtient le produit désiré sous forme d'une poudre ocre (rendement = 42 %).
F = 92-102°C.

$[\alpha]_D^{26} = -82°$ (c = 0,10 ; DMSO).

**Exemple 206:**

**6-méthyl-2-(3-pyridinyl)-3-pyridinyl 5-thio-β-D- xylopyranoside**

En opérant de façon analogue à l'exemple 42, au départ du produit préparé selon l'exemple 205, on obtient le produit désiré sous forme d'une poudre jaune (rendement = 72 %).
F = 160-170°C.

$[\alpha]_D^{25} = -19°$ (c = 0,18; DMSO).

**Exemple 207:**

**6-méthyl-2-(4-pyridinyl)-3-pyridinyl 2,3,4-tri-O-acétyl-5-thio-β-D- xylopyranoside**

En opérant de façon analogue à l'exemple 205, au départ d'acide 4-pyridinylboronique, on obtient le produit désiré sous forme d'une poudre rose pâle (rendement = 54 %).
F = 140-144°C.

$[\alpha]_D^{34} = -54°$ (c = 0,11 ; DMSO).

**Exemple 208:**

**6-méthyl-2-(4-pyridinyl)-3-pyridinyl 5-thio-β-D- xylopyranoside**

En opérant de façon analogue à l'exemple 42, au départ du produit préparé selon l'exemple 207, on obtient le produit désiré sous forme d'une poudre beige (rendement = 95 %).
F = 196-200°C.

$[\alpha]_D^{25} = -35°$ (c = 0,10 ; DMSO).

**Exemple 209:**

**6-(2-fluoro-4-pyridinyl)-3-pyridinyl 2,3,4-tri-O-acétyl-5-thio-β-D- xylopyranoside**

En opérant de façon analogue à l'exemple 187, au départ d'acide 2-fluoro-4-pyridineboronique, on obtient le

produit désiré sous forme d'un solide blanc (rendement = 30 %).
F = 179°C.

$[\alpha]_D^{30} = +1°$ (c = 0,82 ; DMSO).

**Exemple 210:**

**6-(2-fluoro-4-pyridinyl)-3-pyridinyl 5-thio-β-D- xylopyranoside**

En opérant de façon analogue à l'exemple 124, au départ du produit préparé selon l'exemple 209, on obtient le produit désiré sous forme d'un solide blanc (rendement = 56 %).
F = 219°C.

$[\alpha]_D^{32} = -49°$ (c = 0,28 ; DMSO).

**Exemple 211:**

**2-(5-pyrimidinyl)-3-pyridinyl 2,3,4-tri-O-acétyl-5-thio-β-D- xylopyranoside**

En opérant de façon analogue à l'exemple 181, au départ d'acide 5-pyrimidineboronique, on obtient le produit désiré sous forme d'un solide crème (rendement = 48 %).
F = 205°C (recristallisé dans l'éther éthylique).

$[\alpha]_D^{30} = -76°$ (c = 0,61 ; DMSO).

**Exemple 212:**

**2-(5-pyrimidinyl)-3-pyridinyl 5-thio-β-D- xylopyranoside**

En opérant de façon analogue à l'exemple 42, au départ du produit préparé selon l'exemple 211, on obtient le produit désiré sous forme d'une poudre rose (rendement = 36 %).
F = 195°C (recristallisé dans un mélange méthanol/éther éthylique).

$[\alpha]_D^{30} = -38°$ (c = 0,27; DMSO).

**Exemple 213:**

**5-fluoro-6-(6-fluoro-3-pyridinyl)-3-pyridinyl 2,3,4-tri-O-acétyl-5-thio-β-D- xylopyranoside**

On prépare une solution de 0,421 g (1 mM) de 6-chloro-5-fluoro-3-pyridinyl 2,3,4-tri-O-acétyl-5-thio-β-D-xylopyranoside obtenu selon la préparation XI, dans 4 ml de DME. On ajoute 0,211 g (1.5 mM) d'acide 6-fluoro-3-pyridineboronique, 0,082 g (0.1 mM) de [1,1'-bis(diphénylphosphino)ferrocène]dichloropalladium(II)-dichlorométhane puis 1,5 ml (1,5 mM) d'une solution 1 M de carbonate de potassium Le milieu réactionnel est chauffé 30 min à 120°C à l'aide de micro-ondes. Après refroidissement et décantation, la phase organique est prélevée et l'extraction est complétée par l'addition d'acétate d'éthyle. Les phases organiques réunies sont évaporées sous pression réduite et le produit d'évaporation est dissous dans 4 ml de dichlorométhane et lavé successivement par de l'eau, une solution de carbonate de potassium 1 M, puis à l'eau. La phase organique est filtrée sur une membrane hydrophobe et évaporée sous un courant d'azote. On obtient le produit désiré qui est directement utilisé dans l'étape de désacétylation.

**Exemple 214:**

**5-fluoro-6-(6-fluoro-3-pyridinyl)-3-pyridinyl 5-thio-β-D- xylopyranoside**

En opérant de façon analogue à l'exemple 2, au départ du produit préparé selon l'exemple 213, on obtient le produit désiré sous forme d'un solide blanc, avec un rendement de 75%.
F = 184°C.

$[\alpha]_D^{33} = -54°$ (c = 0,10 ; DMSO).

**Exemple 215:**

**5-fluoro-6-(6-méthyl-3-pyridinyl)-3-pyridinyl 2,3,4-tri-O-acétyl-5-thio-β-D- xylopyranoside**

En opérant de façon analogue à l'exemple 213, au départ d'acide 6-méthyl-3-pyridineboronique, on obtient le produit désiré qui est directement utilisé dans l'étape de désacétylation.

**Exemple 216:**

**5-fluoro-6-(6-méthyl-3-pyridinyl)-3-pyridinyl 5-thio-β-D- xylopyranoside**

En opérant de façon analogue à l'exemple 2, au départ du produit préparé selon l'exemple 215, on obtient le produit désiré sous forme d'un solide blanc (rendement = 58 %).
F = 184°C.

$[\alpha]_D^{33} = -54°$ (c = 0,24 ; DMSO).

**Exemple 217:**

**5-(2-méthoxy-5-pyrimidinyl)-3-pyridinyl 2,3,4-tri-O-acétyl-5-thio-β-D- xylopyranoside**

En opérant de façon analogue à l'exemple 213, au départ de 5-bromo-3-pyridinyl 2,3,4-tri-O-acétyl-5-thio-β-D-xylopyranoside obtenu selon la préparation IX et d'acide 2-méthoxy-5-pyrimidineboronique, on obtient le produit désiré qui est directement utilisé dans l'étape de désacétylation.

**Exemple 218:**

**5-(2-méthoxy-5-pyrimidinyl)-3-pyridinyl 5-thio-β-D- xylopyranoside**

En opérant de façon analogue à l'exemple 2, au départ du produit préparé selon l'exemple 217, on obtient le produit désiré sous forme d'un solide blanc (rendement = 46 %).
F = 215°C.

$[\alpha]_D^{30} = -75°$ (c = 0,10 ; DMSO).

**Exemple 219:**

**5-fluoro-6-(2-méthyl-4-pyridinyl)-3-pyridinyl 2,3,4-tri-O-acétyl-5-thio-β-D- xylopyranoside**

En opérant de façon analogue à l'exemple 213, au départ d'acide 2-méthyl-4-pyridineboronique, on obtient le produit désiré qui est directement utilisé dans l'étape de désacétylation.

**Exemple 220:**

**5-fluoro-6-(2-méthyl-4-pyridinyl)-3-pyridinyl 5-thio-β-D- xylopyranoside**

En opérant de façon analogue à l'exemple 2, au départ du produit préparé selon l'exemple 219, on obtient le produit désiré sous forme d'un solide blanc (rendement = 31 %).
F = 143°C.

$[\alpha]_D^{30} = -27°$ (c = 0,10 ; DMSO).

**Exemple 221:**

**5-fluoro-6-(2-méthoxy-3-pyridinyl)-3-pyridinyl 2,3,4-tri-O-acétyl-5-thio-β-D- xylopyranoside**

En opérant de façon analogue à l'exemple 213, au départ d'acide 2-méthoxy-3-pyridineboronique, on obtient le produit désiré qui est directement utilisé dans l'étape de désacétylation.

**Exemple 222:**

**5-fluoro-6-(2-méthoxy-3-pyridinyl)-3-pyridinyl 5-thio-β-D- xylopyranoside**

En opérant de façon analogue à l'exemple 2, au départ du produit préparé selon l'exemple 221, on obtient le produit désiré sous forme d'un solide blanc (rendement = 77 %).
F = 141˚C.

$[\alpha]_D^{30} = -35°$ (c = 0,10 ; DMSO).

**Exemple 223:**

**5-fluoro-6-(1-méthyl-*1H*-pyrazol- 4-yl)- 3-pyridinyl 2,3,4-tri- O- acétyl- 5-thio- β-D-xylopyranoside**

En opérant de façon analogue à l'exemple 213, au départ de 1-méthyl-4-(pinacolborane)-*1H*-pyrazole, on obtient le produit désiré qui est directement utilisé dans l'étape de désacétylation.

**Exemple 224:**

**5-fluoro-6-(1-méthyl-*1H*-pyrazol-4-yl)-3-pyridinyl 5-thio-β-D- xylopyranoside**

En opérant de façon analogue à l'exemple 2, au départ du produit préparé selon l'exemple 223, on obtient le produit désiré sous forme d'un solide blanc (rendement = 41 %).
F = 206˚C.

$[\alpha]_D^{30} = -46°$ (c = 0,10 ; DMSO).

**Exemple 225:**

**6-(3,5-diméthyl-4-isoxazolyl)-5-fluoro-3-pyridinyl 2,3,4-tri-O-acétyl-5-thio-β-D-xylopyranoside**

En opérant de façon analogue à l'exemple 213, au départ d'acide 3,5-diméthyl-4-isoxazoleboronique, on obtient le produit désiré qui est directement utilisé dans l'étape de désacétylation.

**Exemple 226:**

**6-(3,5-diméthyl-4-isoxazolyl)-5-fluoro-3-pyridinyl 5-thio-β-D- xylopyranoside**

En opérant de façon analogue à l'exemple 2, au départ du produit préparé selon l'exemple 225, on obtient le produit désiré sous forme d'un solide blanc (rendement = 33 %).
F = 177˚C.

$[\alpha]_D^{30} = -48°$ (c = 0,12 ; DMSO).

**Exemple 227:**

**6-(2-méthoxy-5-pyrimidinyl)-3-pyridinyl 2,3,4-tri-O-acétyl-5-thio-β-D- xylopyranoside**

En opérant de façon analogue à l'exemple 213, au départ de 6-bromo-3-pyridinyl 2,3,4-tri-O-acétyl-5-thio-β-D-xylopyranoside obtenu selon la préparation IV et d'acide 2-méthoxy-5-pyrimidineboronique, on obtient le produit désiré qui est directement utilisé dans l'étape de désacétylation.

**Exemple 228:**

**6-(2-méthoxy-5-pyrimidinyl)-3-pyridinyl 5-thio-β-D- xylopyranoside**

En opérant de façon analogue à l'exemple 2, au départ du produit préparé selon l'exemple 227, on obtient le produit désiré sous forme d'un solide blanc (rendement = 20 %).

F = 134°C.

$[\alpha]_D^{26} = -53°$ (c = 0,10 ; DMSO).

**Exemple 229:**

**5-fluoro-6-(5-pyrimidinyl)-3-pyridinyl 2,3,4-tri-O-acétyl-5-thio-β-D- xylopyranoside**

En opérant de façon analogue à l'exemple 213, au départ d'acide 5-pyrimidineboronique, on obtient le produit désiré qui est directement utilisé dans l'étape de désacétylation.

**Exemple 230:**

**5-fluoro-6-(5-pyrimidinyl)-3-pyridinyl 5-thio-β-D- xylopyranoside**

En opérant de façon analogue à l'exemple 2, au départ du produit préparé selon l'exemple 229, on obtient le produit désiré sous forme d'un solide blanc (rendement = 19 %).
F = 192°C.

$[\alpha]_D^{33} = -34°$ (c = 0,12 ; DMSO).

**Exemple 231:**

**5-(3-chloro-2-pyridinyl)-3-pyridinyl 2,3,4-tri-O-acétyl-5-thio-β-D- xylopyranoside**

A partir d'une solution de 0,448 g (1 mM) de 5-bromo-3-pyridinyl 2,3,4-tri-O-acétyl-5-thio-β-D-xylopyranoside obtenu selon la préparation IX dans 2 ml de DME, on prépare le boronate selon la méthode décrite à l'exemple 49. Le milieu réactionnel est ensuite filtré puis rincé avec du DME. On ajoute à la solution résultante 0,422 g (1.66 mM) de 2,3-dichloro-pyridine, 0,020 g (0,025 mM) de [1,1'-bis(diphénylphosphino)ferrocène]dichloropalladium(II)-dichlorométhane puis 1,5 ml (1,5 mM) d'une solution 1M de carbonate de potassium. Le milieu réactionnel est chauffé 30 min à 120°C à l'aide de micro-ondes. Après refroidissement et décantation, la phase organique est prélevée et l'extraction est complétée par l'addition d'acétate d'éthyle. Les phases organiques réunies sont évaporées sous pression réduite et le produit d'évaporation est dissout dans 4 ml de dichlorométhane et lavé successivement par de l'eau, une solution de carbonate de potassium 1M, puis à l'eau. La phase organique est filtrée sur une membrane hydrophobe et évaporée sous un courant d'azote. On obtient le produit désiré qui est directement utilisé dans l'étape de désacétylation.

**Exemple 232**

**5-(3-chloro-2-pyridinyl)-3-pyridinyl 5-thio-β-D- xylopyranoside**

En opérant de façon analogue à l'exemple 2, au départ du produit préparé selon l'exemple 231 on obtient le produit désiré sous forme d'un solide blanc (rendement = 50%).
F = 193°C

$[\alpha]_D^{30} = -46°$ (c = 0,18 ; DMSO).

**Exemple 233:**

**5-(5-méthyl-2-pyridinyl)-3-pyridinyl 2,3,4-tri-O-acétyl-5-thio-β-D- xylopyranoside**

En opérant de façon analogue à l'exemple 231, au départ de 2-bromo-5-méthylpyridine, on obtient le produit désiré qui est directement utilisé dans l'étape de désacétylation.

**Exemple 234:**

**5-(5-méthyl-2-pyridinyl)-3-pyridinyl 5-thio-β-D- xylopyranoside**

En opérant de façon analogue à l'exemple 2, au départ du produit préparé selon l'exemple 233, on obtient le

produit désiré sous forme d'un solide blanc (rendement = 33 %).
F = 199°C.

$[\alpha]_D^{29} = -64°$ (c = 0,18 ; DMSO).

**Exemple 235:**

**5-(4-méthyl-2-pyridinyl)-3-pyridinyl 2,3,4-tri-O-acétyl-5-thio-β-D- xylopyranoside**

En opérant de façon analogue à l'exemple 231, au départ de 2-bromo-4-méthylpyridine, on obtient le produit désiré qui est directement utilisé dans l'étape de désacétylation.

**Exemple 236:**

**5-(4-méthyl-2-pyridinyl)-3-pyridinyl 5-thio-β-D- xylopyranoside**

En opérant de façon analogue à l'exemple 2, au départ du produit préparé selon l'exemple 235, on obtient le produit désiré sous forme d'une poudre blanche (rendement = 20 %).
F = 166°C.

$[\alpha]_D^{29} = -80°$ (c = 0,10 ; DMSO).

**Exemple 237:**

**5-(6-méthyl-2-pyridinyl)-3-pyridinyl 2,3,4-tri-O-acétyl-5-thio-β-D- xylopyranoside**

En opérant de façon analogue à l'exemple 231, au départ de 2-bromo-6-méthylpyridine, on obtient le produit désiré qui est directement utilisé dans l'étape de désacétylation.

**Exemple 238:**

**5-(6-méthyl-2-pyridinyl)-3-pyridinyl 5-thio-β-D- xylopyranoside**

En opérant de façon analogue à l'exemple 2, au départ du produit préparé selon l'exemple 237, on obtient le produit désiré sous forme d'une poudre blanche (rendement = 49 %).
F = 188°C.

$[\alpha]_D^{29} = -68°$ (c = 0,30 ; DMSO).

**Exemple 239:**

**6-méthyl-2-(3,5-diméthyl-4-isoxazolyl)-3-pyridinyl 5-thio-β-D- xylopyranoside**

On mélange 0,3 g (0,59 mM) de 2-iodo-6-méthyl-3-pyridinyl 2,3,4-tri-O-acétyl-5-thio-β-D-xylopyranoside obtenu selon la préparation V, 0,1 g (0,71 mM) d'acide 3,5-diméthyl-4-isoxazoleboronique, 0,384 g (1,17 mM) de carbonate de césium et 0,327 g de tétrakis(triphénylphosphine)palladium greffé sur résine polystyrène dans ml de DME et 3,5 ml de méthanol. On chauffe à 120°C pendant 30 minutes à l'aide de micro-ondes. Le mélange réactionnel est ensuite filtré, le solide résiduel est rincé au méthanol et le filtrat est concentré sous pression réduite. Le résidu d'évaporation est purifié par chromatographie sur colonne de silice en éluant à l'aide d'un mélange dichlorométhane/méthanol (80/20 ; v/v) pour donner le produit attendu sous forme d'une poudre blanche avec un rendement de 70%.
F = 70-84°C.

$[\alpha]_D^{31} = -64°$ (c = 0,11 ; DMSO).

**Exemple 240:**

**2-(6-fluoro-3-pyridinyl)-3-pyridinyl 5-thio-β-D- xylopyranoside**

En opérant de façon analogue à l'exemple 239, au départ de 2-bromo-3-pyridinyl 2,3,4-tri-O-acétyl-5-thio-β-D-xylopyranoside obtenu selon la préparation III et d'acide 6-tluoro-3-pyridineboronique, on obtient le produit désiré sous forme d'une poudre blanche (rendement = 14 %).
F = 199°C.

$[\alpha]_D^{32} = -54°$ (c = 0,26 ; DMSO).

**Exemple 241:**

**2-(3,5-diméthyl-4-isoxazolyl)-4-pyridinyl 5-thio-β-D- xylopyranoside**

En opérant de façon analogue à l'exemple 239, au départ de 2-bromo-4-pyridinyl 2,3,4-tri-O-acétyl-5-thio-β-D-xylopyranoside obtenu selon la préparation VII et d'acide 3,5-diméthyl-4-isoxazoleboronique, on obtient le produit désiré sous forme de cristaux blancs (rendement = 25 %).
F = 143-147°C (recristallisé dans l'eau).

$[\alpha]_D^{27} = -77°$ (c = 0,22 ; DMSO).

**Exemple 242:**

**5-(4-méthoxy-3-pyridinyl)-3-pyridinyl 5-thio-β-D- xylopyranoside**

En opérant de façon analogue à l'exemple 239, au départ de 5-bromo-3-pyridinyl 2,3,4-tri-O-acétyl-5-thio-β-D-xylopyranoside obtenu selon la préparation IX et d'acide 4-méthoxy-3-pyridineboronique, on obtient le produit désiré sous forme d'un solide blanc (rendement = 23 %).
F = 154°C.

$[\alpha]_D^{29} = -49°$ (c = 0,30 ; DMSO).

**Exemple 243:**

**6-(4-méthoxy-3-pyridinyl)-3-pyridinyl 5-thio-β-D- xylopyranoside**

En opérant de façon analogue à l'exemple 239, au départ de 6-bromo-3-pyridinyl 2,3,4-tri-O-acétyl-5-thio-β-D-xylopyranoside obtenu selon la préparation IV et d'acide 4-méthoxy-3-pyridineboronique, on obtient le produit désiré sous forme d'un solide blanc (rendement = 37 %).
F = 207°C.

$[\alpha]_D^{27} = -11°$ (c = 0,25 ; DMSO).

**Exemple 244:**

**5-(2-méthyl-4-pyridinyl)-3-pyridinyl 5-thio-β-D-xylopyranoside**

En opérant de façon analogue à l'exemple 239, au départ de 5-bromo-3-pyridinyl 2,3,4-tri-O-acétyl-5-thio-β-D-xylopyranoside obtenu selon la préparation IX et d'acide 2-méthyl-4-pyridineboronique, on obtient le produit désiré sous forme d'un solide blanc (rendement = 28 %).
F = 120°C (recristallisé dans l'eau).

$[\alpha]_D^{29} = -77°$ (c = 0,17 ; DMSO).

**Exemple 245:**

**6-(2-méthyl-4-pyridinyl)-3-pyridinyl 5-thio-β-D- xylopyranoside**

En opérant de façon analogue à l'exemple 239, au départ de 6-bromo-3-pyridinyl 2,3,4-tri-O-acétyl-5-thio-β-D-xylopyranoside obtenu selon la préparation IV et d'acide 2-méthyl-4-pyridineboronique, on obtient le produit désiré sous forme d'une poudre blanche (rendement = 23 %).
F = 214°C (recristallisé dans l'eau).

$[\alpha]_D^{28} = -51°$ (c = 0,52 ; DMSO).

**Exemple 246:**

**6-(5-chloro-3-pyridinyl)-3-pyridinyl 2,3,4-tri-O-acétyl-5-thio-β-D- xylopyranoside**

En opérant de façon analogue à l'exemple 1, au départ de 6-bromo-3-pyridinyl 2,3,4-tri-O-acétyl-5-thio-β-D-xylopyranoside obtenu selon la préparation IV et d'acide 5-chloro-3-pyridineboronique, on obtient le produit désiré sous la forme d'un solide blanc (rendement = 40%).
F = 203-205°C.

$[\alpha]_D^{30} = +8°$ (c = 0,31 ; DMSO).

**Exemple 247:**

**6-(5-chloro-3-pyridinyl)-3-pyridinyl 5-thio-β-D- xylopyranoside**

En opérant de façon analogue à l'exemple 42, au départ du produit préparé selon l'exemple 246, on obtient le produit désiré sous forme d'un solide blanc (rendement = 28 %).
F = 171-172°C.

$[\alpha]_D^{30} = -167°$ (c = 0,24 ; DMSO).

**Exemple 248:**

**5-(4-méthyl-3-pyridinyl)-3-pyridinyl 2,3,4-tri-O-acétyl-5-thio-β-D- xylopyranoside**

En opérant de façon analogue à l'exemple 137, au départ de 3-bromo-4-méthylpyridine, on obtient le produit désiré sous la forme d'un solide blanc (rendement = 9%).
F = 165°C.

$[\alpha]_D^{30} = -9°$ (c = 0,27; DMSO).

**Exemple 249:**

**5-(4-méthyl-3-pyridinyl)-3-pyridinyl 5-thio-β-D- xylopyranoside**

En opérant de façon analogue à l'exemple 2, au départ du produit préparé selon l'exemple 248, on obtient le produit désiré sous forme d'un solide blanc (rendement = 49%).
F = 201 °C.

$[\alpha]_D^{26} = -75°$ (c = 0,23 ; DMSO).

**Exemple 250:**

**2,6-di(4-fluoro-3-pyridinyl)-3-pyridinyl 2,3,4-tri-O-acétyl-5-thio-β-D- xylopyranoside**

En opérant de façon analogue à l'exemple 1, au départ de 2-chloro-6-iodo-3-pyridinyl 2,3,4-tri-O-acétyl-5-thio-β-D-xylopyranoside obtenu selon la préparation X et d'acide 6-fluoro-3-pyridineboronique, on obtient le produit désiré

sous la forme d'une poudre beige (rendement = 15%).
F = 169°C.

$[\alpha]_D^{26} = -35°$ (c = 0,45 ; DMSO).

**Exemple 251:**

**2,6-di(4-fluoro-3-pyridinyl)-3-pyridinyl 5-thio-β-D- xylopyranoside**

En opérant de façon analogue à l'exemple 2, au départ du produit préparé selon l'exemple 250, on obtient le produit désiré sous forme d'une poudre beige (rendement = 30%).
F = 209°C (recristallisé dans un mélange éthanol/eau).

$[\alpha]_D^{26} = -19°$ (c = 0,14 ; DMSO).

**Exemple 252:**

**2-(5-tétrazolyl))-3-pyridinyl 2,3,4-tri-O-acétyl-5-thio-β-D- xylopyranoside**

On prépare un mélange de 0,682 g (1,73 mM) de 2-cyano-3-pyridinyl 2,3,4-tri-O-acétyl-5-thio-β-D-xylopyranoside obtenu selon la préparation XV et 1,24 g (6,05 mM) d'azidotriméthylétain dans 10 ml de toluène et on agite ce mélange pendant 7 jours à 70°C. Après refroidissement, le milieu réactionnel est versé sur un mélange d'eau et d'acétate d'éthyle et amené à pH 1 par ajout d'une solution N d'acide chlorhydrique. La phase aqueuse est séparée, amenée à pH 5 par ajout d'une solution diluée de soude et extraite par de l'acétate d'éthyle. Cette phase organique est séparée, lavée à l'eau, séchée sur sulfate de magnésium et concentrée sous pression réduite. Le produit brut obtenu est purifié par chromatographie sur gel de silice en éluant avec un mélange dichlorométhane/méthanol (95/5 ;v/v). On obtient ainsi le composé attendu sous forme d'une mousse blanche (rendement = 30%).
F = 119-127°C.

$[\alpha]_D^{25} = -147°$ (c = 0,28 ; $CH_2Cl_2$).

**Exemple 253:**

**2-(5-tétrazolyl))-3-pyridinyl 5-thio-β-D- xylopyranoside**

En opérant de façon analogue à l'exemple 42, au départ du produit préparé selon l'exemple 252, on obtient le produit désiré sous forme d'une poudre blanche (rendement = 42%).
F = 170°C (recristallisé dans un mélange éthanol/eau).

$[\alpha]_D^{22} = -153°$ (c = 0,24 ; DMSO).

**Exemple 254:**

**6-(3,5-diméthyl-4-isoxazolyl)-2-fluoro-3-pyridinyl 2,3,4-tri-O-acétyl-5-thio-β-D-xylopyranoside**

En opérant de façon analogue à l'exemple 213, au départ de 2-fluoro-6-iodo-3-pyridinyl 2,3,4-tri-O-acétyl-5-thio-β-D-xylopyranoside obtenu selon la préparation XII et d'acide 3,5-diméthyl-4-isoxazoleboronique, on obtient le produit désiré qui est directement utilisé dans l'étape de désacétylation.

**Exemple 255:**

**6-(3,5-diméthyl-4-isoxazolyl)-2-fluoro-3-pyridinyl 5-thio-β-D- xylopyranoside**

En opérant de façon analogue à l'exemple 2, au départ du produit préparé selon l'exemple 254, on obtient le produit désiré sous forme d'une poudre blanche (rendement = 56%).
F = 101-104°C.

$[\alpha]_D^{32} = -42°$ (c = 0,12 ; DMSO).

**Exemple 256:**

**2-fluoro-6-(2-fluoro-3-pyridinyl)-3-pyridinyl 2,3,4-tri-O-acétyl-5-thio-β-D- xylopyranoside**

En opérant de façon analogue à l'exemple 254, au départ d'acide 2-fluoro-3-pyridineboronique, on obtient le produit désiré qui est directement utilisé dans l'étape de désacétylation.

**Exemple 257:**

**2-fluoro-6-(2-fluoro-3-pyridinyl)-3-pyridinyl 5-thio-β-D- xylopyranoside**

En opérant de façon analogue à l'exemple 2, au départ du produit préparé selon l'exemple 256, on obtient le produit désiré sous forme d'une poudre blanche (rendement = 53%).
F = 186-190°C.

$[\alpha]_D^{32} = -33°$ (c = 0,12 ; DMSO).

**Exemple 258:**

**2-fluoro-6-(5-pyrimidinyl)-3-pyridinyl 2,3,4-tri-O-acétyl-5-thio-β-D- xylopyranoside**

En opérant de façon analogue à l'exemple 254, au départ d'acide 5-pyrimidineboronique, on obtient le produit désiré qui est directement utilisé dans l'étape de désacétylation.

**Exemple 259:**

**2-fluoro-6-(5-pyrimidinyl)-3-pyridinyl 5-thio-β-D- xylopyranoside**

En opérant de façon analogue à l'exemple 2, au départ du produit préparé selon l'exemple 258, on obtient le produit désiré sous forme d'une poudre blanche (rendement = 33%).
F = 177-179°C.

$[\alpha]_D^{32} = -106°$ (c = 0,45 ; DMSO).

**Exemple 260:**

**2-fluoro-6-(6-fluoro-3-pyridinyl)-3-pyridinyl 2,3,4-tri-O-acétyl-5-thio-β-D- xylopyranoside**

En opérant de façon analogue à l'exemple 254, au départ d'acide 6-fluoro-3-pyridineboronique on obtient le produit désiré qui est directement utilisé dans l'étape de désacétylation.

**Exemple 261:**

**2-fluoro-6-(6-fluoro-3-pyridinyl)-3-pyridinyl 5-thio-β-D- xylopyranoside**

En opérant de façon analogue à l'exemple 2, au départ du produit préparé selon l'exemple 260, on obtient le produit désiré sous forme d'une poudre blanche (rendement = 65%).
F = 141-144°C.

$[\alpha]_D^{32} = -38°$ (c = 0,26 ; DMSO).

**Exemple 262:**

**2-fluoro-6-(2-fluoro-4-pyridinyl)-3-pyridinyl 2,3,4-tri-O-acétyl-5-thio-β-D- xylopyranoside**

En opérant de façon analogue à l'exemple 254, au départ d'acide 2-fluoro-4-pyridineboronique, on obtient le produit désiré qui est directement utilisé dans l'étape de désacétylation.

**Exemple 263:**

**2-fluoro-6-(2-fluoro-4-pyridinyl)-3-pyridinyl 5-thio-β-D- xylopyranoside**

En opérant de façon analogue à l'exemple 2, au départ du produit préparé selon l'exemple 262, on obtient le produit désiré sous forme d'une poudre blanche (rendement = 53%).
F = 179-182°C.

$[\alpha]_D^{32} = -37°$ (c = 0,10 ; DMSO).

**Exemple 264:**

**2-fluoro-6-(1-méthyl-1*H*-pyrazol- 4-yl)- 3-pyridinyl 2,3,4-tri- O- acétyl- 5-thio- β-D-xylopyranoside**

En opérant de façon analogue à l'exemple 254, au départ de 1-méthyl-4-(pinacolborane)-1*H*-pyrazole, on obtient le produit désiré qui est directement utilisé dans l'étape de désacétylation.

**Exemple 265:**

**2-fluoro-6-(1-méthyl-1*H*-pyrazol-4-yl)-3-pyridinyl 5-thio-β-D- xylopyranoside**

En opérant de façon analogue à l'exemple 2, au départ du produit préparé selon l'exemple 264, on obtient le produit désiré sous forme d'un solide blanc (rendement = 38%).
F = 183-185°C.

$[\alpha]_D^{32} = -23°$ (c = 0,13 ; DMSO).

**Exemple 266:**

**2-fluoro-6-(6-méthyl-3-pyridinyl)-3-pyridinyl 2,3,4-tri-O-acétyl-5-thio-β-D- xylopyranoside**

En opérant de façon analogue à l'exemple 254, au départ d'acide 6-méthyl-3-pyridineboronique, on obtient le produit désiré qui est directement utilisé dans l'étape de désacétylation.

**Exemple 267:**

**2-fluoro-6-(6-méthyl-3-pyridinyl)-3-pyridinyl 5-thio-β-D- xylopyranoside**

En opérant de façon analogue à l'exemple 2, au départ du produit préparé selon l'exemple 266, on obtient le produit désiré sous forme d'une poudre rose (rendement = 36%).
F = 189-190°C.

$[\alpha]_D^{32} = -20°$ (c = 0,10 ; DMSO).

**Exemple 268:**

**2-fluoro-6-(2-méthyl-4-pyridinyl)-3-pyridinyl 2,3,4-tri-O-acétyl-5-thio-β-D- xylopyranoside**

En opérant de façon analogue à l'exemple 254, au départ d'acide 2-méthyl-4-pyridineboronique, on obtient le produit désiré qui est directement utilisé dans l'étape de désacétylation.

**Exemple 269:**

**2-fluoro-6-(2-méthyl-4-pyridinyl)-3-pyridinyl 5-thio-β-D- xylopyranoside**

En opérant de façon analogue à l'exemple 2, au départ du produit préparé selon l'exemple 268, on obtient le produit désiré sous forme d'une poudre blanche (rendement = 24%).
F = 159-162°C.

$[\alpha]_D^{32} = -23°$ (c = 0,10 ; DMSO).

**Exemple 270:**

**2-fluoro-6-(6-cyano-3-pyridinyl)-3-pyridinyl 2,3,4-tri-O-acétyl-5-thio-β-D- xylopyranoside**

En opérant de façon analogue à l'exemple 254, au départ de 2-cyano-5-(pinacolborane)-pyridine, on obtient le produit désiré qui est directement utilisé dans l'étape de désacétylation.

**Exemple 271:**

**2-fluoro-6-(6-cyano-3-pyridinyl)-3-pyridinyl 5-thio-β-D- xylopyranoside**

En opérant de façon analogue à l'exemple 2, au départ du produit préparé selon l'exemple 270, on obtient le produit désiré sous forme d'une poudre blanche (rendement = 37%).
F = 158-162°C.

$[\alpha]_D^{32} = -19°$ (c = 0,14 ; DMSO).

**Exemple 272:**

**5-fluoro-6-(3-pyridinyl)-3-pyridinyl 2,3,4-tri-O-acétyl-5-thio-β-D- xylopyranoside**

En opérant de façon analogue à l'exemple 213, au départ de 6-chloro-5-fluoro-3-pyridinyl 2,3,4-tri-O-acétyl-5-thio-β-D-xylopyranoside obtenu selon la préparation XI et d'acide 3-pyridineboronique, on obtient le produit désiré qui est directement utilisé dans l'étape de désacétylation.

**Exemple 273:**

**5-fluoro-6-(3-pyridinyl)-3-pyridinyl 5-thio-β-D- xylopyranoside**

En opérant de façon analogue à l'exemple 2, au départ du produit préparé selon l'exemple 272, on obtient le produit désiré sous forme d'une poudre blanche (rendement = 58%).
F = 179°C.

$[\alpha]_D^{32} = -33°$ (c = 0,10 ; DMSO).

**Exemple 274:**

**5-fluoro-6-(2-fluoro-4-pyridinyl)-3-pyridinyl 2,3,4-tri-O-acétyl-5-thio-β-D- xylopyranoside**

En opérant de façon analogue à l'exemple 272, au départ d'acide 2-fluoro-4-pyridineboronique, on obtient le produit désiré qui est directement utilisé dans l'étape de désacétylation.

**Exemple 275:**

**5-fluoro-6-(2-fluoro-4-pyridinyl)-3-pyridinyl 5-thio-β-D- xylopyranoside**

En opérant de façon analogue à l'exemple 2, au départ du produit préparé selon l'exemple 274, on obtient le produit désiré sous forme d'une poudre blanche (rendement = 53%).
F = 209-212°C.

$[\alpha]_D^{27} = -68°$ (c = 0,10 ; DMSO).

**Exemple 276:**

**2-chloro-5-(5-pyrimidinyl)-3-pyridinyl 2,3,4-tri-O-acétyl-5-thio-β-D- xylopyranoside**

En opérant de façon analogue à l'exemple 259, au départ de 5-bromo-2-chloro-3-pyridinyl 2,3,4-tri-O-acétyl-5-thio-β-D-xylopyranoside obtenu selon la préparation XIV, on obtient le produit attendu sous forme d'une mousse blanche (rendement = 48 %).
F = 72-90 °C.

$[\alpha]_D^{25}$ = -44° (c = 0,21 ; DMSO).

**Exemple 277:**

**2-chloro-5-(5-pyrimidinyl)-3-pyridinyl 5-thio-β-D- xylopyranoside**

En opérant de façon analogue à l'exemple 2, au départ du produit préparé selon l'exemple 276, on obtient le produit désiré sous forme de flocons blancs (rendement = 81%).
F = 174-189°C.

$[\alpha]_D^{25}$ = 60° (c = 0,22 ; DMSO).

**Exemple 278:**

**2-chloro-5-(6-fluoro-3-pyridinyl)-3-pyridinyl 2,3,4-tri-O-acétyl-5-thio-β-D- xylopyranoside**

En opérant de façon analogue à l'exemple 276, au départ d'acide 6-fluoro-3-pyridineboronique, on obtient le produit attendu sous forme d'une mousse blanche (rendement = 38 %).
F = 70-90 °C.

$[\alpha]_D^{24}$ = -26° (c = 0,22 ; DMSO).

**Exemple 279:**

**2-chloro-5-(6-fluoro-3-pyridinyl)-3-pyridinyl 5-thio-β-D- xylopyranoside**

En opérant de façon analogue à l'exemple 2, au départ du produit préparé selon l'exemple 278, on obtient le produit désiré sous forme de flocons blancs (rendement = 84%).
F = 176-197°C.

$[\alpha]_D^{30}$ = -51° (c = 0,15 ; DMSO).

**Exemple 280:**

**2-chloro-6-(5-pyrimidinyl)-3-pyridinyl 2,3,4-tri-O-acétyl-5-thio-β-D- xylopyranoside**

En opérant de façon analogue à l'exemple 259, au départ de 2-chloro-6-iodo-3-pyridinyl 2,3,4-tri-O-acétyl-5-thio-β-D-xylopyranoside obtenu selon la préparation X on obtient le produit attendu sous forme d'une mousse blanche (rendement = 37 %).
F = 168 °C.

$[\alpha]_D^{36}$ = -41° (c = 0,11; DMSO).

**Exemple 281:**

**2-chloro-6-(5-pyrimidinyl)-3-pyridinyl 5-thio-β-D- xylopyranoside**

En opérant de façon analogue à l'exemple 2, au départ du produit préparé selon l'exemple 280, on obtient le

produit désiré sous forme d'une poudre blanche (rendement = 20%).
F = 100˚C.

$[\alpha]_D^{26} = 31°$ (c = 0,11 ; DMSO).

**Exemple 282:**

**2-fluoro-5-(2-pyridinyl)-3-pyridinyl 2,3,4-tri-O-acétyl-5-thio-β-D- xylopyranoside**

En opérant de façon analogue à l'exemple 231, au départ de 5-bromo-2-fluoro-3-pyridinyl 2,3,4-tri-O-acétyl-5-thio-β-D-xylopyranoside obtenu selon la préparation VIII et de 2-bromopyridine, on obtient le produit désiré qui est directement utilisé dans l'étape de désacétylation.

**Exemple 283:**

**2-fluoro-5-(2-pyridinyl)-3-pyridinyl 5-thio-β-D- xylopyranoside**

En opérant de façon analogue à l'exemple 2, au départ du produit préparé selon l'exemple 282, on obtient le produit désiré sous forme d'un solide blanc (rendement = 79%).
F = 145-146˚C.

**Exemple 284:**

**2-fluoro-5-(5-pyrimidinyl)-3-pyridinyl 2,3,4-tri-O-acétyl-5-thio-β-D- xylopyranoside**

En opérant de façon analogue à la première partie de l'exemple 137, on prépare, au départ de la 5-bromopyrimidine, la 5-(pinacolborane)pyrimidine , que l'on fait réagir immédiatement dans le même réacteur avec le 5-bromo-2-fluoro-3-pyridinyl 2,3,4-tri-O-acétyl-5-thio-β-D-xylopyranoside obtenu selon la préparation VIII, dans des conditions analogues à celles appliquées pour la préparation de l'exemple 177. On obtient ainsi le produit attendu sous forme d'un solide jaune (rendement = 40 %).
F = 96-97 ˚C.

$[\alpha]_D^{30} = -11°$ (c = 0.24 ; DMSO).

**Exemple 285:**

**2-fluoro-5-(5-pyrimidinyl)-3-pyridinyl 5-thio-β-D- xylopyranoside**

En opérant de façon analogue à l'exemple 2, au départ du produit préparé selon l'exemple 284, on obtient le produit désiré sous forme d'un solide blanc (rendement = 60%).
F = 204-205˚C.
Les structures des composés de formule 1 décrits dans les exemples qui précèdent ont été regroupées dans le tableau suivant :

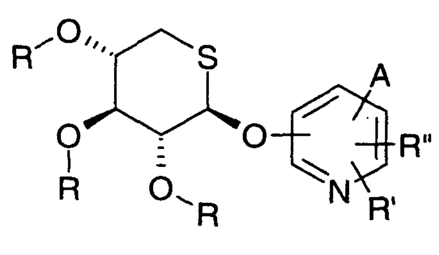

avec A =

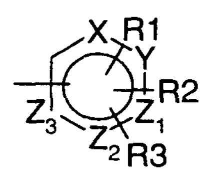

| Ex | Pos -N | R' | R" | Pos-A | A | | | | | | | | R |
|---|---|---|---|---|---|---|---|---|---|---|---|---|---|
| | | | | | X | Y | Z1 | Z2 | Z3 | $R_1$ | $R_2$ | $R_3$ | |
| 1 | 3 | H | H | 6 | 3-N | C | C | C | C | 2-$OCH_3$ | H | H | Ac |
| 2 | 3 | H | H | 6 | 3-N | C | C | C | C | 2-$OCH_3$ | H | H | H |
| 3 | 3 | 4-$CH_3$ | H | 2 | 3-N | C | C | C | C | H | H | H | Ac |
| 4 | 3 | 4-$CH_3$ | H | 2 | 3-N | C | C | C | C | H | H | H | H |
| 5 | 3 | 6-$CH_3$ | H | 2 | 3-O | Is | C | C | C | H | H | H | Ac |
| 6 | 3 | 6-$CH_3$ | H | 2 | 3-O | Is | C | C | C | H | H | H | H |
| 7 | 3 | H | H | 2 | 2-O | Is | C | C | C | 5-$CH_3$ | H | H | Ac |
| 8 | 3 | H | H | 2 | 2-O | Is | C | C | C | 5-$CH_3$ | H | H | H |
| 9 | 3 | 2-Cl | H | 6 | 3-N | C | C | C | C | H | H | H | Ac |
| 10 | 3 | 2-Cl | H | 6 | 3-N | C | C | C | C | H | H | H | H |
| 11 | 3 | 2-F | H | 5 | 4-N | C | C | C | C | H | H | H | Ac |
| 12 | 3 | 2-F | H | 5 | 4-N | C | C | C | C | H | H | H | H |
| 13 | 3 | 2-Cl | H | 6 | 3-N | C | C | C | C | 2-$OCH_3$ | H | H | Ac |
| 14 | 3 | 2-Cl | H | 6 | 3-N | C | C | C | C | 2-$OCH_3$ | H | H | H |
| 15 | 3 | 2-Cl | H | 6 | 3-N | C | C | C | C | 2-F | H | H | Ac |
| 16 | 3 | 2-Cl | H | 6 | 3-N | C | C | C | C | 2-F | H | H | H |
| 17 | 3 | 2-Cl | H | 6 | 4-N | C | C | C | C | 2-F | H | H | Ac |
| 18 | 3 | 2-Cl | H | 6 | 4-N | C | C | C | C | 2-F | H | H | H |
| 19 | 3 | 2-Cl | H | 6 | 3-N | C | C | C | C | 6-F | H | H | Ac |
| 20 | 3 | 2-Cl | H | 6 | 3-N | C | C | C | C | 6-F | H | H | H |
| 21 | 3 | 2-F | H | 6 | 4-N | C | C | C | C | H | H | H | Ac |
| 22 | 3 | 2-F | H | 6 | 4-N | C | C | C | C | H | H | H | H |
| 23 | 3 | 2-Cl | H | 6 | 4-N | C | C | C | C | H | H | H | Ac |
| 24 | 3 | 2-Cl | H | 6 | 4-N | C | C | C | C | H | H | H | H |
| 25 | 3 | H | H | 5 | 3-N | C | C | C | C | 2-Cl | H | H | Ac |
| 26 | 3 | H | H | 5 | 3-N | C | C | C | C | 2-Cl | H | H | H |
| 27 | 3 | H | H | 5 | 3-N | C | C | C | C | 6-$OCH_3$ | H | H | Ac |
| 28 | 3 | H | H | 5 | 3-N | C | C | C | C | 6-$OCH_3$ | H | H | H |
| 29 | 3 | H | H | 5 | 3-N | C | C | C | C | 6-Cl | H | H | Ac |
| 30 | 3 | H | H | 5 | 3-N | C | C | C | C | 6-Cl | H | H | H |
| 31 | 3 | H | H | 5 | 3-N | C | C | C | C | 2-$OCH_3$ | H | H | Ac |

(suite)

| Ex | Pos -N | R' | R" | Pos-A | A | | | | | | | | R |
|---|---|---|---|---|---|---|---|---|---|---|---|---|---|
| 32 | 3 | H | H | 5 | 3-N | C | C | C | C | 2-$OCH_3$ | H | H | H |
| 33 | 3 | H | H | 6 | 2-O | Is | C | C | C | 5-$CH_3$ | H | H | Ac |
| 34 | 3 | H | H | 6 | 2-O | Is | C | C | C | 5-$CH_3$ | H | H | H |
| 35 | 3 | H | H | 6 | 3-N | C | C | C | C | 6-$CH_3$ | H | H | Ac |
| 36 | 3 | H | H | 6 | 3-N | C | C | C | C | 6-$CH_3$ | H | H | H |
| 37 | 3 | H | H | 6 | 3-N | C | C | C | C | 6-Cl | H | H | Ac |
| 38 | 3 | H | H | 6 | 3-N | C | C | C | C | 6-Cl | H | H | H |
| 39 | 3 | H | H | 6 | 3-N | C | C | C | C | 2-Cl | H | H | Ac |
| 40 | 3 | H | H | 6 | 3-N | C | C | C | C | 2-Cl | H | H | H |
| 41 | 3 | 5-Cl | H | 6 | 4-N | C | C | C | C | H | H | H | Ac |
| 42 | 3 | 5-Cl | H | 6 | 4-N | C | C | C | C | H | H | H | H |
| 43 | 3 | 5-F | H | 6 | 4-N | C | C | C | C | H | H | H | Ac |
| 44 | 3 | 5-F | H | 6 | 4-N | C | C | C | C | H | H | H | H |
| 45 | 2 | H | H | 6 | 4-N | C | C | C | C | H | H | H | Ac |
| 46 | 2 | H | H | 6 | 4-N | C | C | C | C | H | H | H | H |
| 47 | 2 | H | H | 6 | 3-N | C | C | C | C | H | H | H | Ac |
| 48 | 2 | H | H | 6 | 3-N | C | C | C | C | H | H | H | H |
| 49 | 3 | H | H | 5 | 2-N | C | C | C | C | 5-F | H | H | Ac |
| 50 | 3 | H | H | 5 | 2-N | C | C | C | C | 5-F | H | H | H |
| 51 | 3 | 2-F | H | 5 | 3-N | C | C | C | C | H | H | H | Ac |
| 52 | 3 | 2-F | H | 5 | 3-N | C | C | C | C | H | H | H | H |
| 53 | 3 | H | H | 5 | 4-N | C | C | C | C | 3-$CH_3$ | H | H | Ac |
| 54 | 3 | H | H | 5 | 4-N | C | C | C | C | 3-$CH_3$ | H | H | H |
| 55 | 3 | H | H | 5 | 2-N | C | C | C | C | 3-$CH_3$ | H | H | Ac |
| 56 | 3 | H | H | 5 | 2-N | C | C | C | C | 3-$CH_3$ | H | H | H |
| 57 | 3 | H | H | 5 | 5-S | Is | C | N | C | 2-$CH_3$ | 4-$CH_3$ | - | Ac |
| 58 | 3 | H | H | 5 | 5-S | Is | C | N | C | 2-$CH_3$ | 4-$CH_3$ | - | H |
| 59 | 3 | H | H | 5 | 4-N | Is | N | C | C | 1-$CH_3$ | 3-$CF_3$ | H | Ac |
| 60 | 3 | H | H | 5 | 4-N | Is | N | C | C | 1-$CH_3$ | 3-$CF_3$ | H | H |
| 61 | 3 | H | H | 5 | 4-N | Is | N | C | C | 5-$CH_3$ | 3-$CF_3$ | H | Ac |
| 62 | 3 | H | H | 5 | 4-N | Is | N | C | C | 5-$CH_3$ | 3-$CF_3$ | H | H |
| 63 | 3 | H | H | 5 | 4-O | Is | N | C | C | 5-$CH_3$ | H | - | Ac |
| 64 | 3 | H | H | 5 | 4-O | Is | N | C | C | 5-$CH_3$ | H | - | H |
| 65 | 3 | H | H | 5 | 2-N | C | C | N | C | H | H | H | Ac |
| 66 | 3 | H | H | 5 | 2-N | C | C | N | C | H | H | H | |
| 67 | 3 | H | H | 5 | 2-N | C | N | C | C | H | H | H | Ac |

(suite)

| Ex | Pos -N | R' | R" | Pos-A | A | | | | | | | | R |
|---|---|---|---|---|---|---|---|---|---|---|---|---|---|
| 68 | 3 | H | H | 5 | 2-N | C | N | C | C | H | H | H | H |
| 69 | 3 | H | H | 5 | 2-S | Is | C | N | C | H | H | - | Ac |
| 70 | 3 | H | H | 5 | 2-S | Is | C | N | C | H | H | - | H |
| 71 | 3 | H | H | 5 | 5-N | Is | C | N | C | 1-$CH_3$ | H | H | Ac |
| 72 | 3 | H | H | 5 | 5-N | Is | C | N | C | 1-$CH_3$ | H | H | H |
| 73 | 3 | H | H | 5 | 4-N | C | C | C | C | 2-Cl | H | H | Ac |
| 74 | 3 | H | H | 5 | 4-N | C | C | C | C | 2-Cl | H | H | H |
| 75 | 3 | H | H | 5 | 4-N | C | C | C | C | 3-Cl | H | H | Ac |
| 76 | 3 | H | H | 5 | 4-N | C | C | C | C | 3-Cl | H | H | H |
| 77 | 3 | H | H | 6 | 3-N | C | C | C | C | 6-$OCH_3$ | H | H | Ac |
| 78 | 3 | H | H | 6 | 3-N | C | C | C | C | 6-$OCH_3$ | H | H | H |
| 79 | 3 | 4-$CH_3$ | H | 2 | 3-O | Is | C | C | C | H | H | H | Ac |
| 80 | 3 | 4-$CH_3$ | H | 2 | 3-O | Is | C | C | C | H | H | H | H |
| 81 | 3 | 4-$CH_3$ | H | 2 | 4-O | Is | N | C | C | 3-$CH_3$ | 5-$CH_3$ | - | Ac |
| 82 | 3 | 4-$CH_3$ | H | 2 | 4-O | Is | N | C | C | 3-$CH_3$ | 5-$CH_3$ | - | H |
| 83 | 3 | H | H | 4 | 3-N | C | C | C | C | H | H | H | Ac |
| 84 | 3 | H | H | 4 | 3-N | C | C | C | C | H | H | H | H |
| 85 | 3 | H | H | 2 | 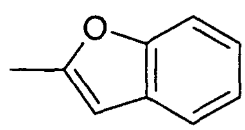 | | | | | | | H | Ac |
| 86 | 3 | H | H | 2 | 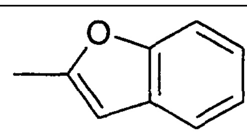 | | | | | | | H | H |
| 87 | 3 | H | H | 4 | 3-O | Is | C | C | C | H | H | H | Ac |
| 88 | 3 | H | H | 4 | 3-O | Is | C | C | C | H | H | H | H |
| 89 | 3 | H | H | 4 | 4-N | C | C | C | C | H | H | H | Ac |
| 90 | 3 | H | H | 4 | 4-N | C | C | C | C | H | H | H | H |
| 91 | 3 | H | H | 5 | 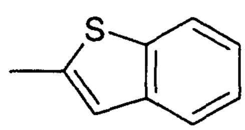 | | | | | | | H | Ac |
| 92 | 3 | H | H | 5 | 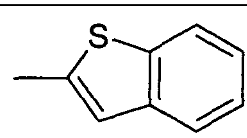 | | | | | | | H | H |
| 93 | 3 | H | H | 2 | 2-S | Is | C | C | C | H | H | H | Ac |
| 94 | 3 | H | H | 2 | 2-S | Is | C | C | C | H | H | H | H |

(suite)

| Ex | Pos -N | R' | R" | Pos-A | A | | | | | | | | R |
|---|---|---|---|---|---|---|---|---|---|---|---|---|---|
| 95 | 3 | H | H | 5 | 2-O | Is | C | C | C | 5-$CH_3$ | H | H | Ac |
| 96 | 3 | H | H | 5 | 2-O | Is | C | C | C | 5-$CH_3$ | H | H | H |
| 97 | 3 | H | H | 6 | 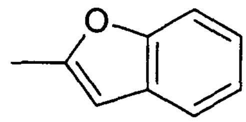 | | | | | | | H | Ac |
| 98 | 3 | H | H | 6 | 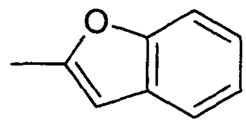 | | | | | | | H | H |
| 99 | 3 | H | H | 6 | 3-S | Is | C | C | C | H | H | H | Ac |
| 100 | 3 | H | H | 6 | 3-S | Is | C | C | C | H | H | H | H |
| 101 | 3 | H | H | 2 | 2-O | Is | C | C | C | H | H | H | Ac |
| 102 | 3 | H | H | 2 | 2-O | Is | C | C | C | H | H | H | H |
| 103 | 3 | H | H | 6 | 2-O | Is | C | C | C | H | H | H | Ac |
| 104 | 3 | H | H | 6 | 2-O | Is | C | C | C | H | H | H | H |
| 105 | 3 | H | H | 2 | 4-N | C | C | C | C | H | H | H | Ac |
| 106 | 3 | H | H | 2 | 4-N | C | C | C | C | H | H | H | H |
| 107 | 3 | H | H | 6 | 2-S | Is | C | C | C | H | H | H | Ac |
| 108 | 3 | H | H | 6 | 2-S | Is | C | C | C | H | H | H | H |
| 109 | 3 | H | H | 5 | 2-O | Is | C | C | C | H | H | H | Ac |
| 110 | 3 | H | H | 5 | 2-O | Is | C | C | C | H | H | H | H |
| 111 | 3 | H | H | 2 | 3-N | C | C | C | C | H | H | H | Ac |
| 112 | 3 | H | H | 2 | 3-N | C | C | C | C | H | H | H | H |
| 113 | 3 | H | H | 2 | 3-O | Is | C | C | C | H | H | H | Ac |
| 114 | 3 | H | H | 2 | 3-O | Is | C | C | C | H | H | H | H |
| 115 | 3 | H | H | 5 | 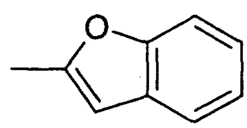 | | | | | | | H | Ac |
| 116 | 3 | H | H | 5 | 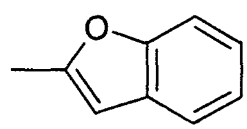 | | | | | | | H | H |
| 117 | 3 | H | H | 6 | 3-O | Is | C | C | C | H | H | H | Ac |
| 118 | 3 | H | H | 6 | 3-O | Is | C | C | C | H | H | H | H |
| 119 | 3 | H | H | 6 | 3-N | C | C | C | C | H | H | H | Ac |
| 120 | 3 | H | H | 6 | 3-N | C | C | C | C | H | H | H | H |
| 121 | 3 | H | H | 6 | 4-N | C | C | C | C | H | H | H | Ac |
| 122 | 3 | H | H | 6 | 4-N | C | C | C | C | H | H | H | H |

(suite)

| Ex | Pos -N | R' | R" | Pos-A | A | | | | | | | | R |
|---|---|---|---|---|---|---|---|---|---|---|---|---|---|
| 123 | 3 | H | H | 5 | 4-N | C | C | C | C | H | H | H | Ac |
| 124 | 3 | H | H | 5 | 4-N | C | C | C | C | H | H | H | H |
| 125 | 3 | H | H | 5 | 3-O | Is | C | C | C | H | H | H | Ac |
| 126 | 3 | H | H | 5 | 3-O | Is | C | C | C | H | H | H | H |
| 127 | 3 | H | H | 5 | 3-N | C | C | C | C | H | H | H | Ac |
| 128 | 3 | H | H | 5 | 3-N | C | C | C | C | H | H | H | H |
| 129 | 4 | H | H | 2 | 4-N | C | C | C | C | H | H | H | Ac |
| 130 | 4 | H | H | 2 | 4-N | C | C | C | C | H | H | H | H |
| 131 | 4 | H | H | 2 | 3-N | C | C | C | C | 6-F | H | H | Ac |
| 132 | 4 | H | H | 2 | 3-N | C | C | C | C | 6-F | H | H | H |
| 133 | 4 | H | H | 2 | 3-N | C | C | C | C | H | H | H | Ac |
| 134 | 4 | H | H | 2 | 3-N | C | C | C | C | H | H | H | H |
| 135 | 3 | H | H | 5 | 2-S | Is | C | C | C | H | H | H | Ac |
| 136 | 3 | H | H | 5 | 2-S | Is | C | C | C | H | H | H | H |
| 137 | 3 | H | H | 5 | 3-N | C | C | C | C | 5-$OCH_3$ | H | H | Ac |
| 138 | 3 | H | H | 5 | 3-N | C | C | C | C | 5-$OCH_3$ | H | H | H |
| 139 | 3 | H | H | 6 | 4-O | Is | N | C | C | 5-$CH_3$ | H | - | Ac |
| 140 | 3 | H | H | 6 | 4-O | Is | N | C | C | 5-$CH_3$ | H | - | H |
| 141 | 3 | H | H | 6 | 3-N | C | C | C | C | 5-$CH_3$ | H | H | Ac |
| 142 | 3 | H | H | 6 | 3-N | C | C | C | C | 5-$CH_3$ | H | H | H |
| 143 | 3 | H | H | 5 | 3-N | C | C | C | C | 5-$CH_3$ | H | H | Ac |
| 144 | 3 | H | H | 5 | 3-N | C | C | C | C | 5-$CH_3$ | H | H | H |
| 145 | 3 | H | H | 5 | 3-N | C | C | C | C | 5-Cl | H | H | Ac |
| 146 | 3 | H | H | 5 | 3-N | C | C | C | C | 5-Cl | H | H | H |
| 147 | 3 | H | H | 5 | 3-N | C | C | C | C | 6-CN | H | H | Ac |
| 148 | 3 | H | H | 5 | 3-N | C | C | C | C | 6-CN | H | H | H |
| 149 | 3 | H | H | 6 | 3-N | C | C | C | C | 5-F | H | H | Ac |
| 150 | 3 | H | H | 6 | 3-N | C | C | C | C | 5-F | H | H | H |
| 151 | 3 | H | H | 5 | 3-N | C | C | C | C | 2-$CH_3$ | H | H | Ac |
| 152 | 3 | H | H | 5 | 3-N | C | C | C | C | 2-$CH_3$ | H | H | H |
| 153 | 3 | H | H | 5 | 3-N | C | C | C | C | 5-F | H | H | Ac |
| 154 | 3 | H | H | 5 | 3-N | C | C | C | C | 5-F | H | H | H |
| 155 | 3 | H | H | 6 | 3-N | C | C | C | C | 5-$OCH_3$ | H | H | Ac |
| 156 | 3 | H | H | 6 | 3-N | C | C | C | C | 5-$OCH_3$ | H | H | H |

(suite)

| Ex | Pos -N | R' | R" | Pos-A | A | | | | | | | | R |
|---|---|---|---|---|---|---|---|---|---|---|---|---|---|
| 157 | 3 | H | H | 5 | 4-N | Is | N | C | C | 1-$CH_3$ | 3-$CH_3$ | 5-$CH_3$ | Ac |
| 158 | 3 | H | H | 5 | 4-N | Is | N | C | C | 1-$CH_3$ | 3-$CH_3$ | 5-$CH_3$ | H |
| 159 | 3 | H | H | 5 | 4-N | Is | N | C | C | 3-$CH_3$ | 5-$CH_3$ | H | Ac |
| 160 | 3 | H | H | 5 | 4-N | Is | N | C | C | 3-$CH_3$ | 5-$CH_3$ | H | H |
| 161 | 3 | H | H | 6 | 3-N | C | C | C | C | 6-CN | H | H | Ac |
| 162 | 3 | H | H | 6 | 3-N | C | C | C | C | 6-CN | H | H | H |
| 163 | 3 | H | H | 6 | 4-N | Is | N | C | C | 1-$CH_3$ | 3-$CH_3$ | 5-$CH_3$ | Ac |
| 164 | 3 | H | H | 6 | 4-N | Is | N | C | C | 1-$CH_3$ | 3-$CH_3$ | 5-$CH_3$ | H |
| 165 | 3 | H | H | 6 | 4-N | Is | N | C | C | 1-$CH_3$ | H | H | Ac |
| 166 | 3 | H | H | 6 | 4-N | Is | N | C | C | 1-$CH_3$ | H | H | H |
| 167 | 3 | H | H | 6 | 4-N | Is | N | C | C | 3-$CH_3$ | 5-$CH_3$ | H | Ac |
| 168 | 3 | H | H | 6 | 4-N | Is | N | C | C | 3-$CH_3$ | 5-$CH_3$ | H | H |
| 169 | 3 | H | H | 5 | 3-N | C | C | C | C | 6-F | H | H | Ac |
| 170 | 3 | H | H | 5 | 3-N | C | C | C | C | 6-F | H | H | H |
| 171 | 3 | H | H | 5 | 4-N | Is | N | C | C | 1-$CH_3$ | H | H | Ac |
| 172 | 3 | H | H | 5 | 4-N | Is | N | C | C | 1-$CH_3$ | H | H | H |
| 173 | 3 | H | H | 6 | 5-S | Is | C | N | C | 2-$CH_3$ | 4-$CH_3$ | - | Ac |
| 174 | 3 | H | H | 6 | 5-S | Is | C | N | C | 2-$CH_3$ | 4-$CH_3$ | - | H |
| 175 | 3 | H | H | 6 | 3-N | C | C | C | C | 2-$CH_3$ | H | H | Ac |
| 176 | 3 | H | H | 6 | 3-N | C | C | C | C | 2-$CH_3$ | H | H | H |
| 177 | 3 | H | H | 5 | 5-N | C | N | C | C | H | H | H | Ac |
| 178 | 3 | H | H | 5 | 5-N | C | N | C | C | H | H | H | H |
| 179 | 3 | H | H | 5 | 4-O | Is | N | C | C | 3-$CH_3$ | 5-$CH_3$ | - | Ac |
| 180 | 3 | H | H | 5 | 4-O | Is | N | C | C | 3-$CH_3$ | 5-$CH_3$ | - | H |
| 181 | 3 | H | H | 2 | 4-O | Is | N | C | C | 3-$CH_3$ | 5-$CH_3$ | - | Ac |
| 182 | 3 | H | H | 2 | 4-O | Is | N | C | C | 3-$CH_3$ | 5-$CH_3$ | - | H |
| 183 | 3 | H | H | 5 | 3-N | C | C | C | C | 2-F | H | H | Ac |
| 184 | 3 | H | H | 5 | 3-N | C | C | C | C | 2-F | H | H | H |
| 185 | 3 | H | H | 5 | 4-N | C | C | C | C | 2-F | H | H | Ac |
| 186 | 3 | H | H | 5 | 4-N | C | C | C | C | 2-F | H | H | H |
| 187 | 3 | H | H | 6 | 3-N | C | C | C | C | 2-F | H | H | Ac |
| 188 | 3 | H | H | 6 | 3-N | C | C | C | C | 2-F | H | H | H |
| 189 | 3 | H | H | 6 | 5-N | C | N | C | C | H | H | H | Ac |
| 190 | 3 | H | H | 6 | 5-N | C | N | C | C | H | H | H | H |
| 191 | 3 | H | H | 6 | 4-O | Is | N | C | C | 3-$CH_3$ | 5-$CH_3$ | - | Ac |
| 192 | 3 | H | H | 6 | 4-O | Is | N | C | C | 3-$CH_3$ | 5-$CH_3$ | - | H |
| 193 | 3 | H | H | 6 | 3-N | C | C | C | C | 6-F | H | H | Ac |

(suite)

| Ex | Pos -N | R' | R" | Pos-A | A | | | | | | | | R |
|---|---|---|---|---|---|---|---|---|---|---|---|---|---|
| 194 | 3 | H | H | 6 | 3-N | C | C | C | C | 6-F | H | H | H |
| 195 | 3 | 2-F | H | 5 | 4-N | C | C | C | C | 2-F | H | H | Ac |
| 196 | 3 | 2-F | H | 5 | 4-N | C | C | C | C | 2-F | H | H | H |
| 197 | 3 | 2-F | H | 5 | 3-N | C | C | C | C | 6-F | H | H | Ac |
| 198 | 3 | 2-F | H | 5 | 3-N | C | C | C | C | 6-F | H | H | H |
| 199 | 3 | H | H | 5 | 3-S | Is | C | C | C | H | H | H | Ac |
| 200 | 3 | H | H | 5 | 3-S | Is | C | C | C | H | H | H | H |
| 201 | 3 | H | H | 6 | 4-N | C | C | C | C | 2-Cl | H | H | Ac |
| 202 | 3 | H | H | 6 | 4-N | C | C | C | C | 2-Cl | H | H | H |
| 203 | 3 | H | H | 4 | 4-O | Is | N | C | C | 3-$CH_3$ | 5-$CH_3$ | - | Ac |
| 204 | 3 | H | H | 4 | 4-O | Is | N | C | C | 3-$CH_3$ | 5-$CH_3$ | - | H |
| 205 | 3 | 6-$CH_3$ | H | 2 | 3-N | C | C | C | C | H | H | H | Ac |
| 206 | 3 | 6-$CH_3$ | H | 2 | 3-N | C | C | C | C | H | H | H | H |
| 207 | 3 | 6-$CH_3$ | H | 2 | 4-N | C | C | C | C | H | H | H | Ac |
| 208 | 3 | 6-$CH_3$ | H | 2 | 4-N | C | C | C | C | H | H | H | H |
| 209 | 3 | H | H | 6 | 4-N | C | C | C | C | 2-F | H | H | Ac |
| 210 | 3 | H | H | 6 | 4-N | C | C | C | C | 2-F | H | H | H |
| 211 | 3 | H | H | 2 | 5-N | C | N | C | C | H | H | H | Ac |
| 212 | 3 | H | H | 2 | 5-N | C | N | C | C | H | H | H | H |
| 213 | 3 | 5-F | H | 6 | 3-N | C | C | C | C | 6-F | H | H | Ac |
| 214 | 3 | 5-F | H | 6 | 3-N | C | C | C | C | 6-F | H | H | H |
| 215 | 3 | 5-F | H | 6 | 3-N | C | C | C | C | 6-$CH_3$ | H | H | Ac |
| 216 | 3 | 5-F | H | 6 | 3-N | C | C | C | C | 6-$CH_3$ | H | H | H |
| 217 | 3 | H | H | 5 | 5-N | C | N | C | C | 2-$OCH_3$ | H | H | Ac |
| 218 | 3 | H | H | 5 | 5-N | C | N | C | C | 2-$OCH_3$ | H | H | H |
| 219 | 3 | 5-F | H | 6 | 4-N | C | C | C | C | 2-$CH_3$ | H | H | Ac |
| 220 | 3 | 5-F | H | 6 | 4-N | C | C | C | C | 2-$CH_3$ | H | H | H |
| 221 | 3 | 5-F | H | 6 | 3-N | C | C | C | C | 2-$OCH_3$ | H | H | Ac |
| 222 | 3 | 5-F | H | 6 | 3-N | C | C | C | C | 2-$OCH_3$ | H | H | H |
| 223 | 3 | 5-F | H | 6 | 4-N | Is | N | C | C | 1-$CH_3$ | H | H | Ac |
| 224 | 3 | 5-F | H | 6 | 4-N | Is | N | C | C | 1-$CH_3$ | H | H | H |
| 225 | 3 | 5-F | H | 6 | 4-O | Is | N | C | C | 3-$CH_3$ | 5-$CH_3$ | - | Ac |
| 226 | 3 | 5-F | H | 6 | 4-O | Is | N | C | C | 3-$CH_3$ | 5-$CH_3$ | - | H |

(suite)

| Ex | Pos -N | R' | R" | Pos-A | A | | | | | | | | R |
|---|---|---|---|---|---|---|---|---|---|---|---|---|---|
| 227 | 3 | H | H | 6 | 5-N | C | N | C | C | 2-$OCH_3$ | H | H | Ac |
| 228 | 3 | H | H | 6 | 5-N | C | N | C | C | 2-$OCH_3$ | H | H | H |
| 229 | 3 | 5-F | H | 6 | 5-N | C | N | C | C | H | H | H | Ac |
| 230 | 3 | 5-F | H | 6 | 5-N | C | N | C | C | H | H | H | H |
| 231 | 3 | H | H | 5 | 2-N | C | C | C | C | 3-Cl | H | H | Ac |
| 232 | 3 | H | H | 5 | 2-N | C | C | C | C | 3-Cl | H | H | H |
| 233 | 3 | H | H | 5 | 2-N | C | C | C | C | 5-$CH_3$ | H | H | Ac |
| 234 | 3 | H | H | 5 | 2-N | C | C | C | C | 5-$CH_3$ | H | H | H |
| 235 | 3 | H | H | 5 | 2-N | C | C | C | C | 4-$CH_3$ | H | H | Ac |
| 236 | 3 | H | H | 5 | 2-N | C | C | C | C | 4-$CH_3$ | H | H | H |
| 237 | 3 | H | H | 5 | 2-N | C | C | C | C | 6-$CH_3$ | H | H | Ac |
| 238 | 3 | H | H | 5 | 2-N | C | C | C | C | 6-$CH_3$ | H | H | H |
| 239 | 3 | 6-$CH_3$ | H | 2 | 4-O | Is | N | C | C | 3-$CH_3$ | 5-$CH_3$ | - | H |
| 240 | 3 | H | H | 2 | 3-N | C | C | C | C | 6-F | H | H | H |
| 241 | 4 | H | H | 2 | 4-O | Is | N | C | C | 3-$CH_3$ | 5-$CH_3$ | - | H |
| 242 | 3 | H | H | 5 | 3-N | C | C | C | C | 4-$OCH_3$ | H | H | H |
| 243 | 3 | H | H | 6 | 3-N | C | C | C | C | 4-$OCH_3$ | H | H | H |
| 244 | 3 | H | H | 5 | 4-N | C | C | C | C | 2-$CH_3$ | H | H | H |
| 245 | 3 | H | H | 6 | 4-N | C | C | C | C | 2-$CH_3$ | H | H | H |
| 246 | 3 | H | H | 6 | 3-N | C | C | C | C | 5-Cl | H | H | Ac |
| 247 | 3 | H | H | 6 | 3-N | C | C | C | C | 5-Cl | H | H | H |
| 248 | 3 | H | H | 5 | 3-N | C | C | C | C | 4-$CH_3$ | H | H | Ac |
| 249 | 3 | H | H | 5 | 3-N | C | C | C | C | 4-$CH_3$ | H | H | H |
| 252 | 3 | H | H | 2 | 5-N | Is | N | N | N | H | - | - | Ac |
| 253 | 3 | H | H | 2 | 5-N | Is | N | N | N | H | - | - | H |
| 254 | 3 | 2-F | H | 6 | 4-O | Is | N | C | C | 3-$CH_3$ | 5-$CH_3$ | - | Ac |
| 255 | 3 | 2-F | H | 6 | 4-O | Is | N | C | C | 3-$CH_3$ | 5-$CH_3$ | - | H |
| 256 | 3 | 2-F | H | 6 | 3-N | C | C | C | C | 2-F | H | H | Ac |
| 257 | 3 | 2-F | H | 6 | 3-N | C | C | C | C | 2-F | H | H | H |
| 258 | 3 | 2-F | H | 6 | 5-N | C | N | C | C | H | H | H | Ac |
| 259 | 3 | 2-F | H | 6 | 5-N | C | N | C | C | H | H | H | H |
| 260 | 3 | 2-F | H | 6 | 3-N | C | C | C | C | 6-F | H | H | Ac |
| 261 | 3 | 2-F | H | 6 | 3-N | C | C | C | C | 6-F | H | H | H |
| 262 | 3 | 2-F | H | 6 | 4-N | C | C | C | C | 2-F | H | H | Ac |

(suite)

| Ex | Pos -N | R’ | R" | Pos-A | A | | | | | | | | R |
|---|---|---|---|---|---|---|---|---|---|---|---|---|---|
| 263 | 3 | 2-F | H | 6 | 4-N | C | C | C | C | 2-F | H | H | H |
| 264 | 3 | 2-F | H | 6 | 4-N | ls | N | C | C | 1-$CH_3$ | H | H | Ac |
| 265 | 3 | 2-F | H | 6 | 4-N | ls | N | C | C | 1-$CH_3$ | H | H | H |
| 266 | 3 | 2-F | H | 6 | 3-N | C | C | C | C | 6-$CH_3$ | H | H | Ac |
| 267 | 3 | 2-F | H | 6 | 3-N | C | C | C | C | 6-$CH_3$ | H | H | H |
| 268 | 3 | 2-F | H | 6 | 4-N | C | C | C | C | 2-$CH_3$ | H | H | Ac |
| 269 | 3 | 2-F | H | 6 | 4-N | C | C | C | C | 2-$CH_3$ | H | H | H |
| 270 | 3 | 2-F | H | 6 | 3-N | C | C | C | C | 6-CN | H | H | Ac |
| 271 | 3 | 2-F | H | 6 | 3-N | C | C | C | C | 6-CN | H | H | H |
| 272 | 3 | 5-F | H | 6 | 3-N | C | C | C | C | H | H | H | Ac |
| 273 | 3 | 5-F | H | 6 | 3-N | C | C | C | C | H | H | H | H |
| 274 | 3 | 5-F | H | 6 | 4-N | C | C | C | C | 2-F | H | H | Ac |
| 275 | 3 | 5-F | H | 6 | 4-N | C | C | C | C | 2-F | H | H | H |
| 276 | 3 | 2-Cl | H | 5 | 5-N | C | N | C | C | H | H | H | Ac |
| 277 | 3 | 2-Cl | H | 5 | 5-N | C | N | C | C | H | H | H | H |
| 278 | 3 | 2-Cl | H | 5 | 3-N | C | C | C | C | 6-F | H | H | Ac |
| 279 | 3 | 2-Cl | H | 5 | 3-N | C | C | C | C | 6-F | H | H | H |
| 280 | 3 | 2-Cl | H | 6 | 5-N | C | N | C | C | H | H | H | Ac |
| 281 | 3 | 2-Cl | H | 6 | 5-N | C | N | C | C | H | H | H | H |
| 282 | 3 | 2-F | H | 5 | 2-N | C | C | C | C | H | H | H | Ac |
| 283 | 3 | 2-F | H | 5 | 2-N | C | C | C | C | H | H | H | H |
| 284 | 3 | 2-F | H | 5 | 5-N | C | N | C | C | H | H | H | Ac |
| 285 | 3 | 2-F | H | 5 | 5-N | C | N | C | C | H | H | H | H |

Dans le tableau ci-dessus :

- Pos-N indique la position du groupe thioxyloside par rapport à l’atome d’azote du cycle pyridine,
- Pos-A indique la position de l’hétérocycle A par rapport à l’atome d’azote du cycle pyridine,
- X indique la nature de l’hétéroatome primaire de l’hétérocycle A et sa position par rapport à la liaison de l’hétérocycle A avec le noyau pyridine,
- « ls » signifie liaison simple,
- pour les substituants R1, R2 et R3, le chiffre indique la position du substituant sur l’hétérocycle A par rapport à l’hétéroatome X.

A titre d’exemple, le composé N° 226 est le composé de formule :

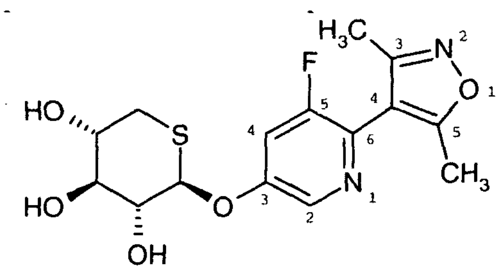

L'activité antithrombotique des composés selon l'invention a été étudiée *in vivo* chez le rat grâce à un test reproduisant une thrombose veineuse.

La thrombose veineuse a été induite selon le protocole décrit dans Thromb. Haemost. 1992, 67(1), 176-179. L'activité par voie orale a été étudiée selon le protocole opératoire décrit ci-dessous.

L'expérimentation est réalisée sur des rats mâles Wistar, non à jeun, pesant 250 à 280 g et répartis en groupes de 10 animaux chacun. Les produits à tester sont administrés par voie orale (tubage) en solution ou en suspension dans une solution de méthylcellulose à 0,5 % dans l'eau. La concentration des composés est calculée de façon à faire absorber une quantité de solution de 10 ml/kg par voie orale. Une thrombose est induite à un temps T après l'administration du produit et le thrombus formé est prélevé et pesé. Pour induire cette thrombose, on réalise une stase veineuse sous hypercoagulation, selon la technique décrite par WESSLER (J. Applied Physiol. 1959, 943-946) en utilisant en tant qu'agent hypercoagulant une solution de facteur X activé (Xa), fournie par la société Biogenic (Montpellier), et dosée à 7,5 nKat/kg. La stase veineuse est effectuée 10 secondes exactement après l'injection de l'agent hypercoagulant. L'activité des composés testés a été contrôlée à différentes doses, après qu'ils aient été administrés. L'induction de la thrombose a été faite 2 heures après l'administration du composé. A titre d'exemple, les résultats des tests précédents sont reportés dans les tableaux suivants pour quelques composés selon l'invention (l'activité est exprimée par le pourcentage d'inhibition de la formation du thrombus, observé en présence du composé selon l'invention, par rapport au poids du thrombus formé en l'absence du composé).

**Tableau I**

| Activité par voie orale | | | |
|---|---|---|---|
| N° Exemple | Dose (mg/kg) | Temps (h) | Activité |
| 22 | 6 | 2 | 100 |
| 24 | 6 | 2 | 96 |
| 30 | 6 | 2 | 97 |
| 40 | 6 | 2 | 96 |
| 108 | 6 | 2 | 95 |
| 122 | 6 | 2 | 93 |
| 130 | 6 | 2 | 91 |
| 140 | 6 | 2 | 96 |
| 142 | 6 | 2 | 97 |
| 224 | 6 | 2 | 99 |
| 226 | 6 | 2 | 97 |

Ces résultats montrent que les composés selon l'invention présentent une bonne activité antithrombotique, après administration par voie orale

La présente invention a donc pour objet un composé de formule (I) selon l'invention ainsi que ses sels avec un acide, solvates et hydrates pharmaceutiquement acceptables pour leur utilisation en tant que médicament. Le composé de formule (I) ou un de sels, solvates ou hydrates pharmaceutiquement acceptables pourra être utilisé pour la préparation d'un médicament antithrombotique destiné, en particulier, au traitement ou à la prévention des troubles de la circulation veineuse ou artérielle, et notamment, pour corriger certains paramètres hématologiques sensibles au niveau veineux, ou pour compenser une insuffisance cardiaque. Le composé de formule (I) ou un de sels, solvates ou

hydrates pharmaceutiquement acceptables pourra également être utilisé pour la préparation d'un médicament destiné à la prévention d'une resténose après angioplastie transluminale artérielle ou coronarienne, ou encore pour prévenir ou traiter des pathologies de type thromboembolique risquant de survenir suite par exemple à un acte chirurgical comme une arthroplastie de hanche ou de genou. Les composés selon l'invention pourront encore être utilisés en tant que principes actifs de médicaments destinés à prévenir les accidents vasculaires au niveau cérébral ou cardiaque.

La présente invention a donc également pour objet des compositions pharmaceutiques contenant un composé de formule (I) ou un de ses sels, solvates ou hydrates pharmaceutiquement acceptables. Ces compositions pharmaceutiques contiennent en général des excipients convenables. Lesdits excipients sont choisis selon la forme pharmaceutique et le mode d'administration souhaités, en particulier orale ou injectable.

Ces compositions pharmaceutiques sont préparées selon les méthodes classiques bien connues de l'homme du métier. Par exemple, les composés selon l'invention peuvent être formulés avec des excipients physiologiquement acceptables pour obtenir une forme injectable à utiliser directement, une forme injectable à préparer extemporanément ou une forme solide pour administration par voie orale telle que, par exemple, une gélule ou un comprimé.

A titre d'exemple, une forme injectable peut être préparée de préférence par lyophilisation d'une solution filtrée et stérilisée contenant le composé selon l'invention et un excipient soluble en quantité nécessaire et suffisante pour obtenir une solution isotonique après addition extemporanée d'eau pour injection. La solution obtenue pourra être administrée soit en une seule injection sous-cutanée ou intramusculaire, soit sous forme d'une perfusion lente. Une forme administrable par voie orale sera de préférence présentée sous forme d'une gélule contenant le composé de l'invention broyé finement ou mieux, micronisé, et mélangé avec des excipients connus de l'homme du métier, tels que par exemple du lactose, de l'amidon prégélatinisé et du stéarate de magnésium.

Afin d'obtenir l'effet thérapeutique ou prophylactique désiré, chaque dose unitaire peut contenir 10 à 500 mg d'au moins un composé selon l'invention.

## Revendications

**1.** Composé du thioxylose, **caractérisé en ce qu'**il est choisi parmi :

a) les composés de formule :

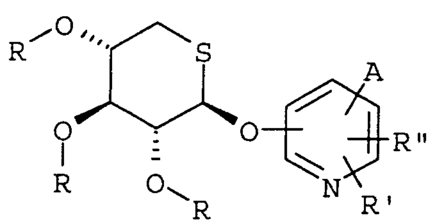

I

dans laquelle :

- le groupe pentapyranosyle représente un groupe 5-thio-β-D-xylopyranosyle,
- R représente un atome d'hydrogène ou un groupe acyle en $C_2$-$C_6$,
- R' et R" représentent chacun indépendamment, un atome d'hydrogène, un atome d'halogène, un groupe alkyle en $C_1$-$C_4$ ou un groupe 6-fluoro-3-pyridinyle,
- A représente un hétérocycle aromatique à 5 ou 6 chaînons de formule :

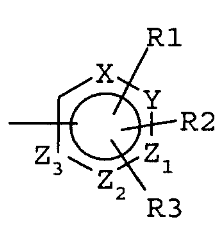

dans laquelle :

- X représente un atome d'azote, d'oxygène ou de soufre,

- Y représente un atome de carbone ou une liaison simple,
- $Z_1$, $Z_2$ et $Z_3$ représentent chacun indépendamment, un atome de carbone ou d'azote,
- $R_1$, $R_2$ et $R_3$ représentent chacun indépendamment, un atome d'hydrogène ou un atome d'halogène, un groupe cyano, un groupe alkyle en $C_1$-$C_4$, un groupe alcoxy en $C_1$-$C_4$, un groupe trifluorométhyle ; ou
- $R_1$ et $R_2$ forment ensemble avec les atomes de l'hétérocycle auxquels ils sont rattachés un cycle aromatique comprenant 6 atomes de carbone, A représentant ainsi un groupe bicyclique fusionné,

b) les sels d'addition de ces composés.

**2.** Composé selon la revendication 1, **caractérisé en ce que** A représente un groupe benzofuranyle ou benzothiényle.

**3.** Composé selon la revendication 1 ou 2, **caractérisé en ce que** le groupe 5-thio-β-D-xylopyranosyle est en position 3 sur le cycle pyridine.

**4.** Composé selon l'une des revendications 1 à 3, **caractérisé en ce que** R' et R'' représentent chacun un atome d'hydrogène, un atome d'halogène ou un groupe méthyle.

**5.** Composé selon l'une des revendications 1 à 4, **caractérisé en ce que** A représente un cycle pyridinyle.

**6.** Composé selon l'une des revendications 1 à 5, **caractérisé en ce que** R représente un atome d'hydrogène.

**7.** Composé selon l'une des revendications 1 à 5, **caractérisé en ce que** R représente un groupe $COCH_3$.

**8.** Procédé de fabrication d'un composé selon la revendication 1, **caractérisé en ce que** l'on effectue les étapes consistant à :

a) faire réagir un pyridinol de formule :

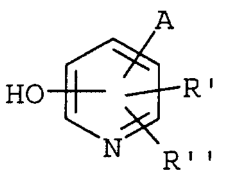

II

dans laquelle :

- R' et R'' représentent chacun indépendamment, un atome d'hydrogène, un atome d'halogène ou un groupe alkyle en $C_1$-$C_4$,
- A représente un cycle aromatique à 5 ou 6 chaînons de formule :

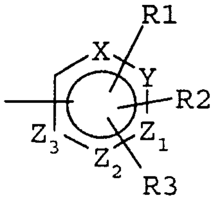

dans laquelle :

- X représente un atome d'azote, d'oxygène ou de soufre,
- Y représente un atome de carbone ou une liaison simple,
- $Z_1$, $Z_2$ et $Z_3$ représentent chacun indépendamment, un atome de carbone ou d'azote,
- $R_1$, $R_2$ et $R_3$ représentent chacun indépendamment, un atome d'hydrogène ou un atome d'halogène, un groupe cyano, un groupe alkyle en $C_1$-$C_4$, un groupe alcoxy en $C_1$-$C_4$, un groupe trifluorométhyle ; ou
- $R_1$ et $R_2$ forment ensemble avec les atomes de l'hétérocycle auxquels ils sont rattachés un cycle aromatique

comprenant 6 atomes de carbone, A représentant ainsi un groupe bicyclique fusionné, avec un dérivé du 5-thioxylopyranose de formule :

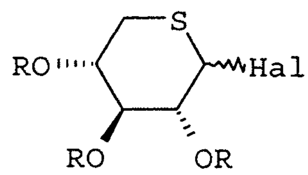

dans laquelle Hal représente un halogène et R représente un groupe acyle en $C_2$-$C_6$, dans un solvant aprotique, en présence d'un sel d'argent ou d'un sel de zinc en milieu anhydre, à une température comprise entre 25 et 110°C et pendant 1 à 10 heures, pour obtenir le composé de formule :

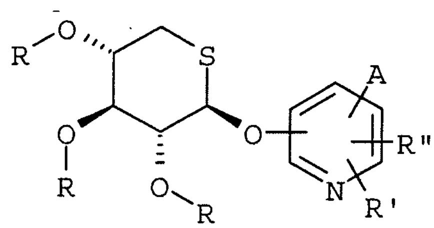

I

dans laquelle A, R, R' et R" conservent la même signification que dans les composés de départ ;

b) si nécessaire, faire réagir le composé de formule I obtenu ci-dessus avec une solution d'ammoniac dans du méthanol pour réaliser la désacylation et ainsi remplacer le groupe acyle par des atomes d'hydrogène et obtenir le composé de formule :

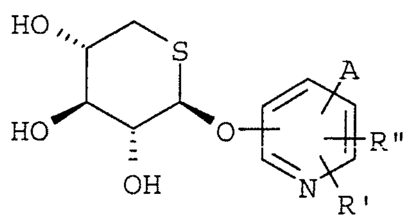

Ia

dans laquelle $R_1$ et $R_2$ conservent la même signification que ci-dessus ;
c) si nécessaire, faire réagir l'un des composés I ou Ia obtenus ci-dessus avec un acide selon des méthodes connues de l'homme de métier pour obtenir le sel d'addition correspondant.

**9.** Procédé de fabrication d'un composé selon la revendication 8, **caractérisé en ce que** l'étape (b) est réalisée par action d'un alcoolate métallique, en quantité catalytique, dans le méthanol, à une température comprise entre 0 et 30°C et pendant 0,5 à 2 heures pour obtenir le composé de formule Ia à partir du composé de formule I dans laquelle R représente un groupe acyle en $C_2$-$C_6$.

**10.** Procédé de fabrication d'un composé selon la revendication 1, **caractérisé en ce que** l'on effectue les étapes consistant à :

a) faire réagir le tétraacétylthioxylose de formule

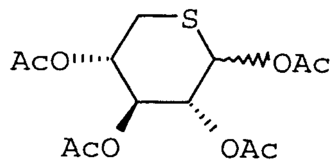

dans laquelle Ac représente le groupe acétyle avec un composé de formule :

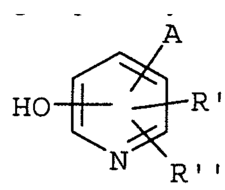

II

dans laquelle :

- R' et R" représentent chacun indépendamment, un atome d'hydrogène, un atome d'halogène ou un groupe alkyle en $C_1$-$C_4$,
- A représente un cycle aromatique à 5 ou 6 chaînons de formule :

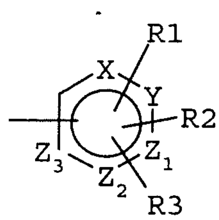

dans laquelle :

- X représente un atome d'azote, d'oxygène ou de soufre,
- Y représente un atome de carbone ou une liaison simple,
- $Z_1$, $Z_2$ et $Z_3$ représentent chacun indépendamment, un atome de carbone ou d'azote,
- $R_1$, $R_2$ et $R_3$ représentent chacun indépendamment, un atome d'hydrogène ou un atome d'halogène, un groupe cyano, un groupe alkyle en $C_1$-$C_4$, un groupe alcoxy en $C_1$-$C_4$, un groupe trifluorométhyle ; ou
- $R_1$ et $R_2$ forment ensemble avec les atomes de l'hétérocycle auxquels ils sont rattachés un cycle aromatique comprenant 6 atomes de carbone, A représentant ainsi un groupe bicyclique fusionné,

dans un solvant aprotique, en présence d'un catalyseur de type acide de Lewis, à une température comprise entre 20 et 60 °C et pendant 1 à 2 heures, pour obtenir le composé de formule :

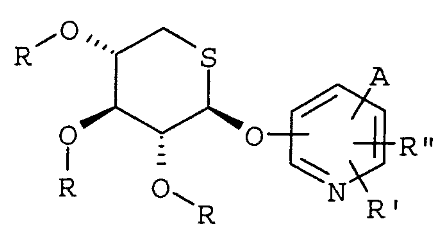

Ib

dans laquelle A, R, R' et R" conservent la même signification que dans les composés de départ.
b) si nécessaire échanger les groupes acétyle par des atomes d'hydrogène selon les méthodes appliquées dans les procédés précédents pour obtenir le composé de formule Ia.

**11.** Procédé de fabrication d'un composé selon la revendication 1, **caractérisé en ce que** l'on effectue les étapes consistant à :

a) faire réagir un composé de formule :

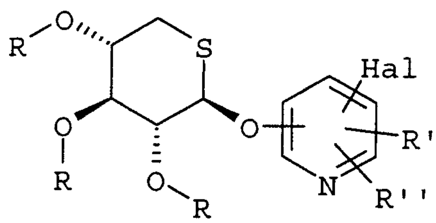

dans laquelle Hal est un atome d'halogène, R' et R" représentent chacun indépendamment un atome d'hydrogène, un atome d'halogène, autre que le brome ou l'iode, ou un groupe alkyle en $C_1$-$C_4$, et R représente un atome d'hydrogène ou un groupe acyle en $C_2$-$C_6$ ;
avec un acide hétéroarylboronique ou un hétéroarylboronate d'alkyle de formule :

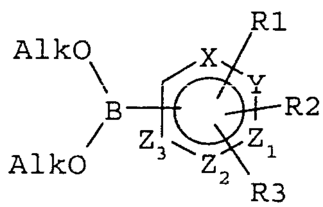

dans laquelle :

- X représente un atome d'azote, d'oxygène ou de soufre,
- Y représente un atome de carbone ou une liaison simple,
- $Z_1$, $Z_2$ et $Z_3$ représentent chacun indépendamment, un atome de carbone ou d'azote,
- $R_1$, $R_2$ et $R_3$ représentent chacun indépendamment, un atome d'hydrogène ou un atome d'halogène, un groupe cyano, un groupe alkyle en $C_1$-$C_4$, un groupe alcoxy en $C_1$-$C_4$, un groupe trifluorométhyle ; ou
- $R_1$ et $R_2$ forment ensemble avec les atomes de l'hétérocycle auxquels ils sont rattachés un cycle aromatique comprenant 6 atomes de carbone, A représentant ainsi un groupe bicyclique fusionné,
- Alk représente un atome d'hydrogène ou un groupe alkyle en $C_1$-$C_4$ ;

l'ensemble

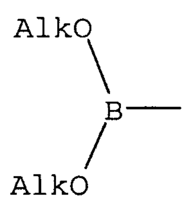

pouvant en outre représenter un groupe "pinacolborane"
en présence d'un catalyseur au palladium, d'un solvant polaire, et du fluorure de césium ou du carbonate de sodium, à une température comprise entre 70°C et 150°C pendant 5 minutes à 72 heures, pour obtenir le composé de formule :

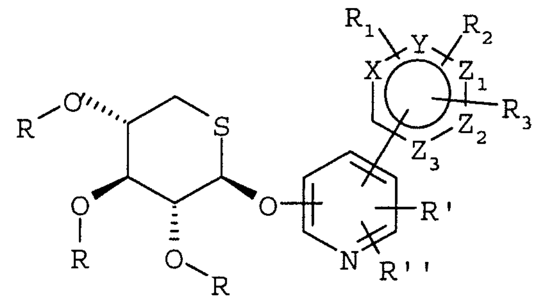

dans laquelle :

R, $R_1$, $R_2$, $R_3$, R', R", X, Y, $Z_1$, $Z_2$ et $Z_3$ conservent la même signification que dans les produits de départ.

**12.** Procédé de fabrication d'un composé selon la revendication 1, **caractérisé en ce que** l'on effectue les étapes consistant à :

a) faire réagir un acide pyridineboronique glycosylé ou un pyridinylboronate glycosylé de formule:

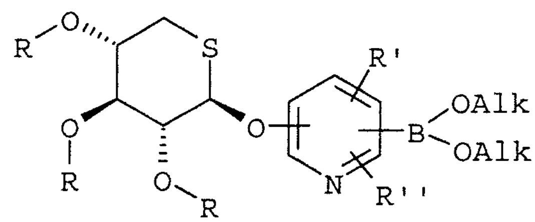

dans laquelle R représente un atome d'hydrogène ou un groupe acyle en $C_2$-$C_6$, R' et R" représentent chacun indépendamment un atome d'hydrogène, un atome

d'halogène, autre que le brome ou l'iode, ou un groupe alkyle en $C_1$-$C_4$ et Alk représente un atome d'hydrogène ou un groupe alkyle en $C_1$-$C_4$.

avec un halogénure d'hétéroaryle de formule:

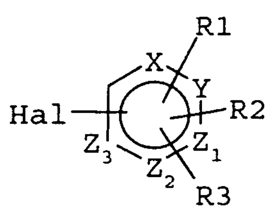

dans laquelle Hal représente un halogène et $R_1$, $R_2$ et $R_3$ représentent chacun indépendamment, un atome d'hydrogène ou un atome d'halogène, un groupe cyano, un groupe alkyle en $C_1$-$C_4$, un groupe alcoxy en $C_1$-$C_4$, un groupe trifluorométhyle ; ou $R_1$ et $R_2$ forment ensemble avec les atomes de l'hétérocycle auxquels ils sont rattachés un cycle aromatique comprenant 6 atomes de carbone, A représentant ainsi un groupe bicyclique fusionné,

en présence d'un catalyseur au palladium, d'un solvant protique polaire et du fluorure de césium ou du carbonate de sodium, à une température comprise entre 70°C et 150°C pendant 5 minutes à 72 heures, pour obtenir le composé de formule

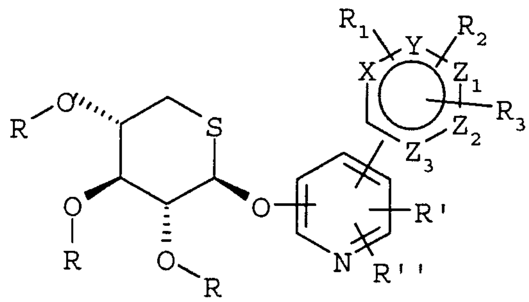

dans laquelle :

R, $R_1$, $R_2$, $R_3$, R', R", X, Y, $Z_1$, $Z_2$ et $Z_3$ conservent la même signification que dans les produits de départ.

**13.** Composé selon l'une quelconque des revendications 1 à 6 pour son utilisation en tant que substance pharmacologiquement active.

**14.** Utilisation d'un composé selon l'une quelconque des revendications 1 à 6 pour la préparation d'un médicament destiné à la prévention ou au traitement des thromboses, ou des thromboses veineuses.

**15.** Utilisation d'un composé selon l'une quelconque des revendications 1 à 6 pour la préparation d'un médicament destiné à la prévention ou au traitement de l'insuffisance cardiaque.

**16.** Utilisation d'un composé selon l'une quelconque des revendications 1 à 6 pour la préparation d'un médicament destiné à la prévention d'une resténose suite à une angioplastie ou de pathologies de type thromboembolique.

## Claims

**1.** A thioxylose compound, **characterized in that** it is chosen from:

a) the compounds of formula:

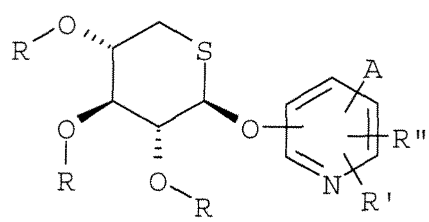

I

in which:

- the pentapyranosyl group represents a 5-thio-β-D-xylopyranosyl group,
- R represents a hydrogen atom or a $C_2$-$C_6$ acyl group,
- R' and R" each independently represent a hydrogen atom, a halogen atom, a $C_1$-$C_4$ alkyl group or a 6-fluoro-3-pyridinyl group,
- A represents a 5- or 6- membered aromatic heterocycle of formula:

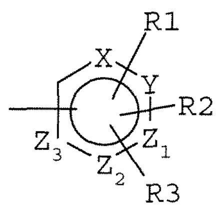

in which:

- X represents a nitrogen, oxygen or sulfur atom,
- Y represents a carbon atom or a single bond,
- $Z_1$, $Z_2$ and $Z_3$ each independently represent a carbon or nitrogen atom,
- $R_1$, $R_2$ and $R_3$ each independently represent a hydrogen atom, a halogen atom, a cyano group, a $C_1$-$C_4$ alkyl group, a $C_1$-$C_4$ alkoxy group or a trifluoromethyl group; or
- $R_1$ and $R_2$ form, together with the atoms of the heterocycle to which they are attached, an aromatic ring comprising 6 carbon atoms, A thus representing a fused bicyclic group,

b) the addition salts of said compound.

2. The compound as claimed in claim 1, **characterized in that** A represents a benzofuranyl or a benzothienyl group.

3. The compound as claimed in claim 1 or 2, **characterized in that** the 5-thio-β-D-xylopyranosyl group is in the 3 position on the pyridine ring.

4. The compound as claimed in one of claims 1 to 3, **characterized in that** R' and R" each represent a hydrogen atom, a halogen atom or a methyl group.

5. The compound as claimed in one of claims 1 to 4, **characterized in that** A represents a pyridinyl ring.

6. The compound as claimed in one of claims 1 to 5, **characterized in that** R represents a hydrogen atom.

7. The compound as claimed in one of claims 1 to 6, **characterized in that** R represents a $COCH_3$ group.

8. A process for the manufacture of a compound as claimed in claim 1, **characterized in that** the following steps are carried out:

a) reacting a pyridinol of formula:

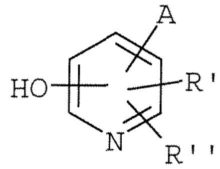

II

in which:

- R' and R" each independently represent a hydrogen atom, a halogen atom or a $C_1$-$C_4$ alkyl group,
- A represents a 5- or 6- membered aromatic ring of formula:

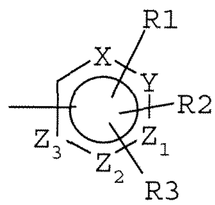

in which:

- X represents a nitrogen, oxygen or sulfur atom,
- Y represents a carbon atom or a single bond,
- $Z_1$, $Z_2$ and $Z_3$ each independently represent a carbon or nitrogen atom,
- $R_1$, $R_2$ and $R_3$ each independently represent a hydrogen atom, a halogen atom, a cyano group, a $C_1$-$C_4$ alkyl group, a $C_1$-$C_4$ alkoxy group, or a trifluoromethyl group; or
- $R_1$ and $R_2$ form, together with the atoms of the heterocycle to which they are attached, an aromatic ring comprising 6 carbon atoms, A thus representing a fused bicyclic group,

with a 5-thioxylopyranose derivative of formula:

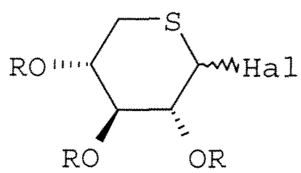

in which Hal represents a halogen and R represents a $C_2$-$C_6$ acyl group, in an aprotic solvent, in the presence of a silver salt or of a zinc salt, in an anhydrous medium, at a temperature of between 25 and 110°C and for 1 to 10 hours, in order to obtain the compound of formula:

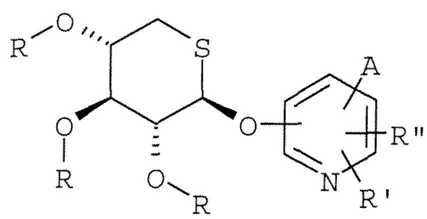

I

in which A, R, R' and R" retain the same meanings as in the starting compounds;

b) if necessary, reacting the compound of formula I obtained above with a solution of ammonia in methanol in order to bring about the deacylation and thus to replace the acyl group by hydrogen atoms and to obtain the compound of formula:

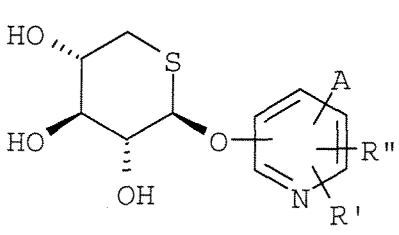

Ia

in which $R_1$ and $R_2$ retain the same meanings as above;

c) if necessary, reacting one of the compounds I or Ia obtained above with an acid according to methods known to a person skilled in the art in order to obtain the corresponding addition salt.

**9.** The process for the manufacture of a compound as claimed in claim 8, **characterized in that** stage (b) is brought about by the action of a metal alkoxide, in a catalytic amount in methanol, at a temperature of between 0 and 30˚C and for 0.5 to 2 hours, in order to obtain the compound of formula Ia from the compound of formula I in which R represents a $C_2$-$C_6$ acyl group.

**10.** A process for the manufacture of a compound as claimed in claim 1, **characterized in that** the following steps are carried out:

a) reacting the tetraacetylthioxylose of formula:

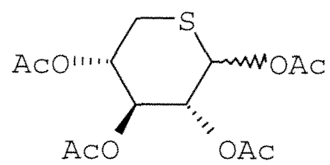

in which Ac represents the acetyl group, with a compound of formula:

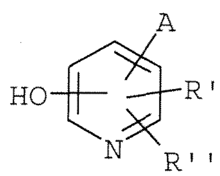

II

in which:

- R' and R" each independently represent a hydrogen atom, a halogen atom or a $C_1$-$C_4$ alkyl group,
- A represents a 5- or 6- membered aromatic ring of formula:

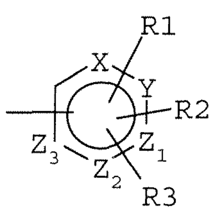

in which:

- X represents a nitrogen, oxygen or sulfur atom,
- Y represents a carbon atom or a single bond,
- $Z_1$, $Z_2$ and $Z_3$ each independently represent a carbon or nitrogen atom,
- $R_1$, $R_2$ and $R_3$ each independently represent a hydrogen atom, a halogen atom, a cyano group, a $C_1$-$C_4$ alkyl group, a $C_1$-$C_4$ alkoxy group or a trifluoromethyl group; or
- $R_1$ and $R_2$ form, together with the atoms of the heterocycle to which they are attached, an aromatic ring comprising 6 carbon atoms, A thus representing a fused bicyclic group,

in an aprotic solvent, in the presence of a catalyst of Lewis acid type, at a temperature of between 20 and 60˚C and for 1 to 2 hours, in order to obtain the compound of formula:

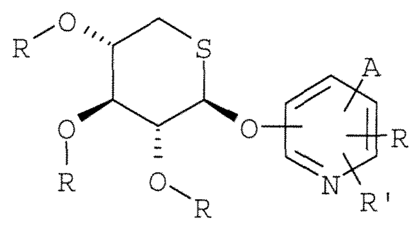

Ib

in which A, R, R' and R" retain the same meanings as in the starting compounds, b) if necessary, exchanging the acetyl groups with hydrogen atoms according to the methods applied in the preceding processes, in order to obtain the compound of formula Ia.

**11.** A process for the manufacture of a compound as claimed in claim 1, **characterized in that** the following steps are carried out:

a) reacting a compound of formula:

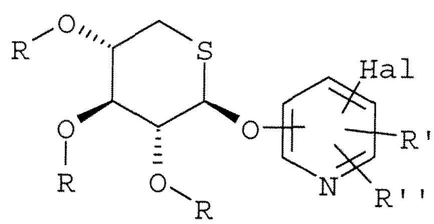

in which Hal is a halogen atom, R' and R" each independently represent a hydrogen atom, a halogen atom, other than bromine or iodine, or a $C_1$-$C_4$ alkyl group, and R represents a hydrogen atom or a $C_2$-$C_6$ acyl group; with a heteroarylboronic acid or an alkyl heteroarylboronate of formula:

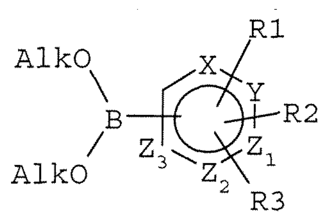

in which:

- X represents a nitrogen, oxygen or sulfur atom,
- Y represents a carbon atom or a single bond,
- $Z_1$, $Z_2$ and $Z_3$ each independently represent a carbon or nitrogen atom,
- $R_1$, $R_2$ and $R_3$ each independently represent a hydrogen atom, a halogen atom, preferably a fluorine atom, a cyano group, a $C_1$-$C_4$ alkyl group, a $C_1$-$C_4$ alkoxy group or a trifluoromethyl group; or
- $R_1$ and $R_2$ form, together with the atoms of the heterocycle to which they are attached, an aromatic ring comprising 6 carbon atoms, A thus representing a fused bicyclic group,
- Alk represents a hydrogen atom or a $C_1$-$C_4$ alkyl group;

it being possible for the combination

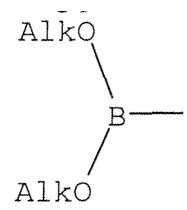

in addition to represent a "pinacolatoboryl" group,
in the presence of a palladium catalyst, of a polar solvent and of cesium fluoride or sodium carbonate, at a temperature of between 70°C and 150°C for 5 minutes to 72 hours, in order to obtain the compound of formula:

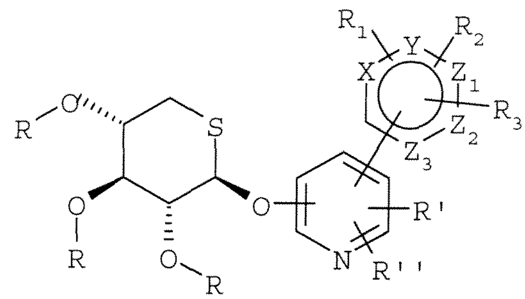

in which:
R, $R_1$, $R_2$, $R_3$, R', R", X, Y, $Z_1$, $Z_2$ and $Z_3$ retain the same meanings as in the starting materials.

**12.** A process for the manufacture of a compound as claimed in claim 1, **characterized in that** the following steps are carried out:

a) reacting a glycosylated pyridineboronic acid or a glycosylated pyridinylboronate of formula:

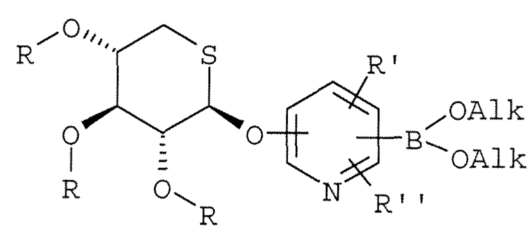

in which R represents a hydrogen atom or a $C_2$-$C_6$ acyl group, R' and R" each independently represent a hydrogen atom, a halogen atom, other than bromine or iodine, or a $C_1$-$C_4$ alkyl group and Alk represents a hydrogen atom or a $C_1$-$C_4$ alkyl group,
with a heteroaryl halide of formula:

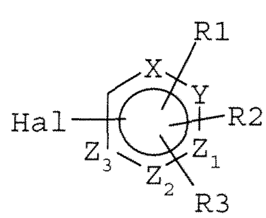

in which Hal represents a halogen and $R_1$, $R_2$ and $R_3$ each independently represent a hydrogen atom, a halogen atom, a cyano group, a $C_1$-$C_4$ alkyl group, a $C_1$-$C_4$ alkoxy group or a trifluoromethyl group; or $R_1$ and $R_2$ form,

together with the atoms of the heterocycle to which they are attached, an aromatic ring comprising 6 carbon atoms, A thus representing a fused bicyclic group,
in the presence of a palladium catalyst, of a polar protic solvent and cesium fluoride or sodium carbonate, at a temperature of between 70°C and 150°C for 5 minutes to 72 hours, in order to obtain the compound of formula:

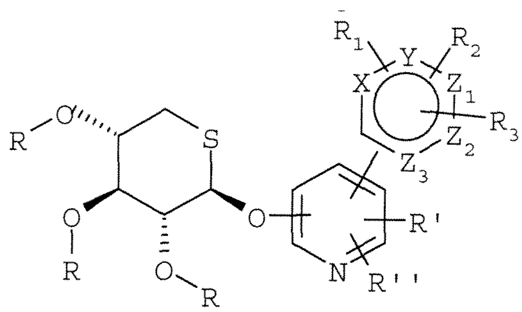

in which:

R, $R_1$, $R_2$, $R_3$, R', R", X, Y, $Z_1$, $Z_2$ and $Z_3$ retain the same meanings as in the starting materials.

**13.** The compound as claimed in any one of claims 1 to 6 for its use as pharmacologically active substance.

**14.** The use of a compound as claimed in any one of claims 1 to 6 for the preparation of a medicament intended for the prevention or treatment of thrombosis or venous thrombosis.

**15.** The use of a compound as claimed in any one of claims 1 to 6 for the preparation of a medicament intended for the prevention or treatment of cardiac insufficiency.

**16.** The use of a compound as claimed in any one of claims 1 to 6 for the preparation of a medicament intended for the prevention of restenosis subsequent to an angioplasty or of pathologies of thromboembolic type.

**Patentansprüche**

**1.** Thioxylose-Verbindung, **dadurch gekennzeichnet, daß** sie ausgewählt ist aus:

a) den Verbindungen der Formel:

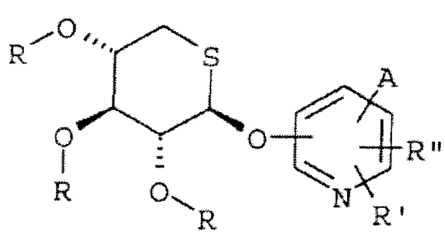

I

worin:

- die Pentapyranosyl-Gruppe eine 5-Thio-β-D-xylopyranosyl-Gruppe darstellt,
- R ein Wasserstoffatom oder eine $C_2$-$C_6$-Acylgruppe darstellt,
- R' und R" jeweils unabhängig ein Wasserstoffatom, ein Halogenatom, eine $C_1$-$C_4$-Alkylgruppe oder eine 6-Fluoro-3-pyridinyl-Gruppe darstellen,
- A einen fünf- oder sechsgliedrigen aromatischen Heterozyklus folgender Formel darstellt:

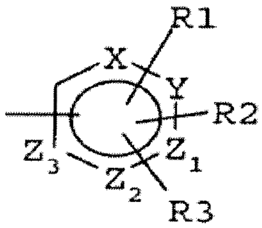

worin:

- X ein Stickstoff-, Sauerstoff- oder Schwefelatom darstellt,
- Y ein Kohlenstoffatom oder eine einfache Bindung darstellt,
- $Z_1$, $Z_2$ und $Z_3$ jeweils unabhängig ein Kohlenstoff- oder Stickstoffatom darstellen,
- $R_1$, $R_2$ und $R_3$ jeweils unabhängig ein Wasserstoffatom oder ein Halogenatom, eine Cyanogruppe, eine $C_1$-$C_4$-Alkylgruppe, eine $C_1$-$C_4$-Alkoxygruppe, eine Trifluormethylgruppe darstellen, oder
- $R_1$ und $R_2$ zusammen mit den Atomen des Heterozyklus, an die sie gebunden sind, einen aromatischen Ring mit 6 Kohlenstoffatomen bilden, wodurch A eine fusionierte bizyklische Gruppe darstellt,

b) den Additionssalzen dieser Verbindungen.

**2.** Verbindung nach Anspruch 1, **dadurch gekennzeichnet, daß** A eine Benzofuranyl- oder Benzothienylgruppe darstellt.

**3.** Verbindung nach Anspruch 1 oder 2, **dadurch gekennzeichnet, daß** die 5-Thio-β-D-xylopyranosyl-Gruppe in Stellung 3 an dem Pyridinring ist.

**4.** Verbindung nach einem der Ansprüche 1 bis 3, **dadurch gekennzeichnet, daß** R' und R'' jeweils ein Wasserstoffatom, ein Halogenatom oder eine Methylgruppe darstellen.

**5.** Verbindung nach einem der Ansprüche 1 bis 4, **dadurch gekennzeichnet, daß** A einen Pyridinylring darstellt.

**6.** Verbindung nach einem der Ansprüche 1 bis 5, **dadurch gekennzeichnet, daß** R ein Wasserstoffatom darstellt.

**7.** Verbindung nach einem der Ansprüche 1 bis 5, **dadurch gekennzeichnet, daß** R eine $COCH_3$-Gruppe darstellt.

**8.** Verfahren zum Herstellen einer Verbindung nach Anspruch 1, **dadurch gekennzeichnet, daß** die Schritte durchgeführt werden, die darin bestehen:

a) ein Pyridinol der Formel:

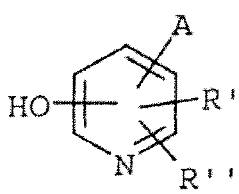

II

worin:

- R' und R'' jeweils unabhängig ein Wasserstoffatom, ein Halogenatom oder eine $C_1$-$C_4$-Alkylgruppe darstellen,
- A einen fünf- oder sechsgliedrigen aromatischen Ring folgender Formel darstellt:

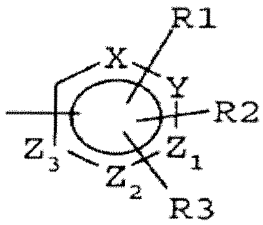

worin:

- X ein Stickstoff-, Sauerstoff- oder Schwefelatom darstellt,
- Y ein Kohlenstoffatom oder eine einfache Bindung darstellt,
- $Z_1$, $Z_2$ und $Z_3$ jeweils unabhängig ein Kohlenstoff- oder Stickstoffatom darstellen,
- $R_1$, $R_2$ und $R_3$ jeweils unabhängig ein Wasserstoffatom oder ein Halogenatom, eine Cyanogruppe, eine $C_1$-$C_4$-Alkylgruppe, eine $C_1$-$C_4$-Alkoxygruppe, eine Trifluormethylgruppe darstellen, oder
- $R_1$ und $R_2$ zusammen mit den Atomen des Heterozyklus, an die sie gebunden sind, einen aromatischen Ring mit 6 Kohlenstoffatomen bilden, wodurch A eine fusionierte bizyklische Gruppe darstellt,

mit einem 5-Thioxylopyranose-Derivat der Formel:

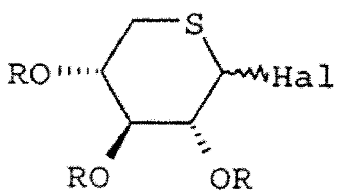

worin Hal ein Halogen und R eine $C_2$-$C_6$-Acylgruppe darstellt, in einem aprotischen Lösungsmittel, in Gegenwart eines Silbersalzes oder eines Zinksalzes in wasserfreiem Medium, bei einer Temperatur zwischen 25 und 110°C für 1 bis 10 Stunden reagieren zu lassen, um die Verbindung folgender Formel zu erhalten:

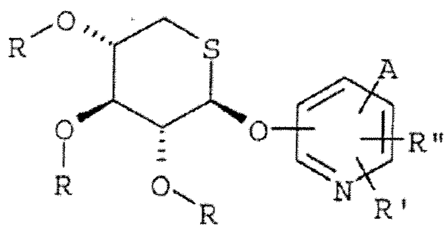

I

worin A, R, R' und R" die gleiche Bedeutung wie bei den Ausgangsverbindungen behalten,

b) falls erforderlich die oben erhaltene Verbindung der Formel I mit einer Lösung aus Ammoniak in Methanol reagieren zu lassen, um die Deacylierung zu vollziehen und um so die Acylgruppe durch Wasserstoffatome zu ersetzen und die Verbindung folgender Formel zu erhalten:

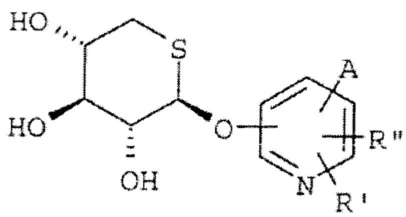

Ia

worin $R_1$ und $R_2$ die gleiche Bedeutung wie oben behalten,

c) falls erforderlich eine der oben erhaltenen Verbindungen I oder Ia nach seitens des Fachmannes bekannten Verfahren mit einer Säure reagieren zu lassen, um das entsprechende Additionssalz zu erhalten.

**9.** Verfahren zum Herstellen einer Verbindung nach Anspruch 8, **dadurch gekennzeichnet, daß** Schritt (b) durch Wirkung eines Metallalkoholates, in katalytischer Menge, in Methanol, bei einer Temperatur zwischen 0 und 30˚C für 0,5 bis 2 Stunden durchgeführt wird, um die Verbindung der Formel Ia anhand der Verbindung der Formel I, worin R eine $C_2$-$C_6$-Acylgruppe darstellt, zu erhalten.

**10.** Verfahren zum Herstellen einer Verbindung nach Anspruch 1, **dadurch gekennzeichnet, daß** die Schritte durchgeführt werden, die darin bestehen:

a) die Tetraacetylthioxylose der Formel

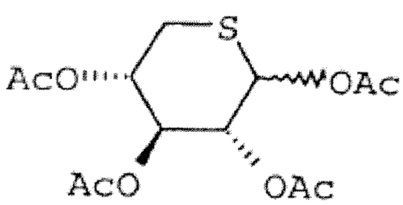

worin Ac die Acetylgruppe darstellt, mit einer Verbindung folgender Formel:

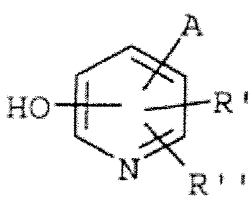

II

worin:

- R' und R" jeweils unabhängig ein Wasserstoffatom, ein Halogenatom oder eine $C_1$-$C_4$-Alkylgruppe darstellen,
- A einen fünf- oder sechsgliedrigen aromatischen Ring folgender Formel darstellt:

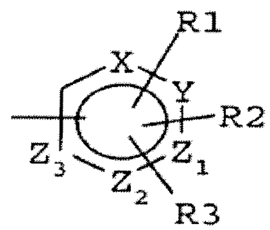

worin:

- X ein Stickstoff-, Sauerstoff- oder Schwefelatom darstellt,
- Y ein Kohlenstoffatom oder eine einfache Bindung darstellt,
- $Z_1$, $Z_2$ und $Z_3$ jeweils unabhängig ein Kohlenstoff- oder Stickstoffatom darstellen,
- $R_1$, $R_2$ und $R_3$ jeweils unabhängig ein Wasserstoffatom oder ein Halogenatom, eine Cyanogruppe, eine $C_1$-$C_4$-Alkylgruppe, eine $C_1$-$C_4$-Alkoxygruppe, eine Trifluormethylgruppe darstellen, oder
- $R_1$ und $R_2$ zusammen mit den Atomen des Heterozyklus, an die sie gebunden sind, einen aromatischen Ring mit 6 Kohlenstoffatomen bilden, wodurch A eine fusionierte bizyklische Gruppe darstellt,

in einem aprotischen Lösungsmittel, in Gegenwart eines Katalysators vom Typ LewisSäure, bei einer Temperatur zwischen 20 und 60°C für 1 bis 2 Stunden reagieren zu lassen, um die Verbindung folgender Formel zu erhalten:

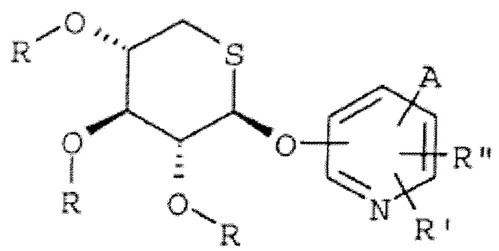

Ib

worin A, R, R' und R" die gleiche Bedeutung wie bei den Ausgangsverbindungen behalten,
b) falls erforderlich die Acetylgruppen nach den Methoden, die bei den vorhergehenden Verfahren für den Erhalt der Verbindung der Formel Ia angewandt werden, durch Wasserstoffatome zu ersetzen.

**11.** Verfahren zum Herstellen einer Verbindung nach Anspruch 1, **dadurch gekennzeichnet, daß** die Schritte durchgeführt werden, die darin bestehen:

a) eine Verbindung der Formel:

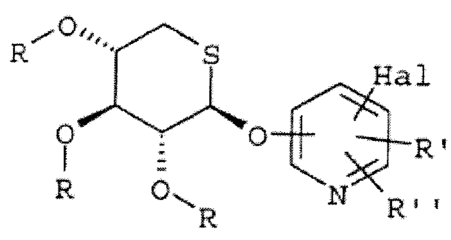

worin Hal ein Halogenatom ist, R' und R" jeweils unabhängig ein Wasserstoffatom, ein anderes Halogenatom als Brom oder Iod oder eine $C_1$-$C_4$-Alkylgruppe darstellen und R ein Wasserstoffatom oder eine $C_2$-$C_6$-Acylgruppe darstellt,
mit einer Heteroarylboronsäure oder einem Alkylheteroarylboronat der Formel:

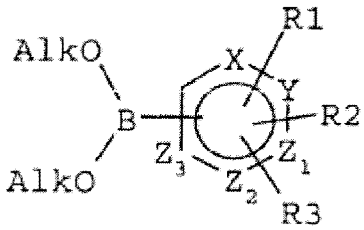

worin:

- X ein Stickstoff-, Sauerstoff- oder Schwefelatom darstellt,
- Y ein Kohlenstoffatom oder eine einfache Bindung darstellt,
- $Z_1$, $Z_2$ und $Z_3$ jeweils unabhängig ein Kohlenstoff- oder Stickstoffatom darstellen,
- $R_1$, $R_2$ und $R_3$ jeweils unabhängig ein Wasserstoffatom oder ein Halogenatom, eine Cyanogruppe, eine $C_1$-$C_4$-Alkylgruppe, eine $C_1$-$C_4$-Alkoxygruppe, eine Trifluormethylgruppe darstellen, oder
- $R_1$ und $R_2$ zusammen mit den Atomen des Heterozyklus, an die sie gebunden sind, einen aromatischen Ring mit 6 Kohlenstoffatomen bilden, wodurch A eine fusionierte bizyklische Gruppe darstellt,
- Alk ein Wasserstoffatom oder eine $C_1$-$C_4$-Alkylgruppe darstellt, wobei das Ganze

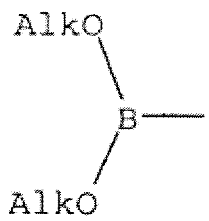

ferner eine "Pinakolboran"-Gruppe darstellen kann

in Gegenwart eines Palladium-Katalysators, eines polaren Lösungsmittels und Cäsiumfluorid oder Natriumcarbonat, bei einer Temperatur zwischen 70°C und 150°C für 5 Minuten bis 72 Stunden reagieren zu lassen, um die Verbindung folgender Formel zu erhalten:

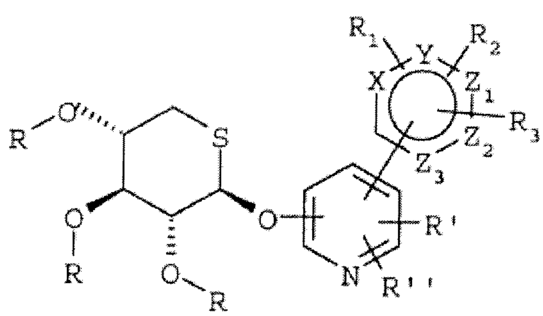

worin:
R, $R_1$, $R_2$, $R_3$, R', R", X, Y, $Z_1$, $Z_2$, und $Z_3$ die gleiche Bedeutung wie bei den Ausgangsprodukten behalten.

**12.** Verfahren zum Herstellen einer Verbindung nach Anspruch 1, **dadurch gekennzeichnet, daß** die Schritte durchgeführt werden, die darin bestehen:

a) eine glykosylierte Pyridinboronsäure oder ein glykosyliertes Pyridinylboronat folgender Formel:

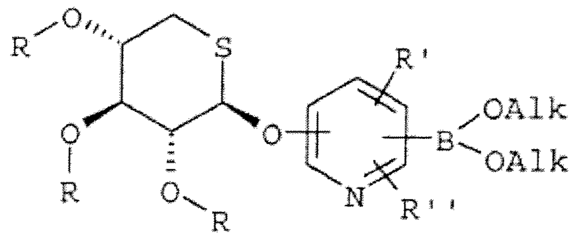

worin R ein Wasserstoffatom oder eine $C_2$-$C_6$-Acylgruppe darstellt, R' und R" jeweils unabhängig ein Wasserstoffatom, ein anderes Halogenatom als Brom oder Iod oder eine $C_1$-$C_4$-Alkylgruppe darstellen und Alk ein Wasserstoffatom oder eine $C_1$-$C_4$-Alkylgruppe darstellt,
mit einem Heteroarylhalogenid folgender Formel:

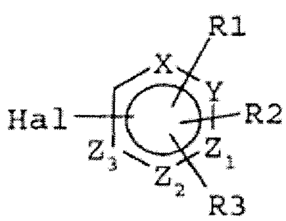

worin Hal ein Halogen darstellt und $R_1$, $R_2$ und $R_3$ jeweils unabhängig ein Wasserstoffatom oder ein Halogenatom, eine Cyanogruppe, eine $C_1$-$C_4$-Alkylgruppe, eine $C_1$-$C_4$-Alkoxygruppe, eine Trifluormethylgruppe darstellen oder $R_1$ und $R_2$ zusammen mit den Atomen des Heterozyklus, an die sie gebunden sind, einen aromatischen Ring mit 6 Kohlenstoffatomen bilden, wodurch A eine fusionierte bizyklische Gruppe darstellt,
in Gegenwart eines Palladium-Katalysators, eines polaren protischen Lösungsmittels und Cäsiumfluorid oder Natriumcarbonat, bei einer Temperatur zwischen 70°C und 150°C für 5 Minuten bis 72 Stunden reagieren zu lassen, um die Verbindung folgender Formel zu erhalten:

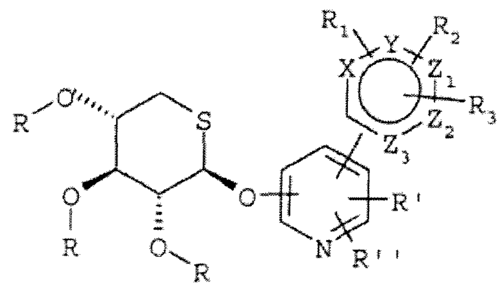

worin:

R, $R_1$, $R_2$, $R_3$, R', R", X, Y, $Z_1$, $Z_2$, und $Z_3$ die gleiche Bedeutung wie bei den Ausgangsprodukten behalten.

**13.** Verbindung nach einem der Ansprüche 1 bis 6 für deren Verwendung als pharmakologisch wirksame Substanz.

**14.** Verwendung einer Verbindung nach einem der Ansprüche 1 bis 6 für die Zubereitung eines Medikaments, das zur Vorbeugung oder zur Behandlung von Thrombosen oder venösen Thrombosen bestimmt ist.

**15.** Verwendung einer Verbindung nach einem der Ansprüche 1 bis 6 für die Zubereitung eines Medikaments, das zur Vorbeugung oder zur Behandlung von Herzinsuffizienz bestimmt ist.

**16.** Verwendung einer Verbindung nach einem der Ansprüche 1 bis 6 für die Zubereitung eines Medikaments, das zur Vorbeugung einer Restenose infolge einer Angioplastie oder von Pathologien vom Typ thromboembolische Patho-

logie bestimmt ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 051023 B1
- US 4877808 A
- EP 421829 B1
- WO 2005030785 A
- EP 0365397 A


### Littérature non-brevet citée dans la description


- *J. Med. Chem.,* vol. 36 (7), 898-903
- *J. Biol. Chem.,* vol. 270 (6), 2662-68
- *Thromb. Haemost.,* 1999, vol. 81, 945-950
- The Carbohydrate, Chemistry and Biochemistry. Academic Press, 1972, 292-294
- *Thromb. Haemost.,* 1992, vol. 67 (1), 176-179
- **WESSLER.** *J. Applied Physiol.,* 1959, 943-946